# EUROPEAN PATENT SPECIFICATION

(11) **EP 2 583 098 B1**
(45) Date of publication and mention of the grant of the patent: **08.08.2018**
(21) Application number: 11726315.2
(22) Date of filing: 15.06.2011
(51) Int. Cl.: G01N 33/50

(54) **BIOMARKERS FOR THE TREATMENT OF PSORIASIS**
BIOMARKER ZUR BEHANDLUNG VON SCHUPPENFLECHTE
BIOMARQUEURS POUR LE TRAITEMENT DU PSORIASIS

(30) Priority: 21.03.2011 US 201161454916 P; 15.06.2010 US 355116 P
(43) Date of publication of application: 24.04.2013
(73) Proprietor: CELGENE CORPORATION, Summit, NJ 07901 (US)
(72) Inventor: SCHAFER, Peter, H., Somerset, NJ 08873 (US); LIN, Yong, Brooklyn, New York City, NY 11220 (US); SUTHERLAND, Donna, Greenbrook, NJ 08812 (US)
(74) Representative: Jones Day
(86) International application number: PCT/US2011/040430
(87) International publication number: WO 2011/159750

(56) References cited:
- US-A1- 2008 234 359
- US-A1- 2009 239 926
- M. A. LOWES: "Increase in TNF- and inducible nitric oxide synthase-expressing dendritic cells in psoriasis and reduction with efalizumab (anti-CD11a)", PROCEEDINGS OF THE NATIONAL ACADEMY OF SCIENCES, vol. 102, no. 52, 27 December 2005 (2005-12-27), pages 19057-19062, XP55003608, ISSN: 0027-8424, DOI: 10.1073/pnas.0509736102
- MALAVIYA R ET AL: "Etanercept induces apoptosis of dermal dendritic cells in psoriatic plaques of responding patients", JOURNAL OF THE AMERICAN ACADEMY OF DERMATOLOGY, C.V. MOSBY, ST. LOUIS, MO, US, vol. 55, no. 4, 1 October 2006 (2006-10-01), pages 590-597, XP027116774, ISSN: 0190-9622 [retrieved on 2006-09-21]
- DE MEIJERE ARMIN: "BONDING PROPERTIES OF CYCLOPROPANE AND THEIR CHEMICAL CONSEQUENCES.", ANGEWANDTE CHEMIE - INTERNATIONAL EDITION IN ENGLISH 1979 NOV, vol. 18, no. 11, November 1979 (1979-11), pages 809-826,
- ANAND B S ET AL: "NOVEL DIPEPTIDE PRODRUGS OF ACYCLOVIR FOR OCULAR HERPES INFECTIONS: BIOREVERSION, ANTIVIRAL ACTIVITY AND TRANSPORT ACROSS RABBIT CORNEA", CURRENT EYE RESEARCH, IRL PRESS, OXFORD, GB, vol. 26, no. 3-04, 1 March 2003 (2003-03-01), pages 151-163, XP009036464, ISSN: 0271-3683, DOI: 10.1076/CEYR.26.3.151.14893

## Description

### 1. FIELD

Provided herein is monitoring of specific biomarkers in samples obtained from skin biopsies before and during therapy for psoriasis. Also provided herein is monitoring of expression of one or more specific genes or proteins before and during the therapy.

### 2. BACKGROUND

Psoriasis is a chronic autoimmune inflammatory skin disorder characterized by epidermal hyperproliferation of keratinocytes and endothelial cells, and inflammatory cell accumulation (*e.g.,* activated T cells). Griffiths CE, J. Eur. Acad. Dermatol. Venereol. 2003, 17 Suppl 2:1-5; Creamer JD, et al., Clin. Exp. Dermatol. 1995, 20(1):6-9. Also, recent evidence suggests the involvement of natural killer (NK) and NK T cells in the pathogenesis of psoriasis as these cells produce interferon-gamma (IFN-γ) which has been shown to play a role in psoriasis keratinocyte proliferation. Bos JD, et al., Br. J. Dermatol. 2005, 152(6):1098-107.

Clinically the main symptoms of psoriasis are gray or silvery flaky patches on the skin that are red and inflamed underneath. Central to the proposed pathogenic pathway are cytokines, chemokines and other inflammatory mediators produced by activated keratinocytes, dendritic cells, neutrophils, and NK T cells which are believed to induce both keratinocytes proliferation and lymphocyte migration. Creamer JD, et al., Clin. Exp. Dermatol. 1995, 20(1):6-9; Bos JD, et al., Br. J. Dermatol. 2005, 152(6):1098-107; Bowcock et al., Nat. Rev. Immunol. 2005, 5(9):699-71. Proinflammatory mediators shown to be elevated in the psoriasis skin lesions include, tumor necrosis factor-alpha (TNF-α), interleukin-6 (IL-6), IL-8, IL-12, IFN-γ, and inducible nitric oxide synthase (iNOS). LaDuca JR, et al., Dermatol. Clin. 2001, 19(4):617-35; Duan H, et al., J. Dermatol. Sci. 2001, 26(2):119-24; Gottlieb et al., J. Immunol. 2005, 175(4):2721-9. Furthermore, low expression levels of the anti-inflammatory cytokine IL-10 were observed in psoriasis lesions. Asadullah K, et al., Curr. Drug Targets Inflamm. Allergy. 2004, 3(2):185-92.

Since the pathogenesis of psoriasis is reported to involve upregulation of at least TNF-α, IFN-γ, IL-6, IL-8 and IL-12 in addition to reductions in IL-10, it is thought that PDE4 inhibitors may provide therapeutic benefits in the treatment of psoriasis. A need exists for reliable biomarkers for psoriasis that can provide accurate assessment with regard to prognosis and efficacy of a particular treatment.

US 2008/234359 describes solid forms comprising (+)-2-[1-(3-Ethoxy-4-methoxyphenyl)-2-methylsulfonylethyl]-4-acetylaminoisoindoline-1,3-dione and methods of using the same for treating or preventing diseases ameliorated by PDE4 inhibition. Also certain biomarkers including CD11c, ICAM-1, HLA-DR, iNOS and K16 are described.

M.A. Lowes, Proceedings of the national academy of sciences, vol. 102, no.52, 27 December 2005, pages 19057-19062, describes that CD11c+ cells are a major cell type in psoriasis skin lesions, discussing expression of CD11c and iNOS at different time points of psoriasis treatment.

Malaviya R. et al., Journal of the American academy of dermatology, vol. 55, no 4, 1 October 2006, pages 590-597, describes the efficacy of Etanercept in psoriasis treatment, including CD11c as a cell marker.

De Meijere A., Angewandte Chemie, vol. 18, no. 11, November 1979, pages 809-826, is a text book excerpt describing the bonding properties of cyclopropane and their chemical consequences.

Anand B S et al., Current eye research, IRL Press, Oxford GB, vol. 26, no. 3-04, 1 March 2003, pages 151-163 concerns an analysis of dipeptide prodrugs in antiviral treatment.

US 2009/239926 describes methods for treating psoriasis and psoriatic arthritis involving administration of cyclopropyl-N-{2-[(1S)-1-(3-ethoxy-4-methoxyphenyl)-2-(methylsulfonyl)ethyl]-3-oxoisoindoline-4-yl}carboxamide as well as pharmaceutical compositions and single unit dose forms.

### 3. SUMMARY

Described herein are biomarkers for predicting or monitoring the efficacy of a treatment for psoriasis.

In one embodiment, provided herein is a method of predicting or monitoring the efficacy of a psoriasis treatment by measuring the level of one or more specific biomarkers in cells obtained from patients before or during the treatment. In one embodiment, the cells are obtained by skin biopsies. In another embodiment, the biomarkers include, CD11c, CD3, CD56, Langerin, and/or Foxp3. In one embodiment, the treatment is administration of a PDE4 modulator provided herein elsewhere.

In another embodiment, provided herein is the use of specific mRNAs as biomarkers to predict or ascertain the efficacy and progress of a treatment for psoriasis. In one embodiment, the mRNA levels of Keratin 16, IL-12/IL-23 p40, IL-23 p19, IL-17A, IL-22, DEFB4, IL-8, MX-1, IL-10, IFN-γ and/or CXCL9 can be used to predict whether a treatment is likely to be successful in treating psoriasis. Further, the expression of these genes can be used to monitor efficacy/progress of the treatment once the treatment begins. In one embodiment, the treatment is administration of a PDE4 modulator provided herein elsewhere.

In yet another embodiment, a method for monitoring patient compliance with a drug treatment protocol is provided. The method comprises measuring the expression level of at least one biomarker provided herein in a sample from the patient, and determining if the expression level is increased or decreased in the patient sample compared to the expression level in a control untreated sample, wherein an increased or decreased expression indicates patient compliance with the drug treatment protocol.

Also described is a useful for predicting the likelihood of an effective treatment of psoriasis. The kit comprises a solid support, nucleic acids contacting the support, where the nucleic acids are complementary to at least 20, 50, 100, 200, 350, or more bases of an mRNA biomarker provided herein, and a means for detecting the expression of the mRNA in a biological sample.

Such a kit can employ, for example a dipstick, a membrane, a chip, a disk, a test strip, a filter, a microsphere, a slide, a multiwell plate, or an optical fiber. The solid support of the kit can be, for example, a plastic, silicon, a metal, a resin, glass, a membrane, a particle, a precipitate, a gel, a polymer, a sheet, a sphere, a polysaccharide, a capillary, a film, a plate, or a slide. The biological sample can be, for example, a cell culture, a cell line, a tissue, an oral tissue, gastrointestinal tissue, an organ, an organelle, a biological fluid, a blood sample, a urine sample, or a skin sample. The biological sample can be, for example, a skin biopsy.

### 4. DETAILED DESCRIPTION

The methods provided herein are based, in part, on the discovery that the presence and level of certain molecules or mRNAs in cell samples can be utilized as biomarkers to indicate the effectiveness or progress of a treatment for psoriasis. In particular, these biomarkers can be used to predict, assess and track the effectiveness of patient treatment or to monitor the patient's compliance to the treatment regimen.

### 4.1 Brief Description of Figures

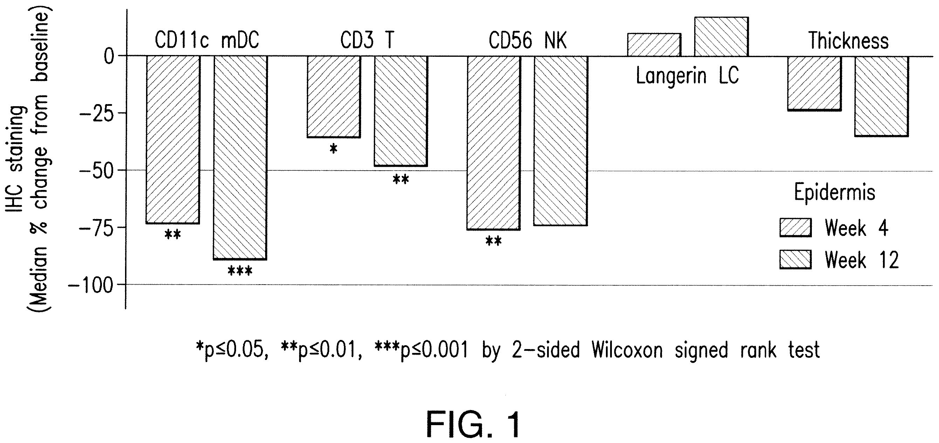

**FIG. 1** illustrates change in epidermal cells (CD11+ dendritic cells; CD3+ T cells; CD56+ NK cells; and Langerhans cells) at 4 weeks and 12 weeks after the administration of 20 mg of (S)-N-(2-(1-(3-ethoxy-4-methoxyphenyl)-2-(methylsulfonyl)ethyl)-1,3-dioxoisoindolin-4-yl)acetamide twice daily (b.i.d.).

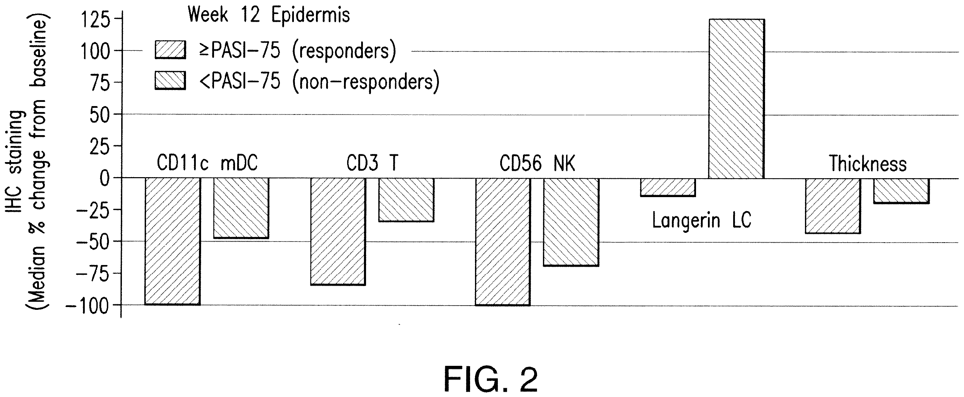

**FIG. 2** illustrates change in epidermal cells (CD11+ dendritic cells; CD3+ T cells; CD56+ NK cells; and Langerhans cells) at 12 weeks after the administration of 20 mg of (S)-N-(2-(1-(3-ethoxy-4-methoxyphenyl)-2-(methylsulfonyl)ethyl)-1,3-dioxoisoindolin-4-yl)acetamide twice daily (b.i.d.) in responders and non-responders.

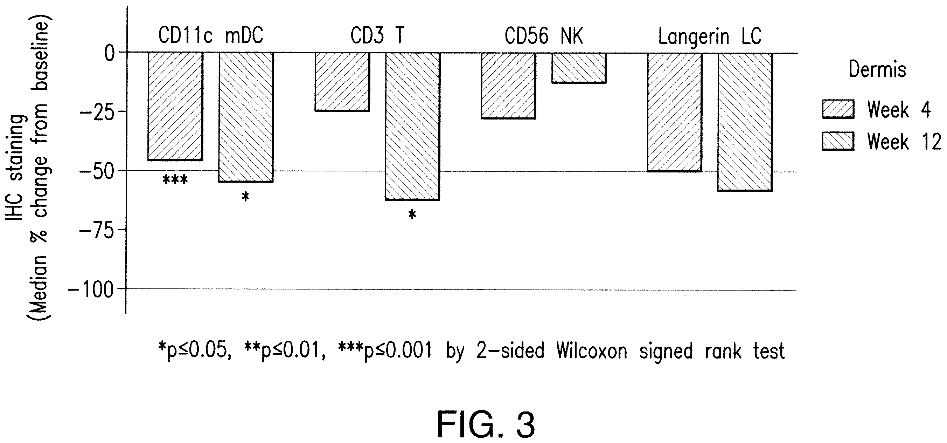

**FIG. 3** illustrates change in dermal cells (CD11+ dendritic cells; CD3+ T cells; CD56+ NK cells; and Langerhans cells) at 4 weeks and 12 weeks after the administration of 20 mg of (S)-N-(2-(1-(3-ethoxy-4-methoxyphenyl)-2-(methylsulfonyl)ethyl)-1,3-dioxoisoindolin-4-yl)acetamide twice daily (b.i.d.).

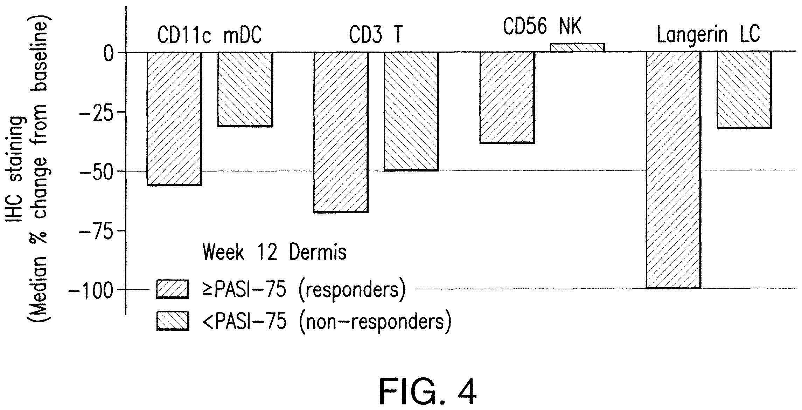

**FIG. 4** illustrates change in dermal cells (CD11+ dendritic cells; CD3+ T cells; CD56+ NK cells; and Langerhans cells) at 12 weeks after the administration of 20 mg of (S)-N-(2-(1-(3-ethoxy-4-methoxyphenyl)-2-(methylsulfonyl)ethyl)-1,3-dioxoisoindolin-4-yl)acetamide twice daily (b.i.d.) in responders and non-responders.

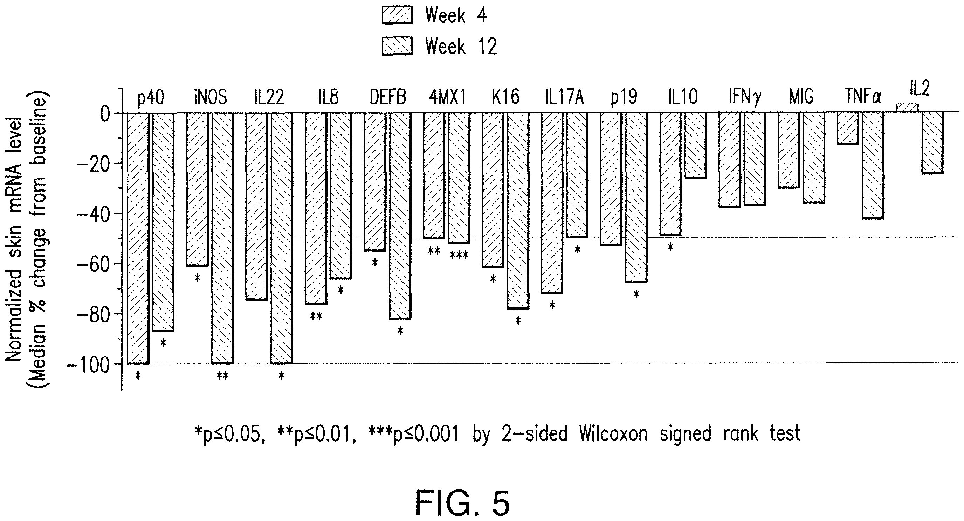

**FIG. 5** illustrates change in gene expression proinflammatory gene products associated with pathogenesis of psoriasis (IL-12/23 p40; iNOS; IL-22; IL-8; DEFB4; MX1; Keratin 16; IL-17A; IL-23 p19; IL-10; IFNγ; MIG; TNFα; and IL-2) at 4 weeks and 12 weeks after the administration of 20 mg of (S)-N-(2-(1-(3-ethoxy-4-methoxyphenyl)-2-(methylsulfonyl)ethyl)-1,3-dioxoisoindolin-4-yl)acetamide twice daily (b.i.d.).

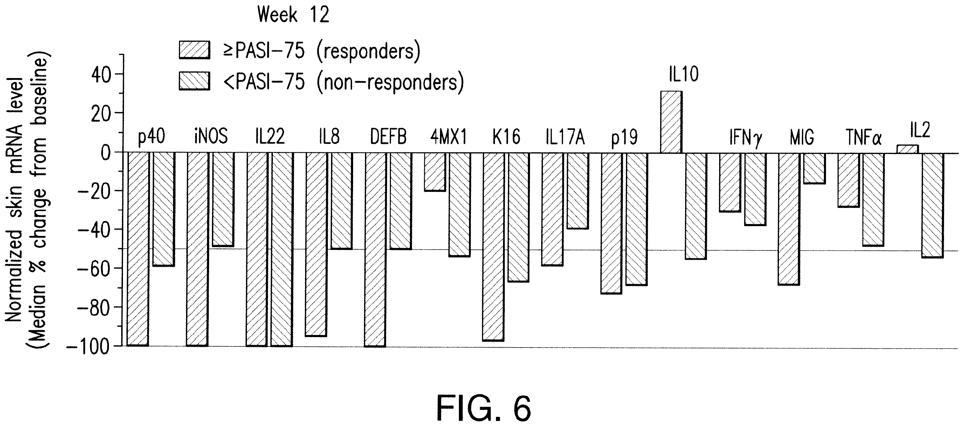

**FIG. 6** illustrates change in gene expression proinflammatory gene products associated with pathogenesis of psoriasis (IL-12/23 p40; iNOS; IL-22; IL-8; DEFB4; MX1; Keratin 16; IL-17A; IL-23 p19; IL-10; IFNγ; MIG; TNFα; and IL-2) at 12 weeks after the administration of 20 mg of (S)-N-(2-(1-(3-ethoxy-4-methoxyphenyl)-2-(methylsulfonyl)ethyl)-1,3-dioxoisoindolin-4-yl)acetamide twice daily (b.i.d.) in responders and non-responders.

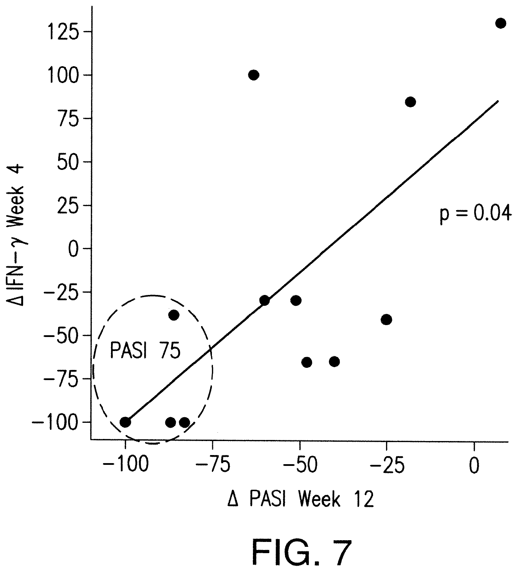

**FIG. 7** illustrates the correlation between change in IFNγ gene expression in psoriasis lesional skin at 4 weeks after the administration of 20 mg of (S)-N-(2-(1-(3-ethoxy-4-methoxyphenyl)-2-(methylsulfonyl)ethyl)-1,3-dioxoisoindolin-4-yl)acetamide twice daily (b.i.d.) and PASI score at 12 weeks after the administration of 20 mg of the same compound twice daily (b.i.d.).

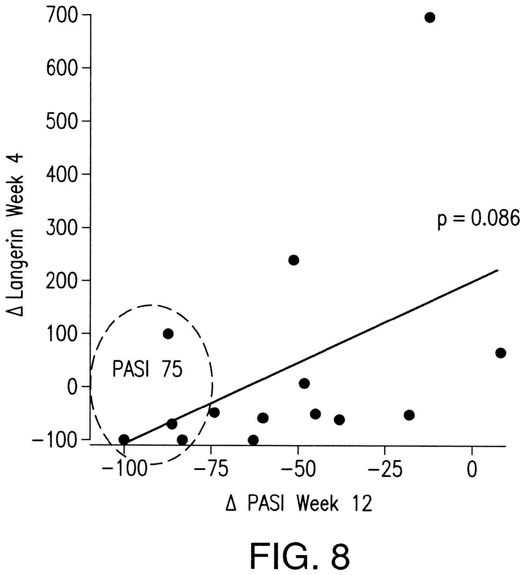

**FIG. 8** illustrates the correlation between change in Langerin staining in psoriasis lesional skin at 4 weeks after the administration of 20 mg of (S)-N-(2-(1-(3-ethoxy-4-methoxyphenyl)-2-(methylsulfonyl)ethyl)-1,3-dioxoisoindolin-4-yl)acetamide twice daily (b.i.d.) and PASI score at 12 weeks after the administration of 20 mg of the same compound twice daily (b.i.d.).

### 4.2 Definitions

As used herein, and unless otherwise specified, the terms "treat," "treating" and "treatment" refer to an action that occurs while a patient is suffering from psoriasis, which reduces the severity of psoriasis, or retards or slows the progression of the cancer.

The term "sensitivity" and "sensitive" when made in reference to treatment is a relative term which refers to the degree of effectiveness of a treatment compound in lessening or decreasing the symptoms of the disease being treated. For example, the term "increased sensitivity" when used in reference to treatment of a cell or patient refers to an increase of, at least a 5%, or more, in the effectiveness in lessening or decreasing the symptoms of psoriasis when measured using any methods well-accepted in the art.

As used herein, and unless otherwise specified, the term "therapeutically effective amount" of a compound is an amount sufficient to provide a therapeutic benefit in the treatment or management of psoriasis, or to delay or minimize one or more symptoms associated with psoriasis. A therapeutically effective amount of a compound means an amount of therapeutic agent, alone or in combination with other therapies, which provides a therapeutic benefit in the treatment or management of psoriasis. The term "therapeutically effective amount" can encompass an amount that improves overall therapy, reduces or avoids symptoms or causes of psoriasis, or enhances the therapeutic efficacy of another therapeutic agent.

As used herein, an "effective patient response" refers to any increase in the therapeutic benefit to the patient. An "effective patient tumor response" can be, for example, a 5%, 10%, 25%, 50%, or 100% decrease in the physical symptoms of psoriasis.

The term "likelihood" generally refers to an increase in the probability of an event. The term "likelihood" when used in reference to the effectiveness of a patient response generally contemplates an increased probability that the symptoms of psoriasis will be lessened or decreased.

The term "predict" generally means to determine or tell in advance. When used to "predict" the effectiveness of a psoriasis treatment, for example, the term "predict" can mean that the likelihood of the outcome of the treatment can be determined at the outset, before the treatment has begun, or before the treatment period has progressed substantially.

The term "monitor," as used herein, generally refers to the overseeing, supervision, regulation, watching, tracking, or surveillance of an activity. For example, the term "monitoring the efficacy of a treatment for psoriasis" refers to tracking the effectiveness in treating psoriasis in a patient or in a cell, usually obtained from a patient. Similarly, the term "monitoring," when used in connection with patient compliance, either individually, or in a clinical trial, refers to the tracking or confirming that the patient is actually following the treatment regimen being tested as prescribed.

As used herein the terms "polypeptide" and "protein" as used interchangeably herein, refer to a polymer of amino acids of three or more amino acids in a serial array, linked through peptide bonds. The term "polypeptide" includes proteins, protein fragments, protein analogues, oligopeptides and the like. The term polypeptide as used herein can also refer to a peptide. The amino acids making up the polypeptide may be naturally derived, or may be synthetic. The polypeptide can be purified from a biological sample.

The term "antibody" is used herein in the broadest sense and covers fully assembled antibodies, antibody fragments which retain the ability to specifically bind to the antigen (*e.g*., Fab, F(ab')2, Fv, and other fragments), single chain antibodies, diabodies, antibody chimeras, hybrid antibodies, bispecific antibodies, humanized antibodies, and the like. The term "antibody" covers both polyclonal and monoclonal antibodies.

The term "expressed" or "expression" as used herein refers to the transcription from a gene to give an RNA nucleic acid molecule at least complementary in part to a region of one of the two nucleic acid strands of the gene. The term "expressed" or "expression" as used herein also refers to the translation from the RNA molecule to give a protein, a polypeptide or a portion thereof.

An mRNA that is "upregulated" is generally "increased" upon a given treatment or condition. An mRNA that is "downregulated" generally refers to a "decrease" in the level of expression of the mRNA in response to a given treatment or condition. In some situations, the mRNA level can remain unchanged upon a given treatment or condition. An mRNA from a patient sample can be "upregulated," *i.e.,* the level of mRNA can be increased, for example, by about 5%, 10%, 20%, 30%, 40%, 50%, 60%, 70%, 90%, 100%, 200%, 300%, 500%, 1,000%, 5,000% or more of the comparative control mRNA level. Alternatively, an mRNA can be "downregulated," *i.e.,* the level of mRNA level can be decreased, for example, by about 99%, 95%, 90%, 80%, 70%, 60%, 50%, 40%, 30%, 20%, 10%, 1% or less of the comparative control mRNA level.

Similarly, the level of a polypeptide or protein biomarker from a patient sample can be increased as compared to a non-treated control. This increase can be about 5%, 10%, 20%, 30%, 40%, 50%, 60%, 70%, 90%, 100%, 200%, 300%, 500%, 1,000%, 5,000% or more of the comparative control protein level. Alternatively, the level of a protein biomarker can be decreased. This decrease can be, for example, present at a level of about 99%, 95%, 90%, 80%, 70%, 60%, 50%, 40%, 30%, 20%, 10%, 1% or less of the comparative control protein level.

The term "PDE4 modulators" refers to a molecule or compound that inhibits PDE4. In one embodiment, the PDE4 modulators can be those available from Celgene Corporation (provided herein elsewhere), which potently inhibit TNF-*α* production, but exhibit modest inhibitory effects on LPS induced IL-1β and IL-12, and do not inhibit IL-6 even at high drug concentrations. In addition, PDE4 inhibitors tend to produce a modest IL-10 stimulation. L.G. Corral, et al., Ann. Rheum. Dis., 58:(Suppl I) 1107-1113 (1999).

The terms "determining", "measuring", "evaluating", "assessing" and "assaying" as used herein generally refer to any form of measurement, and include determining if an element is present or not. These terms include both quantitative and/or qualitative determinations. Assessing may be relative or absolute. "Assessing the presence of' can include determining the amount of something present, as well as determining whether it is present or absent.

The terms "nucleic acid" and "polynucleotide" are used interchangeably herein to describe a polymer of any length composed of nucleotides, *e.g.,* deoxyribonucleotides or ribonucleotides, or compounds produced synthetically, which can hybridize with naturally occurring nucleic acids in a sequence specific manner analogous to that of two naturally occurring nucleic acids, *e.g.,* can participate in Watson-Crick base pairing interactions. As used herein in the context of a polynucleotide sequence, the term "bases" (or "base") is synonymous with "nucleotides" (or "nucleotide"), *i.e.,* the monomer subunit of a polynucleotide. The terms "nucleoside" and "nucleotide" are intended to include those moieties that contain not only the known purine and pyrimidine bases, but also other heterocyclic bases that have been modified. Such modifications include methylated purines or pyrimidines, acylated purines or pyrimidines, alkylated riboses or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, *e.g.,* wherein one or more of the hydroxyl groups are replaced with halogen atoms or aliphatic groups, or are functionalized as ethers, amines, or the like. "Analogues" refer to molecules having structural features that are recognized in the literature as being mimetics, derivatives, having analogous structures, or other like terms, and include, for example, polynucleotides incorporating non-natural nucleotides, nucleotide mimetics such as 2'-modified nucleosides, peptide nucleic acids, oligomeric nucleoside phosphonates, and any polynucleotide that has added substituent groups, such as protecting groups or linking moieties.

The term "complementary" refers to specific binding between polynucleotides based on the sequences of the polynucleotides. As used herein, a first polynucleotide and a second polynucleotide are complementary if they bind to each other in a hybridization assay under stringent conditions, *e.g.* if they produce a given or detectable level of signal in a hybridization assay. Portions of polynucleotides are complementary to each other if they follow conventional base-pairing rules, *e.g.* A pairs with T (or U) and G pairs with C, although small regions (*e.g.* less than about 3 bases) of mismatch, insertion, or deleted sequence may be present.

"Sequence identity" or "identity" in the context of two nucleic acid sequences refers to the residues in the two sequences which are the same when aligned for maximum correspondence over a specified comparison window, and can take into consideration additions, deletions and substitutions.

The term "substantial identity" or "homologous" in their various grammatical forms in the context of polynucleotides generally means that a polynucleotide comprises a sequence that has a desired identity, for example, at least 60% identity, preferably at least 70% sequence identity, more preferably at least 80%, still more preferably at least 90% and even more preferably at least 95%, compared to a reference sequence. Another indication that nucleotide sequences are substantially identical is if two molecules hybridize to each other under stringent conditions.

As used herein, the term "bound" can be used herein to indicate direct or indirect attachment. In the context of chemical structures, "bound" (or "bonded") may refer to the existence of a chemical bond directly joining two moieties or indirectly joining two moieties (*e.g.,* via a linking group or any other intervening portion of the molecule). The chemical bond may be a covalent bond, an ionic bond, a coordination complex, hydrogen bonding, van der Waals interactions, or hydrophobic stacking, or may exhibit characteristics of multiple types of chemical bonds. In certain instances, "bound" includes embodiments where the attachment is direct and also embodiments where the attachment is indirect.

The terms "isolated" and "purified" refer to isolation of a substance (such as mRNA or protein) such that the substance comprises a substantial portion of the sample in which it resides, *i.e.,* greater than the substance is typically found in its natural or un-isolated state. Typically, a substantial portion of the sample comprises, *e.g.,* greater than 1%, greater than 2%, greater than 5%, greater than 10%, greater than 20%, greater than 50%, or more, usually up to about 90%-100% of the sample. For example, a sample of isolated mRNA can typically comprise at least about 1% total mRNA. Techniques for purifying polynucleotides are well known in the art and include, for example, gel electrophoresis, ion-exchange chromatography, affinity chromatography, flow sorting, and sedimentation according to density.

The term "sample" as used herein relates to a material or mixture of materials, typically, although not necessarily, in fluid form, containing one or more components of interest.

"Biological sample" as used herein refers to a sample obtained from a biological subject, including sample of biological tissue or fluid origin, obtained, reached, or collected *in vivo* or *in situ.* A biological sample also includes samples from a region of a biological subject containing precancerous or cancer cells or tissues. Such samples can be, but are not limited to, organs, tissues, fractions and cells isolated from a mammal. Exemplary biological samples include but are not limited to cell lysate, a cell culture, a cell line, a tissue, oral tissue, gastrointestinal tissue, an organ, an organelle, a biological fluid, a blood sample, a urine sample, a skin sample, and the like. Preferred biological samples include but are not limited to whole blood, partially purified blood, PBMCs, tissue biopsies, and the like.

The term "analyte" as used herein, refers to a known or unknown component of a sample.

The term "capture agent," as used herein, refers to an agent that binds an mRNA or protein through an interaction that is sufficient to permit the agent to bind and concentrate the mRNA or protein from a homogeneous mixture.

The term "probe" as used herein, refers to a capture agent that is directed to a specific target mRNA biomarker sequence. Accordingly, each probe of a probe set has a respective target mRNA biomarker. A probe/target mRNA duplex is a structure formed by hybridizing a probe to its target mRNA biomarker.

The term "nucleic acid" or "oligonucleotide probe" refers to a nucleic acid capable of binding to a target nucleic acid of complementary sequence, such as the mRNA biomarkers provided herein, through one or more types of chemical bonds, usually through complementary base pairing, usually through hydrogen bond formation. As used herein, a probe may include natural (*e.g.,* A, G, C, or T) or modified bases (7-deazaguanosine, inosine, etc.). In addition, the bases in a probe may be joined by a linkage other than a phosphodiester bond, so long as it does not interfere with hybridization. It will be understood by one of skill in the art that probes may bind target sequences lacking complete complementarity with the probe sequence depending upon the stringency of the hybridization conditions. The probes are preferably directly labeled with isotopes, for example, chromophores, lumiphores, chromogens, or indirectly labeled with biotin to which a streptavidin complex may later bind. By assaying for the presence or absence of the probe, one can detect the presence or absence of a target mRNA biomarker of interest.

The term "stringent assay conditions" refers to conditions that are compatible to produce binding pairs of nucleic acids, *e.g.,* probes and target mRNAs, of sufficient complementarity to provide for the desired level of specificity in the assay while being generally incompatible to the formation of binding pairs between binding members of insufficient complementarity to provide for the desired specificity. The term "stringent assay conditions"generally refers to the combination of hybridization and wash conditions.

A "label" or a "detectable moiety" in reference to a nucleic acid, refers to a composition that, when linked with a nucleic acid, renders the nucleic acid detectable, for example, by spectroscopic, photochemical, biochemical, immunochemical, or chemical means. Exemplary labels include, but are not limited to, radioactive isotopes, magnetic beads, metallic beads, colloidal particles, fluorescent dyes, enzymes, biotin, digoxigenin, haptens, and the like. A "labeled nucleic acid or oligonucleotide probe" is generally one that is bound, either covalently, through a linker or a chemical bond, or noncovalently, through ionic bonds, van der Waals forces, electrostatic attractions, hydrophobic interactions, or hydrogen bonds, to a label such that the presence of the nucleic acid or probe can be detected by detecting the presence of the label bound to the nucleic acid or probe.

The terms "polymerase chain reaction," or "PCR," as used herein generally refers to a procedure wherein small amounts of a nucleic acid, RNA and/or DNA, are amplified as described, for example, in U.S. Pat. No. 4,683,195 to Mullis. Generally, sequence information from the ends of the region of interest or beyond needs to be available, such that oligonucleotide primers can be designed; these primers will be identical or similar in sequence to opposite strands of the template to be amplified. The 5' terminal nucleotides of the two primers may coincide with the ends of the amplified material. PCR can be used to amplify specific RNA sequences, specific DNA sequences from total genomic DNA, and cDNA transcribed from total cellular RNA, bacteriophage or plasmid sequences, etc. *See generally* Mullis et al., Cold Spring Harbor Symp. Quant. Biol., 51: 263 (1987); Erlich, ed., PCR Technology, (Stockton Press, NY, 1989).

The term "cycle number" or "CT" when used herein in reference to PCR methods, refers to the PCR cycle number at which the fluorescence level passes a given set threshold level. The CT measurement can be used, for example, to approximate levels of mRNA in an original sample. The CT measurement is often used in terms of "dCT" or the "difference in the CT" score, when the CT of one nucleic acid is subtracted from the CT of another nucleic acid.

As used herein, and unless otherwise indicated, the term "optically pure" means a composition that comprises one optical isomer of a compound and is substantially free of other isomers of that compound. For example, an optically pure composition of a compound having one chiral center will be substantially free of the opposite enantiomer of the compound. An optically pure composition of a compound having two chiral centers will be substantially free of other diastereomers of the compound. A typical optically pure compound comprises greater than about 80% by weight of one enantiomer of the compound and less than about 20% by weight of other enantiomers of the compound, more preferably greater than about 90% by weight of one enantiomer of the compound and less than about 10% by weight of the other enantiomers of the compound, even more preferably greater than about 95% by weight of one enantiomer of the compound and less than about 5% by weight of the other enantiomers of the compound, more preferably greater than about 97% by weight of one enantiomer of the compound and less than about 3% by weight of the other enantiomers of the compound, and most preferably greater than about 99% by weight of one enantiomer of the compound and less than about 1% by weight of the other enantiomers of the compound.

The practice of the embodiments provided herein will employ, unless otherwise indicated, conventional techniques of molecular biology, microbiology, and immunology, which are within the skill of those working in the art. Such techniques are explained fully in the literature. Examples of particularly suitable texts for consultation include the following: Sambrook et al. (1989) Molecular Cloning; A Laboratory Manual (2d ed.); D.N Glover, ed. (1985) DNA Cloning, Volumes I and II; M.J. Gait, ed. (1984) Oligonucleotide Synthesis*;* B.D. Hames & SJ. Higgins, eds. (1984) Nucleic Acid Hybridization; B.D. Hames & S.J. Higgins, eds. (1984) Transcription and Translation*;* R.I. Freshney, ed. (1986) Animal Cell Culture; Immobilized Cells and Enzymes (IRL Press, 1986); Immunochemical Methods in Cell and Molecular Biology (Academic Press, London); Scopes (1987) Protein Purification: Principles and Practice (2d ed.; Springer Verlag, N.Y.); and D.M. Weir and C. C. Blackwell, eds. (1986) Handbook of Experimental Immunology, Volumes I-IV.

### 4.3 Biomarkers

Provided herein are methods relating to the use of cell marker molecules and mRNAs as biomarkers to predict or ascertain the efficacy of a treatment for psoriasis. Cell markers or mRNA levels can be used to determine whether a treatment is likely to be successful in cell models of disease.

A biological marker or "biomarker" is a substance whose detection indicates a particular biological state, such as, for example, the progress of psoriasis. In some embodiments, biomarkers can either be determined individually, or several biomarkers can be measured simultaneously.

In some embodiments, a "biomarker" indicates a change in the level of nucleic acid expression that may correlate with the risk or progression of a disease, or with the susceptibility of the disease to a given treatment. In some embodiments, the biomarker is a mRNA or cDNA.

In additional embodiments, a "biomarker" indicates a change in the level of certain cell markers that may correlate with the risk, susceptibility to treatment, or progression of a disease. The relative level of specific cell markers can be determined by methods known in the art. For example, antibody based methods, such as an immunoblot, enzyme-linked immunosorbent assay (ELISA), or other methods can be used.

### 4.3.1 Use of cell markers as biomarkers for predicting the efficacy

Based, in part, on the finding that detectable increase or decrease in certain cell markers are observed during the psoriasis treatment, the levels of these cell markers may be used as a biomarker for predicting the sensitivity of a potential psoriasis treatment. The cell markers include CD3, CD56, Langerin and Foxp3. Each of these biomarkers may be monitored separately, or two or more of the biomarkers may be simultaneously monitored.

In some embodiments, these biomarkers can be used to predict the effectiveness of a psoriasis treatment in a patient. In one embodiment, the level of the biomarker is measured in a biological sample obtained from a potential patient. Alternatively, the cell markers can also be used as a biomarker for an *in vitro* assay to predict the success of a psoriasis treatment, by taking a sample of cells from the patient, culturing them in the presence or absence of the treatment compound, and testing the cells for an increase or decrease in the levels of the biomarkers.

Thus, in one embodiment, provided herein is a method of predicting whether a patient will be responsive to a treatment for psoriasis comprising:
culturing cells from a skin sample of the patient in the presence or absence of the treatment compound;
measuring the level of a cell marker selected from CD3, CD56, Foxp3 and a combination thereof; and
comparing the level of the cell marker in the cells cultured in the presence of the treatment compound to that in cells cultured in the absence of the treatment compound;
wherein a decreased level of the cell marker in the presence of the treatment compound indicates the likelihood of an effective patient response to the treatment compound.

In one embodiment, the level of only one of the cell markers is monitored. In another embodiment, the levels of two or more of the cell markers are monitored simultaneously.

In one described aspect, the sample cells from patient are obtained using a skin biopsy. In one described aspect, the sample cells from patient are obtained from the dermis region of the patient. In another described aspect, the sample cells from patient are obtained from the epidermis region of the patient.

In one embodiment, the treatment compound is a PDE4 inhibitor provided herein elsewhere. In another described aspect, the treatment compound is (S)-N-(2-(1-(3-ethoxy-4-methoxyphenyl)-2-(methylsulfonyl)ethyl)-1,3-dioxoisoindolin-4-yl)acetamide. In another embodiment, the treatment compound is cyclopropanecarboxylic acid {2-[(*1S*)-1-(3-ethoxy-4-methoxy-phenyl)-2-methanesulfonyl-ethyl]-3-oxo-2,3-dihydro-1*H-*isoindol-4-yl}-amide.

In another aspect, described herein is a method of predicting whether a patient will be responsive to a treatment for psoriasis comprising:
culturing the cells from the epidermis region of the patient the presence or absence of the treatment compound;
measuring the level of Langerin in the cells; and
comparing the level of Langerin in the cells cultured in the presence of the treatment compound to that in cells cultured in the absence of the treatment compound;
wherein a decreased level of Langerin in the presence of the treatment compound indicates the likelihood of an effective patient response to the treatment compound.

In one described aspect, the sample cells from patient are obtained using a skin biopsy. In one described aspect, the sample cells from patient are obtained from the dermis region of the patient. In another described aspect, the sample cells from patient are obtained from the epidermis region of the patient.

In one described aspect, the treatment compound is a PDE4 inhibitor provided herein elsewhere. In another described aspect, the treatment compound is (S)-N-(2-(1-(3-ethoxy-4-methoxyphenyl)-2-(methylsulfonyl)ethyl)-1,3-dioxoisoindolin-4-yl)acetamide. In another embodiment, the treatment compound is cyclopropanecarboxylic acid {2-[(*1S*)-1-(3-ethoxy-4-methoxy-phenyl)-2-methanesulfonyl-ethyl]-3-oxo-2,3-dihydro-1*H-*isoindol-4-yl} -amide.

### 4.3.2 Use of cell markers as biomarkers for monitoring the efficacy or patient compliance

In addition to the initial prediction of the likelihood of treatment effectiveness in a patient with psoriasis, the progress of a psoriasis treatment can be followed by monitoring the levels of the cell markers described above. Thus, in some embodiments, a method of assessing or monitoring the effectiveness of a psoriasis treatment in a patient is provided. A sample is obtained from the patient, and the levels of one or more of the above-described biomarkers are measured to determine whether their levels are increased or decreased compared to the levels prior to the initiation of the treatment.

The biomarkers can also be used to track and adjust individual patient treatment effectiveness. The biomarkers can be used to gather information needed to make adjustments in a patient's treatment, increasing or decreasing the dose of an agent as needed. For example, a patient receiving a treatment compound can be tested using a biomarker to see if the dosage is becoming effective, or if a more aggressive treatment plan may be needed.

In one embodiment, provided herein is a method of monitoring patient response to a psoriasis treatment comprising:
measuring the level of a cell marker selected from CD3, CD56, Foxp3, Langerin and a combination thereof, in a first skin sample from the patient,
measuring the level of a cell marker selected from CD3, CD56, Foxp3, Langerin and a combination thereof, in a second skin sample obtained after administering a treatment compound to the patient; and
comparing the levels of the cell marker obtained from first and second skin samples;
wherein a decreased level of the cell marker in the second skin sample indicates an effective response.

Patients can submit the cell sample by any desired means, such as, for example, a skin biopsy. Samples can be taken, for example, every day, once per week, twice per month, once a month, once every two months, quarterly, or yearly, as needed to follow the effectiveness of the treatment. In one embodiment, the tests are conducted once in 4 weeks after the treatment and once in 12 weeks after the treatment. By following the levels of these biomarkers, the treatment effectiveness can be monitored over time.

In one embodiment, the level of only one of the cell markers is monitored. In another embodiment, the levels of two or more of the cell markers are monitored simultaneously.

In one described aspect, the level of CD11c obtained from the dermis region is monitored, and the decrease is about 30%, 35%, 40%, or 45% or more as compared to the level of CD11c in the first biological sample. In another described aspect the level of CD11c obtained from the epidermis region is monitored, and the decrease is about 60%, 65%, 70%, or 75% or more as compared to the level of CD11c in the first biological sample.

In one embodiment, the level of CD3 obtained from the dermis region is monitored, and the decrease is about 15%, 20%, 25%, or 30% or more as compared to the level of CD3 in the first skin sample. In another embodiment, the level of CD3 obtained from the epidermis region is monitored, and the decrease is about 25%, 30%, 35%, or 40% or more as compared to the level of CD3 in the first skin sample.

In one embodiment, the level of CD56 obtained from the dermis region is monitored, and the decrease is about 15%, 20%, 25%, or 30% or more as compared to the level of CD56 in the first skin sample. In another embodiment, the level of CD56 obtained from the epidermis region is monitored, and the decrease is about 60%, 65%, 70%, or 75% or more as compared to the level of CD56 in the first skin sample.

In one embodiment, the level of Langerin obtained from the dermis region is monitored, and the decrease is about 40%, 45%, 50%, or 55% or more as compared to the level of Langerin in the first skin sample.

In one described aspect, the sample cells from patient are obtained using a skin biopsy. In one described aspect, the sample cells from patient are obtained from the dermis region of the patient. In another described aspect, the sample cells from patient are obtained from the epidermis region of the patient.

In one described aspect, the treatment compound is a provided herein elsewhere. In another described aspect, the treatment compound is (S)-N-(2-(1-(3-ethoxy-4-methoxyphenyl)-2-(methylsulfonyl)ethyl)-1,3-dioxoisoindolin-4-yl)acetamide. In another embodiment, the treatment compound is cyclopropanecarboxylic acid {2-[(*1S*)-1-(3-ethoxy-4-methoxy-phenyl)-2-methanesulfonyl-ethyl]-3-oxo-2,3-dihydro-1*H-*isoindol-4-yl} -amide.

In another embodiment, provided herein is a method of to a psoriasis treatment comprising:
measuring the level of Langerin in a first skin sample from the epidermis region of the patient,
measuring the level of Langerin in a second skin sample obtained from the epidermis region of the patient after administering a treatment compound to the patient, and
comparing the levels of Langerin obtained from first and second skin samples;
wherein a decreased level of Langerin in the second skin sample indicates an effective response.

In one embodiment, the decrease in the level of Langerin is amount 5%, 10%, 15%, or 20% as compared to the level of Langerin in the first skin sample.

Again, patients can submit the cell sample by any desired means, such as, for example, a skin biopsy. Samples can be taken, for example, every day, once per week, twice per month, once a month, once every two months, quarterly, or yearly, as needed to follow the effectiveness of the treatment. In one embodiment, the tests are conducted once in 4 weeks after the treatment and once in 12 weeks after the treatment. By following the levels of these biomarkers, the treatment effectiveness can be monitored over time. In one embodiment, the sample cells from patient are obtained using a skin biopsy.

In one described aspect, the treatment compound is a PDE4 inhibitor provided herein elsewhere. In another described aspect, the treatment compound is (S)-N-(2-(1-(3-ethoxy-4-methoxyphenyl)-2-(methylsulfonyl)ethyl)-1,3-dioxoisoindolin-4-yl)acetamide. In another embodiment, the treatment compound is cyclopropanecarboxylic acid {2-[(*1S*)-1-(3-ethoxy-4-methoxy-phenyl)-2-methanesulfonyl-ethyl]-3-oxo-2,3-dihydro-1*H-*isoindol-4-yl} -amide.

In other embodiments, these biomarkers can additionally be used to track or perform quality control on human research trials or to monitor the patient compliance to a drug regimen by providing a means to confirm that the patient is receiving specific drug treatments. These biomarkers can be used in connection with, for example, the management of patient treatment, clinical trials, and cell-based research.

In one embodiment, these biomarkers can be used to track patient compliance during individual treatment regimes, or during clinical trials. For example, the levels of biomarkers can be followed at set intervals during a clinical trial to ensure that the patients included in the trial are taking the drugs as instructed. The treatment of individual patients can also be followed using the procedure. For example, when the level of a particular biomarker is measured, an altered level of the biomarker compared to that of an untreated control indicates at least partial patient compliance with the drug treatment protocol. An altered level of the biomarker that is at a similar quantity to that of a positive control indicates the likelihood of full compliance with the treatment protocol.

Thus, in some embodiments, a method for assessing patient compliance with a drug treatment protocol is provided. A biological sample is obtained from the patient, and the levels of the biomarkers are measured and compared to that of a control untreated sample. An altered levels of biomarkers compared to those of an untreated control sample indicates compliance with the protocol.

In one embodiment, provided herein is a method for monitoring patient compliance with a drug treatment protocol for psoriasis, comprising:
measuring the level of a cell marker selected from CD3, CD56, Foxp3, Langerin and a combination thereof, in a skin sample from the patient, and
determining if the expression level is decreased in the skin sample compared to the expression level in a control untreated sample;
wherein a decreased expression indicates patient compliance with said drug treatment protocol.

Patients can submit the cell sample by any desired means, such as, for example, a skin biopsy. Samples can be taken, for example, every day, once per week, twice per month, once a month, once every two months, quarterly, or yearly, as needed to follow the effectiveness of the treatment. By following the levels of these biomarkers, the patient compliance can be monitored over time.

In one embodiment, the level of only one of the cell markers is monitored. In another embodiment, the levels of two or more of the cell markers are monitored simultaneously.

In one described aspect, the level of CD11c obtained from the dermis region is monitored, and the decrease is about 30%, 35%, 40%, or 45% or more as compared to the level of CD11c in the first biological sample. In another described aspect, the level of CD11c obtained from the epidermis region is monitored, and the decrease is about 60%, 65%, 70%, or 75% or more as compared to the level of CD11c in the first biological sample.

In one embodiment, the level of CD3 obtained from the dermis region is monitored, and the decrease is about 15%, 20%, 25%, or 30% or more as compared to the level of CD3 in the first skin sample. In another embodiment, the level of CD3 obtained from the epidermis region is monitored, and the decrease is about 25%, 30%, 35%, or 40% or more as compared to the level of CD3 in the first skin sample.

In one embodiment, the level of CD56 obtained from the dermis region is monitored, and the decrease is about 15%, 20%, 25%, or 30% or more as compared to the level of CD56 in the first skin sample. In another embodiment, the level of CD56 obtained from the epidermis region is monitored, and the decrease is about 60%, 65%, 70%, or 75% or more as compared to the level of CD56 in the first skin sample.

In one embodiment, the level of Langerin obtained from the dermis region is monitored, and the decrease is about 40%, 45%, 50%, or 55% or more as compared to the level of Langerin in the first skin sample.

In one described aspect, the sample cells from patient are obtained using a skin biopsy. In one described aspect, the sample cells from patient are obtained from the dermis region of the patient. In another described aspect, the sample cells from patient are obtained from the epidermis region of the patient.

In one described aspect, the treatment compound is a PDE4 inhibitor provided herein elsewhere. In another described aspect, the treatment compound is (S)-N-(2-(1-(3-ethoxy-4-methoxyphenyl)-2-(methylsulfonyl)ethyl)-1,3-dioxoisoindolin-4-yl)acetamide. In another embodiment, the treatment compound is cyclopropanecarboxylic acid {2-[(*1S*)-1-(3-ethoxy-4-methoxy-phenyl)-2-methanesulfonyl-ethyl]-3-oxo-2,3-dihydro-1*H-*isoindol-4-yl} -amide.

In another embodiment, provided herein is a method for with a drug treatment protocol for psoriasis, comprising:
measuring the level of Langerin in a skin sample from the epidermis region of said patient, and
determining if the expression level is decreased in the skin sample compared to the expression level in a control untreated sample;
wherein a decreased expression indicates patient compliance with said drug treatment protocol.

In one described aspect, the decrease in the level of Langerin is amount 5%, 10%, 15%, or 20% as compared to the level of Langerin in the first skin sample.

Again, patients can submit the cell sample by any desired means, such as, for example, a skin biopsy. Samples can be taken, for example, every day, once per week, twice per month, once a month, once every two months, quarterly, or yearly, as needed to follow the effectiveness of the treatment. In one described aspect, the tests are conducted once in 4 weeks after the treatment and once in 12 weeks after the treatment. By following the levels of these biomarkers, the treatment effectiveness can be monitored over time. In one described aspect, the sample cells from patient are obtained using a skin biopsy.

In one described aspect, the treatment compound is a PDE4 inhibitor provided herein elsewhere. In another described aspect, the treatment compound is (S)-N-(2-(1-(3-ethoxy-4-methoxyphenyl)-2-(methylsulfonyl)ethyl)-1,3-dioxoisoindolin-4-yl)acetamide. In another embodiment, the treatment compound is cyclopropanecarboxylic acid {2-[(*1S*)-1-(3-ethoxy-4-methoxy-phenyl)-2-methanesulfonyl-ethyl]-3-oxo-2,3-dihydro-1*H-*isoindol-4-yl}-amide.

### 4.3.3 Use of mRNAs as biomarkers for predicting the efficacy

Based, in part, on the finding that detectable decrease in certain mRNAs are observed during the psoriasis treatment, the levels of these mRNAs may be used as a biomarker for predicting the sensitivity of a potential psoriasis treatment. The mRNAs include, but are not limited to, mRNAs for Keratin 16, iNOS, IL-12/IL-23 p40, IL-23 p19, IL-17A, IL-22, DEFB4, IL-8, MX-1, IL-10, IFN-γ and/or CXCL9. Each of these biomarkers may be monitored separately, or two or more of the biomarkers may be simultaneously monitored.

In some embodiments, these biomarkers can be used to predict the effectiveness of a psoriasis treatment in a patient. In one embodiment, the level of the biomarker is measured in a biological sample obtained from a potential patient. Alternatively, the cell markers can also be used as a biomarker for an *in vitro* assay to predict the success of a psoriasis treatment, by taking a sample of cells from the patient, culturing them in the presence or absence of the treatment compound, and testing the cells for an increase or decrease in the levels of the biomarkers.

Thus, in one embodiment, provided herein is a method of predicting whether a patient will be responsive to a treatment for psoriasis comprising:
culturing cells from a skin sample of the patient in the presence or absence of the treatment compound;
measuring the level of an mRNA selected from mRNA for Keratin 16, IL-12/IL-23 p40, IL-23 p19, IL-17A, IL-22, DEFB4, IL-8, MX-1, IL-10, IFN-γ, CXCL9 and a combination thereof; and
comparing the level of the mRNA in the cells cultured in the presence of the treatment compound to that in cells cultured in the absence of the treatment compound;
wherein a decreased level of the mRNA in the presence of the treatment compound indicates the likelihood of an effective patient response to the treatment compound.

In one embodiment, the level of only one of the mRNA is monitored. In another embodiment, the levels of two or more of the mRNAs are monitored simultaneously.

In one described aspect, the treatment compound is a PDE4 inhibitor provided herein elsewhere. In another described aspect, the treatment compound is (S)-N-(2-(1-(3-ethoxy-4-methoxyphenyl)-2-(methylsulfonyl)ethyl)-1,3-dioxoisoindolin-4-yl)acetamide. In another embodiment, the treatment compound is cyclopropanecarboxylic acid {2-[(*1S*)-1-(3-ethoxy-4-methoxy-phenyl)-2-methanesulfonyl-ethyl]-3-oxo-2,3-dihydro-1*H-*isoindol-4-yl}-amide.

### 4.3.4 Use of mRNAs as biomarkers for monitoring the efficacy or patient compliance

In addition to the initial prediction of the likelihood of treatment effectiveness in a patient with psoriasis, the progress of a psoriasis treatment can be followed by monitoring the levels of the mRNAs described above. Thus, in some embodiments, a method of assessing or monitoring the effectiveness of a psoriasis treatment in a patient is provided. A sample is obtained from the patient, and the levels of one or more of the above-described mRNAs are measured to determine whether their levels are increased or decreased compared to the levels prior to the initiation of the treatment.

The biomarkers can also be used to track and adjust individual patient treatment effectiveness. The biomarkers can be used to gather information needed to make adjustments in a patient's treatment, increasing or decreasing the dose of an agent as needed. For example, a patient receiving a treatment compound can be tested using a biomarker to see if the dosage is becoming effective, or if a more aggressive treatment plan may be needed.

In one embodiment, provided herein is a method of monitoring patient response to a psoriasis treatment comprising:
measuring the level of an mRNA selected from mRNA for Keratin 16, IL-12/IL-23 p40, IL-23 p19, IL-17A, IL-22, DEFB4, IL-8, MX-1, IL-10, IFN-γ, CXCL9 and a combination thereof, in a first skin sample from the patient,
measuring the level of an mRNA selected from mRNA for Keratin 16, IL-12/IL-23 p40, IL-23 p19, IL-17A, IL-22, DEFB4, IL-8, MX-1, IL-10, IFN-γ, CXCL9 and a combination thereof, in a second skin sample obtained after administering a treatment compound to the patient, and
comparing the levels of the mRNA obtained from first and second skin samples;
wherein a decreased level of the mRNA in the second skin sample indicates an effective response.

Patients can submit the cell sample by any desired means, such as, for example, a skin biopsy. Samples can be taken, for example, every day, once per week, twice per month, once a month, once every two months, quarterly, or yearly, as needed to follow the effectiveness of the treatment. In one embodiment, the tests are conducted once in 4 weeks after the treatment and once in 12 weeks after the treatment. By following the levels of these biomarkers, the treatment effectiveness can be monitored over time.

In one embodiment, the level of only one of the mRNA is monitored. In another embodiment, the levels of two or more of the mRNAs are monitored simultaneously.

In one embodiment, the level of IL-12/IL-13 p40 mRNA is monitored, and the decrease is about 80%, 85% 90%, or 95% or more as compared to the level of IL-12/IL-13 p40 mRNA in the first skin sample.

In another described aspect, the level of iNOS mRNA is monitored, and the decrease is about 50%, 55%, 60%, or 65% or more as compared to the level of iNOS mRNA in the first biological sample.

In one embodiment, the level of IL-22 mRNA is monitored, and the decrease is about 65%, 70%, 75%, or 80% or more as compared to the level of IL-22 mRNA in the first skin sample.

In another embodiment, the level of IL-8 mRNA is monitored, and the decrease is about 65%, 70%, 75%, or 80% or more as compared to the level of IL-8 mRNA in the first skin sample.

In one embodiment, the level of DEFB4 mRNA is monitored, and the decrease is about 45%, 50%, 55%, or 60% or more as compared to the level of DEFB4 mRNA in the first skin sample.

In another embodiment, the level of MX-1 mRNA is monitored, and the decrease is about 40%, 45%, 50%, or 55% or more as compared to the level of MX-1 mRNA in the first skin sample.

In one embodiment, the level of Keratin 16 mRNA is monitored, and the decrease is about 50%, 55%, 60%, or 65% or more as compared to the level of Keratin 16 mRNA in the first skin sample.

In one embodiment, the level of IL-17A mRNA is monitored, and the decrease is about 60%, 65%, 70%, or 75% or more as compared to the level of IL-17A mRNA in the first skin sample.

In one embodiment, the level of IL-23 p19 mRNA is monitored, and the decrease is about 40% 45%, 50%, or 55% or more as compared to the level of IL-23 p19 mRNA in the first skin sample.

In one embodiment, the level of IL-10 mRNA is monitored, and the decrease is about 40%, 45%, 50%, or 55% or more as compared to the level of IL-10 mRNA in the first skin sample.

In one embodiment, the level of IFN-γ mRNA is monitored, and the decrease is about 30%, 35%, 40%, or 45% or more as compared to the level of IFN-γ mRNA in the first skin sample.

In one embodiment, the level of CXCL9 mRNA is monitored, and the decrease is about 20%, 25%, 30%, or 35% or more as compared to the level of CXCL9 mRNA in the first skin sample.

In one described aspect, the treatment compound is a PDE4 inhibitor provided herein elsewhere. In another described aspect, the treatment compound is (S)-N-(2-(1-(3-ethoxy-4-methoxyphenyl)-2-(methylsulfonyl)ethyl)-1,3-dioxoisoindolin-4-yl)acetamide. In another embodiment, the treatment compound is cyclopropanecarboxylic acid {2-[(*1S*)-1-(3-ethoxy-4-methoxy-phenyl)-2-methanesulfonyl-ethyl]-3-oxo-2,3-dihydro-1*H-*isoindol-4-yl} -amide.

In other embodiments, these biomarkers can additionally be used to track or perform quality control on human research trials or to monitor the patient compliance to a drug regimen by providing a means to confirm that the patient is receiving specific drug treatments. These biomarkers can be used in connection with, for example, the management of patient treatment, clinical trials, and cell-based research.

In one embodiment, these biomarkers can be used to track patient compliance during individual treatment regimes, or during clinical trials. For example, the levels of biomarkers can be followed at set intervals during a clinical trial to ensure that the patients included in the trial are taking the drugs as instructed. The treatment of individual patients can also be followed using the procedure. For example, when the level of a particular biomarker is measured, an altered level of the biomarker compared to that of an untreated control indicates at least partial patient compliance with the drug treatment protocol. An altered level of the biomarker that is at a similar quantity to that of a positive control indicates the likelihood of full compliance with the treatment protocol.

Thus, in some embodiments, a method for assessing patient compliance with a drug treatment protocol is provided. A biological sample is obtained from the patient, and the levels of the biomarkers are measured and compared to that of a control untreated sample. An altered levels of biomarkers compared to those of an untreated control sample indicates compliance with the protocol.

In one embodiment, provided herein is a method for with a drug treatment protocol for psoriasis, comprising:
measuring the level of an mRNA selected from mRNA for Keratin 16, IL-12/IL-23 p40, IL-23 p19, IL-17A, IL-22, DEFB4, IL-8, MX-1, IL-10, IFN-γ, CXCL9 and a combination thereof, in a skin sample from the patient and
determining if the expression level is decreased in the skin sample compared to the expression level in a control untreated sample;
wherein a decreased expression indicates patient compliance with said drug treatment protocol.

Patients can submit the cell sample by any desired means, such as, for example, a skin biopsy. Samples can be taken, for example, every day, once per week, twice per month, once a month, once every two months, quarterly, or yearly, as needed to follow the effectiveness of the treatment. By following the levels of these biomarkers, the patient compliance can be monitored over time.

In one embodiment, the level of only one of the cell markers is monitored. In another embodiment, the levels of two or more of the cell markers are monitored simultaneously.

In one embodiment, the level of IL-12/IL-13 p40 mRNA is monitored, and the decrease is about 80%, 85% 90%, or 95% or more as compared to the level of IL-12/IL-13 p40 mRNA in the first skin sample.

In another described aspect, the level of iNOS mRNA is monitored, and the decrease is about 50%, 55%, 60%, or 65% or more as compared to the level of iNOS mRNA in the first biological sample.

In one embodiment, the level of IL-22 mRNA is monitored, and the decrease is about 65%, 70%, 75%, or 80% or more as compared to the level of IL-22 mRNA in the first skin sample.

In another embodiment, the level of IL-8 mRNA is monitored, and the decrease is about 65%, 70%, 75%, or 80% or more as compared to the level of IL-8 mRNA in the first skin sample.

In one embodiment, the level of DEFB4 mRNA is monitored, and the decrease is about 45%, 50%, 55%, or 60% or more as compared to the level of DEFB4 mRNA in the first skin sample.

In another embodiment, the level of MX-1 mRNA is monitored, and the decrease is about 40%, 45%, 50%, or 55% or more as compared to the level of MX-1 mRNA in the first skin sample.

In one embodiment, the level of Keratin 16 mRNA is monitored, and the decrease is about 50%, 55%, 60%, or 65% or more as compared to the level of Keratin 16 mRNA in the first skin sample.

In one embodiment, the level of IL-17A mRNA is monitored, and the decrease is about 60%, 65%, 70%, or 75% or more as compared to the level of IL-17A mRNA in the first skin sample.

In one embodiment, the level of IL-23 p19 mRNA is monitored, and the decrease is about 40% 45%, 50%, or 55% or more as compared to the level of IL-23 p19 mRNA in the first skin sample.

In one embodiment, the level of IL-10 mRNA is monitored, and the decrease is about 40%, 45%, 50%, or 55% or more as compared to the level of IL-10 mRNA in the first skin sample.

In one embodiment, the level of IFN-γ mRNA is monitored, and the decrease is about 30%, 35%, 40%, or 45% or more as compared to the level of IFN-γ mRNA in the first skin sample.

In one embodiment, the level of CXCL9 mRNA is monitored, and the decrease is about 20%, 25%, 30%, or 35% or more as compared to the level of CXCL9 mRNA in the first skin sample.

In one described aspect, the treatment compound is a PDE4 inhibitor provided herein elsewhere. In another described aspect, the treatment compound is (S)-N-(2-(1-(3-ethoxy-4-methoxyphenyl)-2-(methylsulfonyl)ethyl)-1,3-dioxoisoindolin-4-yl)acetamide. In another embodiment, the treatment compound is cyclopropanecarboxylic acid {2-[(*1S*)-1-(3-ethoxy-4-methoxy-phenyl)-2-methanesulfonyl-ethyl]-3-oxo-2,3-dihydro-1*H-*isoindol-4-yl} -amide.

### 4.4 PDE4 modulators

In some embodiments, the biomarkers provided herein may be used to predict or monitor the efficacy of treatment for psoriasis by a PDE4 modulator. PDE4 modulators described herein include racemic, stereomerically pure and stereomerically enriched PDE4 modulators, stereomerically and enantiomerically pure compounds that have selective cytokine inhibitory activities, and pharmaceutically acceptable salts, solvates, hydrates, stereoisomers, clathrates, and prodrugs thereof. Certain compounds described herein are known PDE4 modulators of Celgene Corporation, NJ.

As used herein and unless otherwise indicated, the term "PDE 4 modulators" encompasses small molecule drugs, *e.g.,* small organic molecules which are not peptides, proteins, nucleic acids, oligosaccharides or other macromolecules. Preferred compounds inhibit TNF-*α* production. Compounds may also have a modest inhibitory effect on LPS induced IL1β and IL12. More preferably, the compounds provided herein are potent PDE 4 inhibitors.

Specific examples of PDE 4 modulators include, but are not limited to, the cyclic imides disclosed in U.S. patent nos. 5,605,914 and 5,463,063; the cycloalkyl amides and cycloalkyl nitriles of U.S. patent nos. 5,728,844, 5,728,845, 5,968,945, 6,180,644 and 6,518,281; the aryl amides (for example N-benzoyl-3-amino-3-(3',4'-dimethoxyphenyl)-propanamide) of U.S. patent nos. 5,801,195, 5,736,570, 6,046,221 and 6,284,780; the imide/amide ethers and alcohols (for example, 3-phthalimido-3-(3',4'-dimethoxyphenyl)propan-1-ol) disclosed in U.S. patent no. 5,703,098; the succinimides and maleimides (for example methyl 3-(3',4',5'6'-petrahydrophthalimdo)-3-(3",4"-dimethoxyphenyl)propionate) disclosed in U.S. patent no. 5,658,940; imido and amido substituted alkanohydroxamic acids disclosed in U.S. patent no. 6,214,857 and WO 99/06041; substituted phenethylsulfones disclosed in U.S. patent nos. 6,011,050 and 6,020,358; fluoroalkoxy-substituted 1,3-dihydro-isoindolyl compounds disclosed in U.S. patent publication no. 2004/0204448; substituted imides (for example, 2-phthalimido-3-(3',4'-dimethoxyphenyl) propane) disclosed in U.S. patent no. 6,429,221; substituted 1,3,4-oxadiazoles (for example, 2-[1-(3-cyclopentyloxy-4-methoxyphenyl)-2-(1,3,4-oxadiazole-2-yl)ethyl]-5-methylisoindoline-1,3-dione) disclosed in U.S. patent no. 6,326,388; cyano and carboxy derivatives of substituted styrenes (for example, 3,3-bis-(3,4-dimethoxyphenyl) acrylonitrile) disclosed in U.S. patent nos. 5,929,117, 6,130,226, 6,262,101 and 6,479,554; isoindoline-1-one and isoindoline-1,3-dione substituted in the 2-position with an α-(3,4-disubstituted phenyl)alkyl group and in the 4- and/or 5-position with a nitrogen-containing group disclosed in WO 01/34606 and U.S. patent no. 6,667,316, for example, cyclopropyl-N-{2-[1-(3-ethoxy-4-methoxyphenyl)-2-(methylsulfonyl)ethyl]-3-oxoisoindolin-4-yl} carboxamide, cyclopropyl-N-{2-[1(S)-(3-ethoxy-4-methoxyphenyl)-2-(methylsulfonyl)ethyl]-3-oxoisoindolin-4-yl}carboxamide, and cyclopropyl-N-{2-[1(R)-(3-ethoxy-4-methoxyphenyl)-2-(methylsulfonyl)ethyl]-3-oxoisoindolin-4-yl} carboxamide; and imido and amido substituted acylhydroxamic acids (for example, (3-(1,3-dioxoisoindoline-2-yl)-3-(3-ethoxy-4-methoxyphenyl) propanoylamino) propanoate disclosed in WO 01/45702 and U.S. patent no. 6,699,899. Other PDE 4 modulators include diphenylethylene compounds disclosed in U.S. patent publication no. 2005/0014727. Other PDE 4 modulators include isoindoline compounds disclosed in U.S. patent publication nos. 2006/0025457 and 2006/0084815. Other specific PDE 4 modulators include 2-[1-(3-ethoxy-4-methoxyphenyl)-2-methylsulfonylethyl]-4-acetylaminoisoindoline-1,3-dione, and stereoisomers thereof. (+)-2-[1-(3-ethoxy-4-methoxyphenyl)-2-methylsulfonylethyl]-4-acetylaminoisoindoline-1,3-dione was disclosed in WO 03/080049.

Additional PDE 4 modulators belong to a family of synthesized chemical compounds of which typical examples include 3-(1,3-dioxobenzo-[f]isoindol-2-yl)-3-(3-cyclopentyloxy-4-methoxyphenyl)propionamide and 3-(1,3-dioxo-4-azaisoindol-2-yl)-3-(3,4-dimethoxyphenyl)-propionamide.

Various PDE4 modulators described herein contain one or more chiral centers, and can exist as racemic mixtures of enantiomers or mixtures of diastereomers. The methods and compositions herein encompass the use of stereomerically pure forms of such compounds, as well as the use of mixtures of those forms. For example, mixtures comprising unequal amounts of the enantiomers of a particular PDE4 modulator may be used in methods and compositions provided herein. These isomers may be asymmetrically synthesized or resolved using standard techniques such as chiral columns or chiral resolving agents. *See, e.g.,* Jacques, J., et al., Enantiomers, Racemates and Resolutions (Wiley-Interscience, New York, 1981); Wilen, S. H., et al., Tetrahedron 33:2725 (1977); Eliel, E. L., Stereochemistry of Carbon Compounds (McGraw-Hill, NY, 1962); and Wilen, S. H., Tables of Resolving Agents and Optical Resolutions p. 268 (E.L. Eliel, Ed., Univ. of Notre Dame Press, Notre Dame, IN, 1972).

As used herein, and unless otherwise indicated, the term "optically pure" means a composition that comprises one optical isomer of a compound and is substantially free of other isomers of that compound. For example, an optically pure composition of a compound having one chiral center will be substantially free of the opposite enantiomer of the compound. An optically pure composition of a compound having two chiral centers will be substantially free of other diastereomers of the compound. A typical optically pure compound comprises greater than about 80% by weight of one enantiomer of the compound and less than about 20% by weight of other enantiomers of the compound, greater than about 90% by weight of one enantiomer of the compound and less than about 10% by weight of the other enantiomers of the compound, greater than about 95% by weight of one enantiomer of the compound and less than about 5% by weight of the other enantiomers of the compound, greater than about 97% by weight of one enantiomer of the compound and less than about 3% by weight of the other enantiomers of the compound or greater than about 99% by weight of one enantiomer of the compound and less than about 1% by weight of the other enantiomers of the compound.

Certain specific PDE4 modulators belong to a class of non-polypeptide cyclic amides disclosed in U.S. patent nos. 5,698,579, 5,877,200, 6,075,041 and 6,200,987, and WO 95/01348. Representative cyclic amides include compounds of the formula: 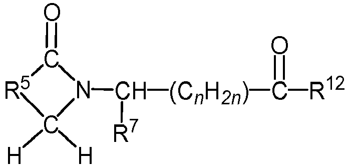
wherein n has a value of 1, 2, or 3;
R⁵ is o-phenylene, unsubstituted or substituted with 1 to 4 substituents each selected independently from the group consisting of nitro, cyano, trifluoromethyl, carbethoxy, carbomethoxy, carbopropoxy, acetyl, carbamoyl, acetoxy, carboxy, hydroxy, amino, alkylamino, dialkylamino, acylamino, alkyl of 1 to 10 carbon atoms, alkyl of 1 to 10 carbon atoms, and halo;
R⁷ is (i) phenyl or phenyl substituted with one or more substituents each selected independently of the other from the group consisting of nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, and halo, (ii) benzyl unsubstituted or substituted with 1 to 3 substituents selected from the group consisting of nitro, cyano, trifluoromethyl, carbothoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, and halo, (iii) naphthyl, and (iv) benzyloxy;
R¹² is -OH, alkoxy of 1 to 12 carbon atoms, or 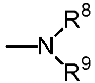
R⁸ is hydrogen or alkyl of 1 to 10 carbon atoms; and
R⁹ is hydrogen, alkyl of 1 to 10 carbon atoms, -COR¹⁰, or -SO₂R¹⁰, wherein R¹⁰ is hydrogen, alkyl of 1 to 10 carbon atoms, or phenyl.

Specific compounds of this class include, but are not limited to:
3-phenyl-2-(1-oxoisoindolin-2-yl)propionic acid;
3-phenyl-2-(1-oxoisoindolin-2-yl)propionamide;
3-phenyl-3-(1-oxoisoindolin-2-yl)propionic acid;
3-phenyl-3-(1-oxoisoindolin-2-yl)propionamide;
3-(4-methoxyphenyl)-3-(1-oxisoindolin-yl)propionic acid;
3-(4-methoxyphenyl)-3-(1-oxisoindolin-yl)propionamide;
3-(3,4-dimethoxyphenyl)-3-(1-oxisoindolin-2-yl)propionicacid;
3-(3,4-dimethoxy-phenyl)-3-(1-oxo-1,3-dihydroisoindol-2-yl)propionamide;
3-(3,4-dimethoxyphenyl)-3-(1-oxisoindolin-2-yl)propionamide;
3 -(3,4-diethoxyphenyl)-3 -(1 -oxoisoindolin-yl)propionic acid;
methyl 3 -(1 -oxoisoindolin-2-yl)-3 -(3 -ethoxy-4-methoxyphenyl)propionate;
3-(1-oxoisoindolin-2-yl)-3-(3-ethoxy-4-methoxyphenyl)propionic acid;
3 -(1 -oxoisoindolin-2-yl)-3 -(3 -propoxy-4-methoxyphenyl)propionic acid;
3 -(1 -oxoisoindolin-2-yl)-3 -(3 -butoxy-4-methoxyphenyl)propionic acid;
3-(1-oxoisoindolin-2-yl)-3-(3-propoxy-4-methoxyphenyl)propionamide;
3-(1-oxoisoindolin-2-yl)-3-(3-butoxy-4-methoxyphenyl)propionamide;
methyl 3-(1-oxoisoindolin-2-yl)-3-(3-butoxy-4-methoxyphenyl)propionate; and
methyl 3-(1-oxoisoindolin-2-yl)-3-(3-propoxy-4-methoxyphenyl)propionate.

Other representative PDE4 modulators include compounds of the formula: 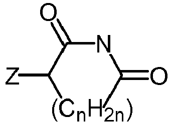 in which Z is: 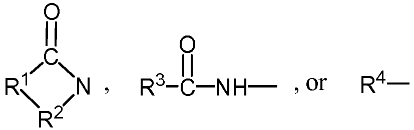 in which:
R¹ is the divalent residue of (i) 3,4-pyridine, (ii) pyrrolidine, (iii) imidizole, (iv) naphthalene, (v) thiophene, or (vi) a straight or branched alkane of 2 to 6 carbon atoms, unsubstituted or substituted with phenyl or phenyl substituted with nitro, cyano, trifluoromethyl, carbethoxy, carbomethoxy, carbopropoxy, acetyl, carbamyl, acetoxy, carboxy, hydroxy, amino, alkyl of 1 to 10 carbon atoms, alkoxy of 1 to 10 carbon atoms, or halo, wherein the divalent bonds of said residue are on vicinal ring carbon atoms;
R² is -CO - or -SO₂ -;
R³ is (i) phenyl substituted with 1 to 3 substituents each selected independently from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo, (ii) pyridyl, (iii) pyrrolyl, (iv) imidazolyl, (iv) naphthyl, (vi) thienyl, (vii) quinolyl, (viii) furyl, or (ix) indolyl;
R⁴ is alanyl, arginyl, glycyl, phenylglycyl, histidyl, leucyl, isoleucyl, lysyl, methionyl, prolyl, sarcosyl, seryl, homoseryl, threonyl, thyronyl, tyrosyl, valyl, benzimidol-2-yl, benzoxazol-2-yl, phenylsulfonyl, methylphenylsulfonyl, or phenylcarbamoyl; and
n has a value of 1, 2, or 3.

Other representative cyclic amides include compounds of the formula: 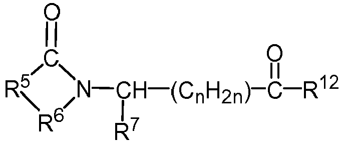 in which:
R⁵ is (i) o-phenylene, unsubstituted or substituted with 1 to 4 substituents each selected independently from nitro, cyano, trifluoromethyl, carbethoxy, carbomethoxy, carbopropoxy, acetyl, carbamoyl, acetoxy, carboxy, hydroxy, amino, alkylamino, dialkylamino, acylamino, alkyl of 1 to 10 carbon atoms, alkoxy of 1 to 10 carbon atoms, or halo, or (ii) the divalent residue of pyridine, pyrrolidine, imidizole, naphthalene, or thiophene, wherein the divalent bonds are on vicinal ring carbon atoms;
R⁶ is -CO -, -CH₂-, or -SO₂-;
R⁷ is (i) hydrogen if R⁶ is -SO₂-, (ii) straight, branched, or cyclic alkyl of 1 to 12 carbon atoms, (iii) pyridyl, (iv) phenyl or phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo, (v) alkyl of 1 to 10 carbon atoms, (vi) benzyl unsubstituted or substituted with 1 to 3 substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo, (vii) naphthyl, (viii) benzyloxy, or (ix) imidazol-4-yl methyl;
R¹² is -OH, alkoxy of 1 to 12 carbon atoms, or 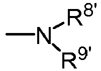
n has a value of 0, 1, 2, or 3;
R^{8'} is hydrogen or alkyl of 1 to 10 carbon atoms; and
R^{9'} is hydrogen, alkyl of 1 to 10 carbon atoms, -COR¹⁰, or -SO₂ R¹⁰ in which R¹⁰ is hydrogen, alkyl of 1 to 10 carbon atoms, or phenyl.

Other representative imides include compounds of the formula: 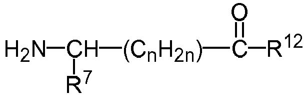 in which:
R⁷ is (i) straight, branched, or cyclic alkyl of 1 to 12 carbon atoms, (ii) pyridyl, (iii) phenyl or phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo, (iv) benzyl unsubstituted or substituted with one to three substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo, (v) naphthyl, (vi) benzyloxy, or (vii) imidazol-4-ylmethyl;
R¹² is -OH, alkoxy of 1 to 12 carbon atoms, -O-CH₂-pyridyl, -O-benzyl or 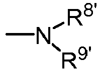 wherein n has a value of 0, 1, 2, or 3;
R^{8'} is hydrogen or alkyl of 1 to 10 carbon atoms; and
R^{9'} is hydrogen, alkyl of 1 to 10 carbon atoms, -CH₂-pyridyl, benzyl, -COR¹⁰, or -SO₂R¹⁰ in which R¹⁰ is hydrogen, alkyl of 1 to 4 carbon atoms, or phenyl.

Other specific PDE4 modulators include the imido and amido substituted alkanohydroxamic acids disclosed in WO 99/06041 and U.S. patent no. 6,214,857. Examples of such compound include, but are not limited to: 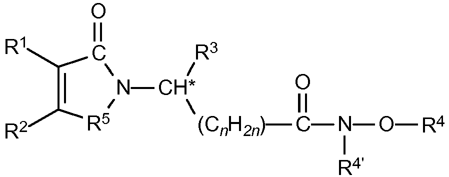 wherein:
each of R¹ and R², when taken independently of each other, is hydrogen, lower alkyl, or R¹ and R², when taken together with the depicted carbon atoms to which each is bound, is *o*-phenylene, *o*-naphthylene, or cyclohexene-1,2-diyl, unsubstituted or substituted with 1 to 4 substituents each selected independently from the group consisting of nitro, cyano, trifluoromethyl, carbethoxy, carbomethoxy, carbopropoxy, acetyl, carbamoyl, acetoxy, carboxy, hydroxy, amino, alkylamino, dialkylamino, acylamino, alkyl of 1 to 10 carbon atoms, alkoxy of 1 to 10 carbon atoms, and halo;
R³ is phenyl substituted with from one to four substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, alkylthio of 1 to 10 carbon atoms, benzyloxy, cycloalkoxy of 3 to 6 carbon atoms, C₄-C₆-cycloalkylidenemethyl, C₃-C₁₀-alkylidenemethyl, indanyloxy, and halo;
R⁴ is hydrogen, alkyl of 1 to 6 carbon atoms, phenyl, or benzyl;
R^{4'} is hydrogen or alkyl of 1 to 6 carbon atoms;
R⁵ is -CH₂-, -CH₂-CO-, -SO₂-, -S-, or -NHCO-; and
n has a value of 0, 1, or 2; or
an acid addition salt of said compounds.

Additional specific PDE4 modulators provided herein include, but are not limited to:
3-(3-ethoxy-4-methoxyphenyl)-N-hydroxy-3-(1-oxoisoindolinyl)propionamide;
3 -(3 -ethoxy-4-methoxyphenyl)-N-methoxy-3 -(1 -oxoisoindolinyl)propionamide;
N-benzyloxy-3-(3-ethoxy-4-methoxyphenyl)-3-phthalimidopropionamide;
N-benzyloxy-3-(3-ethoxy-4-methoxyphenyl)-3-(3-nitrophthalimido)propionamide;
N-benzyloxy-3-(3-ethoxy-4-methoxyphenyl)-3-(1-oxoisoindolinyl)propionamide;
3-(3-ethoxy-4-methoxyphenyl)-N-hydroxy-3-phthalimidopropionamide;
N-hydroxy-3-(3,4-dimethoxyphenyl)-3-phthalimidopropionamide;
3-(3-ethoxy-4-methoxyphenyl)-N-hydroxy-3-(3-nitrophthalimido)propionamide;
N-hydroxy-3-(3,4-dimethoxyphenyl)-3-(1-oxoisoindolinyl)propionamide;
3-(3-ethoxy-4-methoxyphenyl)-N-hydroxy-3-(4-methyl-phthalimido)propionamide;
3-(3-cyclopentyloxy-4-methoxyphenyl)-N-hydroxy-3-phthalimidopropionamide;
3-(3-ethoxy-4-methoxyphenyl)-N-hydroxy-3-(1,3-dioxo-2,3-dihydro-1H-benzo[f]isoindol-2-yl)propionamide;
N-hydroxy-3-{3-(2-propoxy)-4-methoxyphenyl}-3-phthalimidopropionamide;
3-(3-ethoxy-4-methoxyphenyl)-3-(3,6-difluorophthalimido)-N-hydroxypropionamide;
3 -(4-aminophthalimido)-3 -(3 -ethoxy-4-methoxyphenyl)-N-hydroxypropionamide;
3 -(3 -aminophthalimido)-3 -(3 -ethoxy-4-methoxyphenyl)-N-hydroxypropionamide;
3 -(3 -acetoamidophthalimido)-3 -(3 -ethoxy-4-methoxyphenyl)-N-hydroxypropionamide;
N-hydroxy-3-(3,4-dimethoxyphenyl)-3-(1-oxoisoindolinyl)propionamide;
3-(3-cyclopentyloxy-4-methoxyphenyl)-N-hydroxy-3-(1-oxoisoindolinyl) propionamide; and
N-benzyloxy-3-(3-ethoxy-4-methoxyphenyl)-3-(3-nitrophthalimido)propionamide.

Additional PDE 4 modulators provided herein include the phenethylsulfones substituted on the phenethyl group with a oxoisoindoline group. Examples of such compounds include, but are not limited to, those disclosed in U.S. patent no. 6,020,358, which include the following: 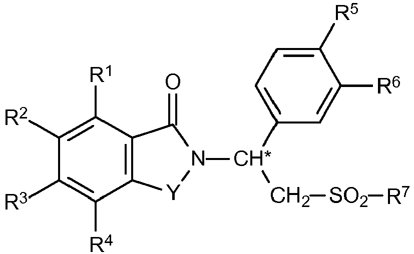 wherein the carbon atom designated * constitutes a center of chirality;
Y is C=O, CH₂, SO₂, or CH₂C=O; each of R¹, R², R³, and R⁴, independently of the others, is hydrogen, halo, alkyl of 1 to 4 carbon atoms, alkoxy of 1 to 4 carbon atoms, nitro, cyano, hydroxy, or -NR⁸R⁹; or any two of R¹, R², R³, and R⁴ on adjacent carbon atoms, together with the depicted phenylene ring are naphthylidene;
each of R⁵ and R⁶, independently of the other, is hydrogen, alkyl of 1 to 4 carbon atoms, alkoxy of 1 to 4 carbon atoms, cyano, or cycloalkoxy of up to 18 carbon atoms;
R⁷ is hydroxy, alkyl of 1 to 8 carbon atoms, phenyl, benzyl, or NR^{8'}R^{9'};
each of R⁸ and R⁹ taken independently of the other is hydrogen, alkyl of 1 to 8 carbon atoms, phenyl, or benzyl, or one of R⁸ and R⁹ is hydrogen and the other is - COR¹⁰ or -SO₂R¹⁰, or R⁸ and R⁹ taken together are tetramethylene, pentamethylene, hexamethylene, or -CH₂CH₂X¹CH₂CH₂- in which X¹ is -O-, -S- or -NH-; and
each of R^{8'} and R^{9'} taken independently of the other is hydrogen, alkyl of 1 to 8 carbon atoms, phenyl, or benzyl, or one of R^{8'} and R^{9'} is hydrogen and the other is - COR^{10'} or -SO₂R^{10'}, or R^{8'} and R^{9'} taken together are tetramethylene, pentamethylene, hexamethylene, or -CH₂CH₂X²CH₂CH₂- in which X² is -O-, -S-, or -NH-.

It will be appreciated that while for convenience the above compounds are identified as phenethylsulfones, they include sulfonamides when R⁷ is NR^{8'}R^{9'}.

Specific groups of such compounds are those in which Y is C=O or CH₂.

A further specific group of such compounds are those in which each of R¹, R², R³, and R⁴ independently of the others, is hydrogen, halo, methyl, ethyl, methoxy, ethoxy, nitro, cyano, hydroxy, or -NR⁸R⁹ in which each of R⁸ and R⁹ taken independently of the other is hydrogen or methyl or one of R⁸ and R⁹ is hydrogen and the other is -COCH₃.

Particular compounds are those in which one of R¹, R², R³, and R⁴ is -NH₂ and the remaining of R¹, R², R³, and R⁴ are hydrogen.

Particular compounds are those in which one of R¹, R², R³, and R⁴ is-NHCOCH₃ and the remaining of R¹, R², R³, and R⁴ are hydrogen.

Particular compounds are those in which one of R¹, R², R³, and R⁴ is-N(CH₃)₂ and the remaining of R¹, R², R³, and R⁴ are hydrogen.

A further preferred group of such compounds are those in which one of R¹, R², R³, and R⁴ is methyl and the remaining of R¹, R², R³, and R⁴ are hydrogen.

Particular compounds are those in which one of R¹, R², R³, and R⁴ is fluoro and the remaining of R¹, R², R³, and R⁴ are hydrogen.

Particular compounds are those in which each of R⁵ and R⁶, independently of the other, is hydrogen, methyl, ethyl, propyl, methoxy, ethoxy, propoxy, cyclopentoxy, or cyclohexoxy.

Particular compounds are those in which R⁵ is methoxy and R⁶ is monocycloalkoxy, polycycloalkoxy, and benzocycloalkoxy.

Particular compounds are those in which R⁵ is methoxy and R⁶ is ethoxy.

Particular compounds are those in which R⁷ is hydroxy, methyl, ethyl, phenyl, benzyl, or NR^{8'}R^{9'} in which each of R^{8'} and R^{9'} taken independently of the other is hydrogen or methyl.

Particular compounds are those in which R⁷ is methyl, ethyl, phenyl, benzyl or NR^{8'}R^{9'} in which each of R^{8'} and R^{9'} taken independently of the other is hydrogen or methyl.

Particular compounds are those in which R⁷ is methyl.

Particular compounds are those in which R⁷ is NR^{8'}R^{9'} in which each of R^{8'} and R^{9'} taken independently of the other is hydrogen or methyl.

Additional PDE4 modulators include fluoroalkoxy-substituted 1,3-dihydro-isoindolyl compounds disclosed in U.S. patent publication no. 2004/0204448. Representative compounds are of formula: 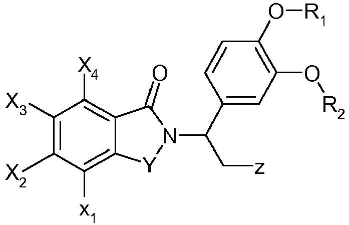 wherein:
Y is -C(O)-, -CH₂, -CH₂C(O)-, -C(O)CH₂-, or SO_{2;}
Z is -H, -C(O)R³, -(C₀₋₁-alkyl)-SO₂-(C₁₋₄-alkyl), -C₁₋₈-alkyl, -CH₂OH, CH₂(O)(C₁₋₈alkyl) or -CN;
R₁ and R₂ are each independently -CHF₂, -C₁₋₈-alkyl, -C₃₋₁₈-cycloalkyl, or -(C₁₋₁₀-alkyl)(C₃₋₁₈-cycloalkyl), and at least one of R₁ and R₂ is CHF₂;
R³ is -NR⁴R⁵, -alkyl, -OH, -O-alkyl, phenyl, benzyl, substituted phenyl, or substituted benzyl;
R⁴ and R⁵ are each independently -H, -C₁₋₈-alkyl, -OH, -OC(O)R⁶;
R⁶ is -C₁₋₈-alkyl, -amino(C₁₋₈-alkyl), -phenyl, -benzyl, or -aryl;
X₁, X₂, X₃, and X₄ are each independently -H, -halogen, -nitro, -NH₂, -CF₃, -C₁₋₆-alkyl, -(C₀₋₄-alkyl)-(C₃₋₆-cycloalkyl), (C₀₋₄-alkyl)-NR⁷R⁸, (C₀₋₄-alkyl)-N(H)C(O)-(R⁸), (C₀₋₄-alkyl)-N(H)C(O)N(R⁷R⁸), (C₀₋₄-alkyl)-N(H)C(O)O(R⁷R⁸), (C₀₋₄-alkyl)-OR⁸, (C₀₋₄-alkyl)-imidazolyl, (C₀₋₄-alkyl)-pyrrolyl, (C₀₋₄-alkyl)-oxadiazolyl, or (C₀₋₄-alkyl)-triazolyl, or two of X₁, X₂, X₃, and X₄ may be joined together to form a cycloalkyl or heterocycloalkyl ring, (*e.g*., X₁ and X₂, X₂ and X₃, X₃ and X₄, X₁ and X₃, X₂ and X₄, or X₁ and X₄ may form a 3, 4, 5, 6, or 7 membered ring which may be aromatic, thereby forming a bicyclic system with the isoindolyl ring); and
R⁷ and R⁸ are each independently H, C₁₋₉-alkyl, C₃₋₆-cycloalkyl, (C₁₋₆-alkyl)-(C₃₋₆-cycloalkyl), (C₁₋₆-alkyl)-N(R⁷R⁸), (C₁₋₆-alkyl)-OR⁸, phenyl, benzyl, or aryl; or a pharmaceutically acceptable salt, solvate, hydrate, stereoisomer, clathrate, or prodrug thereof.

Additional PDE4 modulators include the enantiomerically pure compounds disclosed in U.S. patent publication nos. 2003/0187052, 2004/0167199, and 2005/0014727 and international patent publication nos. WO 2003/080048 and WO 2003.080049. In one described aspect, the compound is an enantiomer of 2-[1-(3-ethoxy-4-methoxyphenyl)-2-methylsulfonylethyl]-4-acetylaminoisoindoline-1,3-dione and an enantiomer of 3-(3,4-dimethoxy-phenyl)-3-(1-oxo-1,3-dihydro-isoindol-2-yl)-propionamide.

In some embodiments, PDE4 modulator is cyclopropanecarboxylic acid {2-[1-(3-ethoxy-4-methoxy-phenyl)-2-methanesulfonyl-ethyl]-3-oxo-2,3-dihydro-1*H*-isoindol-4-yl}-amide, available from Celgene Corp., Warren, NJ. 3-(3,4-Dimethoxy-phenyl)-3-(1-oxo-1,3-dihydro-isoindol-2-yl)-propionamide has the following chemical structure:

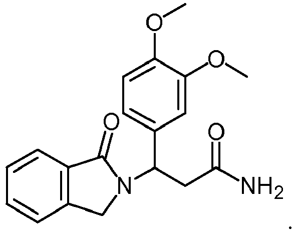

Other PDE4 modulators include, but are not limited to, the cycloalkyl amides and cycloalkyl nitriles disclosed in U.S. patent nos. 5,728,844, 5,728,845, 5,968,945, 6,180,644 and 6,518,281, and WO 97/08143 and WO 97/23457. Representative compounds are of the formula: 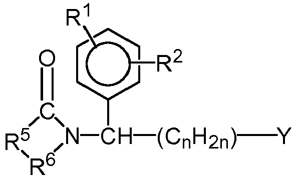 wherein:
one of R¹ and R² is R³-X- and the other is hydrogen, nitro, cyano, trifluoromethyl, carbo(lower)alkoxy, acetyl, carbamoyl, acetoxy, carboxy, hydroxy, amino, lower alkyl, lower alkoxy, halo, or R³-X-;
R³ is monocycloalkyl, bicycloalkyl, or benzocycloalkyl of up to 18 carbon atoms;
X is a carbon-carbon bond, -CH₂-, or -O-;
R⁵ is (i) o-phenylene, unsubstituted or substituted with 1 to 3 substituents each selected independently from nitro, cyano, halo, trifluoromethyl, carbo(lower)alkoxy, acetyl, or carbamoyl, unsubstituted or substituted with lower alkyl, acetoxy, carboxy, hydroxy, amino, lower alkylamino, lower acylamino, or lower alkoxy; (ii) a vicinally divalent residue of pyridine, pyrrolidine, imidazole, naphthalene, or thiophene, wherein the divalent bonds are on vicinal ring carbon atoms; (iii) a vicinally divalent cycloalkyl or cycloalkenyl of 4-10 carbon atoms, unsubstituted or substituted with 1 to 3 substituents each selected independently from the group consisting of nitro, cyano, halo, trifluoromethyl, carbo(lower)alkoxy, acetyl, carbamoyl, acetoxy, carboxy, hydroxy, amino, lower alkylamino, lower alkyl, lower alkoxy, or phenyl; (iv) vinylene disubstituted with lower alkyl; or (v) ethylene, unsubstituted or monosubstituted or disubstituted with lower alkyl;
R⁶ is -CO-, -CH₂-, or -CH₂CO-;
Y is -COZ, -C= N, -OR⁸, lower alkyl, or aryl;
Z is -NH₂, -OH, -NHR, -R⁹, or -OR⁹
R⁸ is hydrogen or lower alkyl;
R⁹ is lower alkyl or benzyl; and,
n has a value of 0, 1, 2, or 3.

In one described aspect, one of R¹ and R² is R³-X- and the other is hydrogen, nitro, cyano, trifluoromethyl, carbo(lower)alkoxy, acetyl, carbamoyl, acetoxy, carboxy, hydroxy, amino, lower alkyl, lower alkoxy, halo, or R3-X-;
R³ is monocycloalkyl of up to 10 carbon atoms, polycycloalkyl of up to 10 carbon atoms, or benzocyclic alkyl of up to 10 carbon atoms;
X is -CH₂-, or -O-;
R⁵ is (i) the vicinally divalent residue of pyridine, pyrrolidine, imidazole, naphthalene, or thiophene, wherein the two bonds of the divalent residue are on vicinal ring carbon atoms;
(ii) a vicinally divalent cycloalkyl of 4-10 carbon atoms, unsubstituted or substituted with 1 to 3 substituents each selected independently from the group consisting of nitro, cyano, halo, trifluoromethyl, carbethoxy, carbomethoxy, carbopropoxy, acetyl, carbamoyl, acetoxy, carboxy, hydroxy, amino, substituted amino, alkyl of 1 to 10 carbon atoms, alkoxy of 1 to 10 carbon atoms, or phenyl;
(iii) di-substituted vinylene, substituted with nitro, cyano, trifluoromethyl, carbethoxy, carbomethoxy, carbopropoxy, acetyl, carbamoyl, carbamoyl substituted with and alkyl of 1 to 3 carbon atoms, acetoxy, carboxy, hydroxy, amino, amino substituted with an alkyl of 1 to 3 carbon atoms, alkyl of 1 to 4 carbon atoms, alkoxy of 1 to 4 carbon atoms, or halo;
(iv) ethylene, unsubstituted or substituted with 1 to 2 substituents each selected independently from nitro, cyano, trifluoromethyl, carbethoxy, carbomethoxy, carbopropoxy, acetyl, carbamoyl, carbamoyl substituted with and alkyl of 1 to 3 carbon atoms, acetoxy, carboxy, hydroxy, amino, amino substituted with an alkyl of 1 to 3 carbon atoms, alkyl of 1 to 4 carbon atoms, alkoxy of 1 to 4 carbon atoms, or halo;
R⁶ is -CO-, -CH₂-, or -CH₂CO-;
Y is -COX, -C≡N, -OR⁸, alkyl of 1 to 5 carbon atoms, or aryl;
X is -NH₂, -OH, -NHR, -R⁹, -OR⁹, or alkyl of 1 to 5 carbon atoms;
R⁸ is hydrogen or lower alkyl;
R⁹ is alkyl or benzyl; and,
n has a value of 0, 1, 2, or 3.

In another described aspect, one of R¹ and R² is R³-X- and the other is hydrogen, nitro, cyano, trifluoromethyl, carbo(lower)alkoxy, acetyl, carbamoyl, acetoxy, carboxy, hydroxy, amino, lower alkyl, lower alkoxy, halo, HF₂CO, F₃CO, or R³-X-;
R³ is monocycloalkyl, bicycloalkyl, benzocyclo alkyl of up to 18 carbon atoms, tetrahydropyran, or tetrahydrofuran;
X is a carbon-carbon bond, -CH₂-, -O-, or -N=;
R⁵ is (i) o-phenylene, unsubstituted or substituted with 1 to 3 substituents each selected independently from nitro, cyano, halo, trifluoromethyl, carbo(lower)alkoxy, acetyl, or carbamoyl, unsubstituted or substituted with lower alkyl, acetoxy, carboxy, hydroxy, amino, lower alkylamino, lower acylamino, or lower alkoxy; (ii) a vicinally divalent residue of pyridine, pyrrolidine, imidazole, naphthalene, or thiophene, wherein the divalent bonds are on vicinal ring carbon atoms; (iii) a vicinally divalent cycloalkyl or cycloalkenyl of 4-10 carbon atoms, unsubstituted or substituted with 1 or more substituents each selected independently from the group consisting of nitro, cyano, halo, trifluoromethyl, carbo(lower)alkoxy, acetyl, carbamoyl, acetoxy, carboxy, hydroxy, amino, lower alkylamino, lower alkyl, lower alkoxy, or phenyl; (iv) vinylene di-substituted with lower alkyl; or (v) ethylene, unsubstituted or monosubstituted or disubstituted with lower alkyl;
R⁶ is -CO-, -CH₂-, or -CH₂CO-;
Y is -COX, -C≡N, -OR⁸, alkyl of 1 to 5 carbon atoms, or aryl;
X is -NH₂, -OH, -NHR, -R⁹, -OR⁹, or alkyl of 1 to 5 carbon atoms;
R⁸ is hydrogen or lower alkyl;
R⁹ is alkyl or benzyl; and,
n has a value of 0, 1, 2, or 3.

Other representative compounds are of formula: 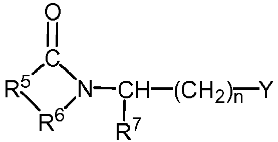 wherein:
Y is -C≡N or CO(CH₂)ₘCH₃;
m is 0, 1, 2, or 3;
R⁵ is (i) o-phenylene, unsubstituted or substituted with 1 to 3 substituents each selected independently from nitro, cyano, trifluoromethyl, carbethoxy, carbomethoxy, carbopropoxy, acetyl, carbamoyl, carbamoyl substituted with and alkyl of 1 to 3 carbon atoms, acetoxy, carboxy, hydroxy, amino, amino substituted with an alkyl of 1 to 3 carbon atoms, alkyl of 1 to 4 carbon atoms, alkoxy of 1 to 4 carbon atoms, or halo; (ii) the divalent residue of pyridine, pyrrolidine, imidizole, naphthalene, or thiophene, wherein the divalent bonds are on vicinal ring carbon atoms; (iii) a divalent cycloalkyl of 4-10 carbon atoms, unsubstituted or substituted with one or more substituents each selected independently of the other from the group consisting of nitro, cyano, trifluoromethyl, carbethoxy, carbomethoxy, carbopropoxy, acetyl, carbamoyl, acetoxy, carboxy, hydroxy, amino, substituted amino, alkyl of 1 to 10 carbon atoms, alkoxy of 1 to 10 carbon atoms, phenyl or halo; (iv) di-substituted vinylene, substituted with nitro, cyano, trifluoromethyl, carbethoxy, carbomethoxy, carbopropoxy, acetyl, carbamoyl, carbamoyl substituted with and alkyl of 1 to 3 carbon atoms, acetoxy, carboxy, hydroxy, amino, amino substituted with an alkyl of 1 to 3 carbon atoms, alkyl of 1 to 4 carbon atoms, alkoxy of 1 to 4 carbon atoms, or halo; or (v) ethylene, unsubstituted or substituted with 1 to 2 substituents each selected independently from nitro, cyano, trifluoromethyl, carbethoxy, carbomethoxy, carbopropoxy, acetyl, carbamoyl, carbamoyl substituted with and alkyl of 1 to 3 carbon atoms, acetoxy, carboxy, hydroxy, amino, amino substituted with an alkyl of 1 to 3 carbon atoms, alkyl of 1 to 4 carbon atoms, alkoxy of 1 to 4 carbon atoms, or halo;
R⁶ is -CO-, -CH₂-, -CH₂CO-, or -SO₂-;
R⁷ is (i) straight or branched alkyl of 1 to 12 carbon atoms; (ii) cyclic or bicyclic alkyl of 1 to 12 carbon atoms; (iii) pyridyl; (iv) phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, straight, branched, cyclic, or bicyclic alkyl of 1 to 10 carbon atoms, straight, branched, cyclic, or bicyclic alkoxy of 1 to 10 carbon atoms, CH₂R where R is a cyclic or bicyclic alkyl of 1 to 10 carbon atoms, or halo; (v) benzyl substituted with one to three substituents each selected independently from the group consisting of nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 10 carbon atoms, or halo; (vi) naphthyl; or (vii) benzyloxy; and
n has a value of 0, 1, 2, or 3.

In another described aspect, the PDE 4 modulators are of formula: 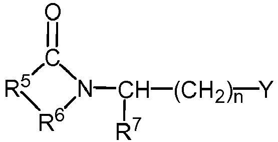 wherein:
R⁵ is (i) the divalent residue of pyridine, pyrrolidine, imidizole, naphthalene, or thiophene, wherein the divalent bonds are on vicinal ring carbon atoms; (ii) a divalent cycloalkyl of 4-10 carbon atoms, unsubstituted or substituted with one or more substituents each selected independently of the other from the group consisting of nitro, cyano, trifluoromethyl, carbethoxy, carbomethoxy, carbopropoxy, acetyl, carbamoyl, acetoxy, carboxy, hydroxy, amino, substituted amino, alkyl of 1 to 10 carbon atoms, alkoxy of 1 to 10 carbon atoms, phenyl or halo; (iii) di-substituted vinylene, substituted with nitro, cyano, trifluoromethyl, carbethoxy, carbomethoxy, carbopropoxy, acetyl, carbamoyl, carbamoyl substituted with and alkyl of 1 to 3 carbon atoms, acetoxy, carboxy, hydroxy, amino, amino substituted with an alkyl of 1 to 3 carbon atoms, alkyl of 1 to 4 carbon atoms, alkoxy of 1 to 4 carbon atoms, or halo; or (iv) ethylene, unsubstituted or substituted with 1 to 2 substituents each selected independently from nitro, cyano, trifluoromethyl, carbethoxy, carbomethoxy, carbopropoxy, acetyl, carbamoyl, carbamoyl substituted with and alkyl of 1 to 3 carbon atoms, acetoxy, carboxy, hydroxy, amino, amino substituted with an alkyl of 1 to 3 carbon atoms, alkyl of 1 to 4 carbon atoms, alkoxy of 1 to 4 carbon atoms, or halo;
R⁶ is -CO-, -CH₂-, -CH₂CO-, or -SO₂-;
R⁷ is (i) cyclic or bicyclic alkyl of 4 to 12 carbon atoms; (ii) pyridyl; (iii) phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, straight, branched, cyclic, or bicyclic alkyl of 1 to 10 carbon atoms, straight, branched, cyclic, or bicyclic alkoxy of 1 to 10 carbon atoms, CH₂R where R is a cyclic or bicyclic alkyl of 1 to 10 carbon atoms, or halo; (iv) benzyl substituted with one to three substituents each selected independently from the group consisting of nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 10 carbon atoms, or halo; (v) naphthyl; or (vi) benzyloxy; and
Y is COX, -C≡N, OR⁸, alkyl of 1 to 5 carbon atoms, or aryl;
X is -NH₂, -OH, -NHR, -R⁹, -OR⁹, or alkyl of 1 to 5 carbon atoms;
R⁸ is hydrogen or lower alkyl;
R⁹ is alkyl or benzyl; and
n has a value of 0, 1, 2, or 3.

Other PDE 4 modulators include, but are not limited to, the aryl amides (for example, an example being N-benzoyl-3-amino-3-(3',4'-dimethoxyphenyl)-propanamide) disclosed in U.S. patent nos. 5,801,195, 5,736,570, 6,046,221 and 6,284,780. Representative compounds are of formula: 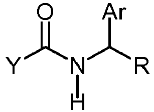 wherein:
Ar is (i) straight, branched, or cyclic, unsubstituted alkyl of 1 to 12 carbon atoms; (ii) straight, branched, or cyclic, substituted alkyl of 1 to 12 carbon atoms; (iii) phenyl; (iv) phenyl substituted with one or more substituents each selected independently of the other from the group consisting of nitro, cyano, trifluoromethyl, carbethoxy, carbomethoxy, carbopropoxy, acetyl, carbamoyl, acetoxy, carboxy, hydroxy, amino, substituted amino, alkyl of 1 to 10 carbon atoms, alkoxy of 1 to 10 carbon atoms, or halo; (v) heterocycle; or (vi) heterocycle substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo;
R is -H, alkyl of 1 to 10 carbon atoms, CH₂OH, CH₂CH₂OH, or CH₂COZ where Z is alkoxy of 1 to 10 carbon atoms, benzyloxy, or NHR¹ where R¹ is H or alkyl of 1 to 10 carbon atoms; and
Y is i) a phenyl or heterocyclic ring, unsubstituted or substituted one or more substituents each selected independently one from the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo or ii) naphthyl. Specific examples of the compounds are of formula: 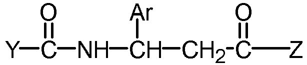 wherein:
   Ar is 3,4-disubstituted phenyl where each substituent is selected independently of the other from the group consisting of nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, and halo;
   Z is alkoxy of 1 to 10 carbon atoms, benzyloxy, amino, or alkylamino of 1 to 10 carbon atoms; and
   Y is (i) a phenyl, unsubstituted or substituted with one or more substituents each selected, independently one from the other, from the group consisting of nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, and halo, or (ii) naphthyl.

Other PDE 4 modulators include, but are not limited to, the imide/amide ethers and alcohols (for example, 3-phthalimido-3-(3',4'-dimethoxyphenyl) propan-1-ol) disclosed in U.S. patent no. 5,703,098. Representative compounds have the formula: 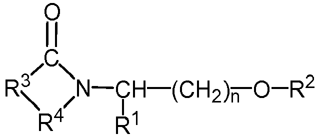 wherein:
R¹ is (i) straight, branched, or cyclic, unsubstituted alkyl of 1 to 12 carbon atoms; (ii) straight, branched, or cyclic, substituted alkyl of 1 to 12 carbon atoms; (iii) phenyl; or (iv) phenyl substituted with one or more substituents each selected independently of the other from the group consisting of nitro, cyano, trifluoromethyl, carbethoxy, carbomethoxy, carbopropoxy, acetyl, carbamoyl, acetoxy, carboxy, hydroxy, amino, acylamino, alkylamino, di(alkyl) amino, alkyl of 1 to 10 carbon atoms, cycloalkyl of 3 to 10 carbon atoms, bicycloalkyl of 5 to 12 carbon atoms, alkoxy of 1 to 10 carbon atoms, cycloalkoxy of 3 to 10 carbon atoms, bicycloalkoxy of 5 to 12 carbon atoms, and halo;
R² is hydrogen, alkyl of 1 to 8 carbon atoms, benzyl, pyridylmethyl, or alkoxymethyl;
R3 is (i) ethylene, (ii) vinylene, (iii) a branched alkylene of 3 to 10 carbon atoms, (iv) a branched alkenylene of 3 to 10 carbon atoms, (v) cycloalkylene of 4 to 9 carbon atoms unsubstituted or substituted with one or more substituents each selected independently from the group consisting of nitro, cyano, trifluoromethyl, carbethoxy, carbomethoxy, carbopropoxy, acetyl, carbamoyl, acetoxy, carboxy, hydroxy, amino, amino substituted with alkyl of 1 to 6 carbon atoms, amino substituted with acyl of 1 to 6 carbon atoms, alkyl of 1 to 10 carbon atoms, alkoxy of 1 to 12 carbon atoms, and halo, (vi) cycloalkenylene of 4 to 9 carbon atoms unsubstituted or substituted with one or more substituents each selected independently from the group consisting of nitro, cyano, trifluoromethyl, carbethoxy, carbomethoxy, carbopropoxy, acetyl, carbamoyl, acetoxy, carboxy, hydroxy, amino, amino substituted with alkyl of 1 to 6 carbon atoms, amino substituted with acyl of 1 to 6 carbon atoms, alkyl of 1 to 10 carbon atoms, alkoxy of 1 to 12 carbon atoms, and halo, (vii) o-phenylene unsubstituted or substituted with one or more substituents each selected independently from the group consisting of nitro, cyano, trifluoromethyl, carbethoxy, carbomethoxy, carbopropoxy, acetyl, carbamoyl, acetoxy, carboxy, hydroxy, amino, amino substituted with alkyl of 1 to 6 carbon atoms, amino substituted with acyl of 1 to 6 carbon atoms, alkyl of 1 to 10 carbon atoms, alkoxy of 1 to 12 carbon atoms, and halo, (viii) naphthyl, or (ix) pyridyl;
R⁴ is -CX-, -CH₂- or -CH₂CX-;
X is O or S; and
n is 0, 1, 2, or 3.

Other PDE 4 modulators include, but are not limited to, the succinimides and maleimides (for example methyl 3-(3',4',5'6'-petrahydrophthalimdo)-3-(3",4"-dimethoxyphenyl)propionate) disclosed in U.S. patent no. 5,658,940. Representative compounds are of formula: 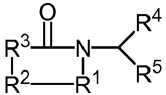 wherein:
R¹ is -CH₂-, -CH₂CO-, or -CO-;
R² and R³ taken together are (i) ethylene unsubstituted or substituted with alkyl of 1-10 carbon atoms or phenyl, (ii) vinylene substituted with two substituents each selected, independently of the other, from the group consisting of alkyl of 1-10 carbon atoms and phenyl, or (iii) a divalent cycloalkyl of 5-10 carbon atoms, unsubstituted or substituted with one or more substituents each selected independently of the other from the group consisting of nitro, cyano, trifluoromethyl, carbethoxy, carbomethoxy, carbopropoxy, acetyl, carbamoyl unsubstituted or substituted with alkyl of 1-3 carbon atoms, acetoxy, carboxy, hydroxy, amino, substituted amino, alkyl of 1 to 10 carbon atoms, alkoxy of 1 to 10 carbon atoms, norbornyl, phenyl or halo;
R⁴ is (i) straight or branched unsubstituted alkyl of 4 to 8 carbon atoms, (ii) cycloalkyl or bicycloalkyl of 5-10 carbon atoms, unsubstituted or substituted with one or more substituents each selected independently of the other from the group consisting of nitro, cyano, trifluoromethyl, carbethoxy, carbomethoxy, carbopropoxy, acetyl, carbamoyl, acetoxy, carboxy, hydroxy, amino, substituted amino, branched, straight or cyclic alkyl of 1 to 10 carbon atoms, alkoxy of 1 to 10 carbon atoms, phenyl or halo, (iii) phenyl substituted with one or more substituents each selected independently of the other from the group consisting of nitro, cyano, trifluoromethyl, carbethoxy, carbomethoxy, carbopropoxy, acetyl, carbamoyl, acetoxy, carboxy, hydroxy, amino, substituted amino, alkyl of 1 to 10 carbon atoms, alkoxy of 1 to 10 carbon atoms, cycloalkyl or bicyctoalkyl of 3 to 10 carbon atoms, cycloalkoxy or bicycloalkoxy of 3 to 10 carbon atoms, phenyl or halo, (iv) pyridine or pyrrolidine, unsubstituted or substituted with one or more substituents each selected independently of the other from the group consisting of nitro, cyano, trifluoromethyl, carbethoxy, carbomethoxy, carbopropoxy, acetyl, carbamoyl, acetoxy, carboxy, hydroxy, amino, substituted amino, alkyl of 1 to 10 carbon atoms, alkoxy of 1 to 10 carbon atoms, phenyl or halo; and,
R⁵ is -COX, -CN, -CH₂COX, alkyl of 1 to 5 carbon atoms, aryl, -CH₂OR, -CH₂ aryl, or -CH₂OH,
where X is NH₂, OH, NHR, or OR⁶,
where R is lower alkyl; and
where R⁶ is alkyl or benzyl.

Other PDE 4 modulators include, but are not limited to, substituted imides (for example, 2-phthalimido-3-(3',4'-dimethoxyphenyl) propane) disclosed in U.S. patent no. 6,429,221. Representative compounds have the formula: 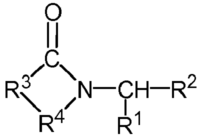 wherein:
R¹ is (i) straight, branched, or cyclic alkyl of 1 to 12 carbon atoms, (ii) phenyl or phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, straight or branched alkyl of 1 to 10 carbon atoms, alkoxy of 1 to 10 carbon atoms, or halo, (iii) benzyl or benzyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo, or (iv) -Y-Ph where Y is a straight, branched, or cyclic alkyl of 1 to 12 carbon atoms and Ph is phenyl or phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo;
R² is -H, a branched or unbranched alkyl of 1 to 10 carbon atoms, phenyl, pyridyl, heterocycle, -CH₂-aryl, or -CH₂-heterocycle;
R³ is i) ethylene, ii) vinylene, iii) a branched alkylene of 3 to 10 carbon atoms, iv) a branched alkenylene of 3 to 10 carbon atoms, v) cycloalkylene of 4 to 9 carbon atoms unsubstituted or substituted with 1 to 2 substituents each selected independently from nitro, cyano, trifluoromethyl, carbethoxy, carbomethoxy, carbopropoxy, acetyl, carbamoyl, acetoxy, carboxy, hydroxy, amino, substituted amino, alkyl of 1 to 4 carbon atoms, alkoxy of 1 to 4 carbon atoms, or halo, vi) cycloalkenylene of 4 to 9 carbon atoms unsubstituted or substituted with 1 to 2 substituents each selected independently from nitro, cyano, trifluoromethyl, carbethoxy, carbomethoxy, carbopropoxy, acetyl, carbamoyl, acetoxy, carboxy, hydroxy, amino, substituted amino, alkyl of 1 to 4 carbon atoms, alkoxy of 1 to 4 carbon atoms, or halo, or vii) o-phenylene unsubstituted or substituted with 1 to 2 substituents each selected independently from nitro, cyano, trifluoromethyl, carbethoxy, carbomethoxy, carbopropoxy, acetyl, carbamoyl, acetoxy, carboxy, hydroxy, amino, substituted amino, alkyl of 1 to 4 carbon atoms, alkoxy 1 to 4 carbon atoms, or halo; and,
R⁴ is -CX, or -CH₂-;
X is O or S.

Other PDE4 modulators include, but are not limited to, substituted 1,3,4-oxadiazoles (for example, 2-[1-(3-cyclopentyloxy-4-methoxyphenyl)-2-(1,3,4-oxadiazole-2-yl)ethyl]-5-methylisoindoline-1,3-dione) disclosed in U.S. patent no. 6,326,388. Representative compounds are of formula: 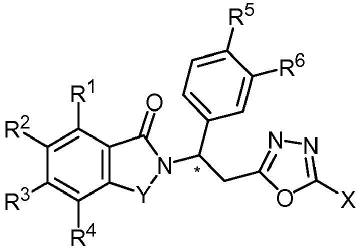 wherein:
the carbon atom designated* constitutes a center of chirality;
Y is C=O, CH₂, SO₂ or CH₂C=O;
X is hydrogen, or alkyl of 1 to 4 carbon atoms;
each of R¹, R², R³, and R⁴, independently of the others, is hydrogen, halo, trifluoromethyl, acetyl, alkyl of 1 to 8 carbon atoms, alkoxy of 1 to 4 carbon atoms, nitro, cyano, hydroxy, -CH₂NR⁸R⁹, -(CH₂)₂NR⁸R⁹, or -NR⁸R⁹ or
any two of R¹, R², R³, and R⁴ on adjacent carbon atoms, together with the depicted benzene ring are naphthylidene, quinoline, quinoxaline, benzimidazole, benzodioxole or 2-hydroxybenzimidazole;
each of R⁵ and R⁶, independently of the other, is hydrogen, alkyl of 1 to 4 carbon atoms, alkoxy of 1 to 6 carbon atoms, cyano, benzocycloalkoxy, cycloalkoxy of up to 18 carbon atoms, bicyloalkoxy of up to 18 carbon atoms, tricylcoalkoxy of up to 18 carbon atoms, or cycloalkylalkoxy of up to 18 carbon atoms;
each of R⁸ and R⁹, taken independently of the other is hydrogen, straight or branched alkyl of 1 to 8 carbon atoms, phenyl, benzyl, pyridyl, pyridylmethyl, or one of R⁸ and R⁹ is hydrogen and the other is -COR¹⁰, or -SO₂R¹⁰, or R⁸ and R⁹ taken together are tetramethylene, pentamethylene, hexamethylene, -CH=NCH=CH-, or-CH₂CH₂X¹CH₂CH₂- in which X¹ is -O-, -S-, or -NH-,
R¹⁰ is hydrogen, alkyl of 1 to 8 carbon atoms, cycloalkyl, cycloalkylmethyl of up to 6 carbon atoms, phenyl, pyridyl, benzyl, imidazolylmethyl, pyridylmethyl, NR¹¹R¹², CH₂R¹⁴R¹⁵, or NR¹¹R¹²,
wherein R¹⁴ and R¹⁵, independently of each other, are hydrogen, methyl, ethyl, or propyl, and
wherein R¹¹ and R¹², independently of each other, are hydrogen, alkyl of 1 to 8 carbon atoms, phenyl, or benzyl; and
the acid addition salts of said compounds which contain a nitrogen atom susceptible of protonation.

In other described aspects, the PDE4 modulators are of formula: 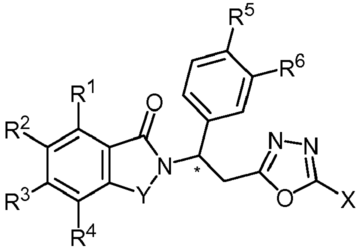 wherein:
the carbon atom designated* constitutes a center of chirality;
Y is C=O, CH₂, SO₂ or CH₂C=O;
X is hydrogen, or alkyl of 1 to 4 carbon atoms;
   (i) each of R¹, R², R³, and R⁴, independently of the others, is hydrogen, halo, trifluoromethyl, acetyl, alkyl of 1 to 8 carbon atoms, alkoxy of 1 to 4 carbon atoms, nitro, cyano, hydroxy, -CH₂NR⁸R⁹, -(CH₂)₂NR⁸R⁹, or -NR⁸R⁹ or
   (ii) any two of R¹, R², R³, and R⁴ on adjacent carbon atoms, together with the depicted benzene ring to which they are bound are naphthylidene, quinoline, quinoxaline, benzimidazole, benzodioxole or 2-hydroxybenzimidazole;
each of R⁵ and R⁶, independently of the other, is hydrogen, alkyl of 1 to 4 carbon atoms, alkoxy of 1 to 6 carbon atoms, cyano, benzocycloalkoxy, cycloalkoxy of up to 18 carbon atoms, bicyloalkoxy of up to 18 carbon atoms, tricylcoalkoxy of up to 18 carbon atoms, or cycloalkylalkoxy of up to 18 carbon atoms;
   (i) each of R⁸ and R⁹, independently of the other, is hydrogen, alkyl of 1 to 8 carbon atoms, phenyl, benzyl, pyridyl, pyridylmethyl, or
   (ii) one of R⁸ and R⁹ is hydrogen and the other is -COR¹⁰, or -SO₂R¹⁰, in which R¹⁰ is hydrogen, alkyl of 1 to 8 carbon atoms, cycloalkyl, cycloalkylmethyl of up to 6 carbon atoms, phenyl, pyridyl, benzyl, imidazolylmethyl, pyridylmethyl, NR¹¹R¹², or CH₂NR¹⁴R¹⁵, wherein R¹¹ and R¹², independently of each other, are hydrogen, alkyl of 1 to 8 carbon atoms, phenyl, or benzyl and R¹⁴ and R¹⁵, independently of each other, are hydrogen, methyl, ethyl, or propyl; or
   (iii) R⁸ and R⁹ taken together are tetramethylene, pentamethylene, hexamethylene, -CH=NCH=CH-, or -CH₂CH₂X¹CH₂CH₂- in which X¹ is -O-, -S-, or -NH-.

Other specific PDE4 modulators include, but are not limited to, cyano and carboxy derivatives of substituted styrenes (for example, 3,3-bis-(3,4-dimethoxyphenyl) acrylonitrile) disclosed in U.S. patent nos. 5,929,117, 6,130,226, 6,262,101 and 6,479,551. Representative compounds are of formula: 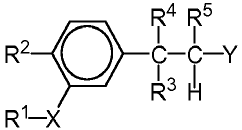 wherein:
(a) X is -O- or -(CₙH₂ₙ)- in which n has a value of 0, 1, 2, or 3, and R¹ is alkyl of one to 10 carbon atoms, monocycloalkyl of up to 10 carbon atoms, polycycloalkyl of up to 10 carbon atoms, or benzocyclic alkyl of up to 10 carbon atoms, or
(b) X is -CH= and R¹ is alkylidene of up to 10 carbon atoms, monocycloalkylidene of up to 10 carbon atoms, or bicycloalkylidene of up to 10 carbon atoms;
R² is hydrogen, nitro, cyano, trifluoromethyl, carbethoxy, carbomethoxy, carbopropoxy, acetyl, carbamoyl, acetoxy, carboxy, hydroxy, amino, lower alkyl, lower alkylidenemethyl, lower alkoxy, or halo;
R³ is (i) phenyl, unsubstituted or substituted with 1 or more substituents each selected independently from nitro, cyano, halo, trifluoromethyl, carbethoxy, carbomethoxy, carbopropoxy, acetyl, carbamoyl, carbamoyl substituted with alkyl of 1 to 3 carbon atoms, acetoxy, carboxy, hydroxy, amino, amino substituted with an alkyl of 1 to 5 carbon atoms, alkyl of up to 10 carbon atoms, cycloalkyl of up to 10 carbon atoms, alkoxy of up to 10 carbon atoms, cycloalkoxy of up to 10 carbon atoms, alkylidenemethyl of up to 10 carbon atoms, cycloalkylidenemethyl of up to 10 carbon atoms, phenyl, or methylenedioxy; (ii) pyridine, substituted pyridine, pyrrolidine, imidizole, naphthalene, or thiophene; (iii) cycloalkyl of 4-10 carbon atoms, unsubstituted or substituted with 1 or more substituents each selected independently from the group consisting of nitro, cyano, halo, trifluoromethyl, carbethoxy, carbomethoxy, carbopropoxy, acetyl, carbamoyl, acetoxy, carboxy, hydroxy, amino, substituted amino, alkyl of 1 to 10 carbon atoms, alkoxy of 1 to 10 carbon atoms, phenyl;
each of R⁴ and R⁵ taken individually is hydrogen or R⁴ and R⁵ taken together are a carbon-carbon bond;
Y is -COZ, -C≡N, or lower alkyl of 1 to 5 carbon atoms;
Z is -OH, -NR⁶R⁶, -R⁷, or -OR⁷; R⁶ is hydrogen or lower alkyl; and R⁷ is alkyl or benzyl.

In other described aspects, the PDE4 modulators are of formula: 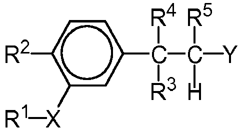 wherein:
(a) X is -O- or -(CₙH₂ₙ)- in which n has a value of 0, 1, 2, or 3, and R¹ is alkyl of one to 10 carbon atoms, monocycloalkyl of up to 10 carbon atoms, polycycloalkyl of up to 10 carbon atoms, or benzocyclic alkyl of up to 10 carbon atoms, or
(b) X is -CH= and R¹ is alkylidene of up to 10 carbon atoms, monocycloalkylidene of up to 10 carbon atoms, or bicycloalkylidene of up to 10 carbon atoms;
R² is hydrogen, nitro, cyano, trifluoromethyl, carbethoxy, carbomethoxy, carbopropoxy, acetyl, carbamoyl, acetoxy, carboxy, hydroxy, amino, lower alkyl, lower alkylidenemethyl, lower alkoxy, or halo;
R³ is pyrrolidine, imidazole or thiophene unsubstituted or substituted with 1 or more substituents each selected independently from the group consisting of nitro, cyano, halo, trifluoromethyl, carbethoxy, carbomethoxy, carbopropoxy, acetyl, carbamoyl, acetoxy, carboxy, hydroxy, amino, substituted amino, alkyl of 1 to 10 carbon atoms, alkoxy of 1 to 10 carbon atoms, or phenyl;
each of R⁴ and R⁵ taken individually is hydrogen or R⁴ and R⁵ taken together are a carbon-carbon bond;
Y is -COZ, -C≡N, or lower alkyl of 1 to 5 carbon atoms;
Z is -OH, -NR⁶R⁶, -R⁷, or -OR⁷; R⁶ is hydrogen or lower alkyl; and R⁷ is alkyl or benzyl.

In certain described aspects, the PDE4 modulators are nitriles of the formula: 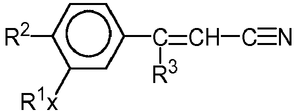 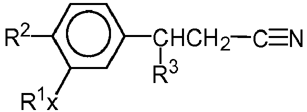 wherein:
(a) X is -O- or -(CₙH₂ₙ)- in which n has a value of 0, 1, 2, or 3, and R¹ is alkyl of up to 10 carbon atoms, monocycloalkyl of up to 10 carbon atoms, polycycloalkyl of up to 10 carbon atoms, or benzocyclic alkyl of up to 10 carbon atoms, or
(b) X is -CH=, and R¹ is alkylidene of up to 10 carbon atoms or monocycloalkylidene of up to 10 carbon atoms;
R² is hydrogen, nitro, cyano, trifluoromethyl, carbethoxy, carbomethoxy, carbopropoxy, acetyl, carbamoyl, acetoxy, carboxy, hydroxy, amino, lower alkyl, lower alkoxy, or halo; and
R³ is (i) phenyl or naphthyl, unsubstituted or substituted with 1 or more substituents each selected independently from nitro, cyano, halo, trifluoromethyl, carbethoxy, carbomethoxy, carbopropoxy, acetyl, carbamoyl, or carbamoyl substituted with alkyl of 1 to 3 carbon atoms, acetoxy, carboxy, hydroxy, amino, amino substituted with an alkyl of 1 to 5 carbon atoms, alkoxy or cycloalkoxy of 1 to 10 carbon atoms; or (ii) cycloalkyl of 4 to 10 carbon atoms, unsubstituted or substituted with one or more substituents each selected independently from the group consisting of nitro, cyano, halo, trifluoromethyl, carbethoxy, carbomethoxy, carbopropoxy, acetyl, carbamoyl, acetoxy, carboxy, hydroxy, amino, substituted amino, alkyl of 1 to 10 carbon atoms, alkoxy of 1 to 10 carbon atoms, or phenyl.

An example is the nitirile of formula:

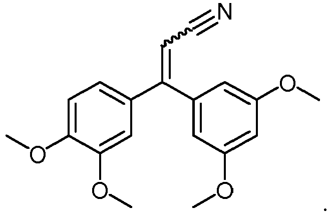

Other PDE4 modulators include, but are not limited to, isoindoline-1-one and isoindoline-1,3-dione substituted in the 2-position with an α-(3,4-disubstituted phenyl)alkyl group and in the 4- and/or 5-position with a nitrogen-containing group disclosed in WO 01/34606 and U.S. patent no. 6,667,316. Representative compounds are of formula: 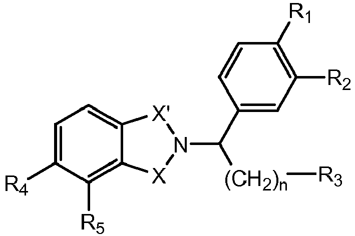 or a pharmaceutically acceptable salt and stereoisomer thereof,
wherein:
one of X and X' is =C=O or =SO₂, and the other of X and X' is =C=O, =CH₂, =SO₂ or =CH₂C=O;
n is 1, 2 or 3;
R₁ and R₂ are each independently (C₁-C₄)alkyl, (C₁-C₄)alkoxy, cyano, (C₃-C₁₈)cycloalkyl, (C₃-C₁₈)cycloalkoxy or (C₃-C₁₈)cycloalkyl-methoxy;
R₃ is SO₂-Y, COZ, CN or (C₁-C₆)hydroxyalkyl, wherein:
   Y is (C₁-C₆)alkyl, benzyl or phenyl;
   Z is -NR₆R₇, (C₁-C₆)alkyl, benzyl or phenyl;
   R₆ is H, (C₁-C₄)alkyl, (C₃-C₁₈)cycloalkyl, (C₂-C₅)alkanoyl, benzyl or phenyl, each of which can be optionally substituted with halo, amino or (C₁-C₄)alkyl-amino;
   R₇ is H or (C₁-C₄)alkyl;
   R₄ and R₅ are taken together to provide -NH-CH₂-R₈-, NH-CO-R₈-, or -N=CH-R₈-, wherein:
      R₈ is CH₂, O, NH, CH=CH, CH=N, or N=CH; or
      one of R₄ and R₅ is H, and the other of R₄ and R₅ is imidazoyl, pyrrolyl, oxadiazolyl, triazolyl, or a structure of formula (A), 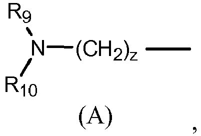 wherein:
         z is 0 or 1;
         R₉ is: H; (C₁-C₄)alkyl, (C₃-C₁₈)cycloalkyl, (C₂-C₅)alkanoyl, or (C₄-C₆)cycloalkanoyl, optionally substituted with halo, amino, (C₁-C₄)alkyl-amino, or (C₁-C₄)dialkyl-amino; phenyl; benzyl; benzoyl; (C₂-C₅)alkoxycarbonyl; (C₃-C₅)alkoxyalkylcarbonyl; N-morpholinocarbonyl; carbamoyl; N-substituted carbamoyl substituted with (C₁-C₄)alkyl; or methylsulfonyl; and
         R₁₀ is H, (C₁-C₄)alkyl, methylsulfonyl, or (C₃-C₅)alkoxyalkylcarbonyl; or
      R₉ and R₁₀ are taken together to provide -CH=CH-CH=CH-, -CH=CH-N=CH-, or (C₁-C₂)alkylidene, optionally substituted with amino, (C₁-C₄)alkyl-amino, or (C₁-C₄)dialkyl-amino; or
      R₄ and R₅ are both structures of formula (A).

In one described aspect, z is not 0 when *(i)* R³ is -SO₂-Y, -COZ, or -CN and *(ii)* one of R⁴ or R⁵ is hydrogen. In another described aspect, R⁹ and R¹⁰, taken together, is-CH=CH-CH=CH-, -CH=CH-N=CH-, or (C₁-C₂)alkylidene substituted by amino, (C₁-C₄)alkyl-amino, or (C₁-C₄)dialkyl-amino. In another described aspect, R₄ and R₅ are both structures of formula (A).
Exemplary compounds are of formula: 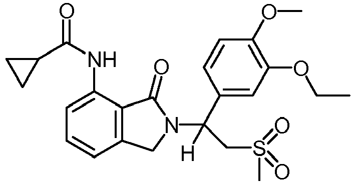 and the enantiomers thereof. Further exemplary compounds are of formulas: 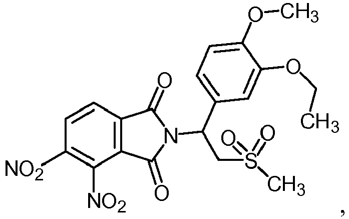 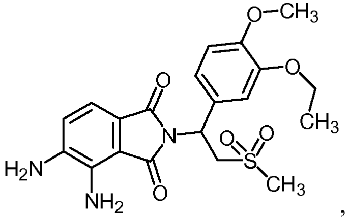 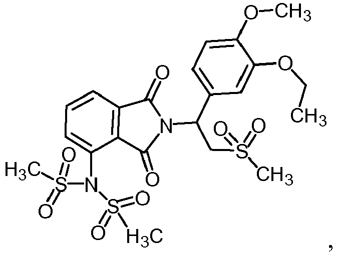 and

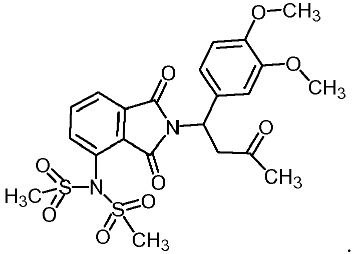

Further examples include, but are not limited to: 2-[1-(3-Ethoxy-4-methoxyphenyl)-2-methylsulfonylethyl]-4,5-dinitroisoindoline-1,3-dione; 2-[1-(3-Ethoxy-4-methoxyphenyl)-2-methylsulfonylethyl]-4,5-diaminoisoindoline-1,3-dione; 7-[1-(3-Ethoxy-4-methoxyphenyl)-2-methylsulfonylethyl]-3 -pyrrolino [3,4-e]benzimidazole-6,8-dione; 7-[1-(3-Ethoxy-4-methoxyphenyl)-2-methylsulfonylethyl]hydro-3-pyrrolino[3,4-e]benzimidazole-2,6,8-trione; 2-[1-(3-Ethoxy-4-methoxyphenyl)-2-methylsulfonylethyl]-3-pyrrolino[3,4-f]quinoxaline-1,3-dione; Cyclopropyl-N-{2-[1-(3-ethoxy-4-methoxyphenyl)-2-methylsulfonylethyl]-1,3-dioxoisoindolin-4-yl}carboxamide; 2-Chloro-N-{2-[1-(3-ethoxy-4-methoxyphenyl)-2-methylsulfonylethyl]-1,3-dioxoisoindolin-4-yl}acetamide; 2-Amino-N-{2-[1-(3-ethoxy-4-methoxyphenyl)-2-methylsulfonylethyl]-1,3-dioxoisoindolin-4-yl}acetamide; 2-N,N-Dimethylamino-N-{2-[-(3-ethoxy-4-methoxyphenyl)-2-methylsulfonylethyl]-1,3-dioxoisoindolin-4-yl} acetamide; N-{2-[1-(3-ethoxy-4-methoxyphenyl)-2-methylsulfonylethyl]-1,3-dioxoisoindolin-4-yl}-2,2,2-trifluoroacetamide; N-{2-[1-(3-Ethoxy-4-methoxyphenyl)-2-methylsulfonylethyl]-1,3-dioxoisoindolin-4-yl}methoxycarboxamide; 4-[1-Aza-2-(dimethylamino)vinyl]-2-[1-(3-ethoxy-4-methoxyphenyl)-2-methylsulfonylethyl]isoindoline-1,3-dione; 4-[1-Aza-2-(dimethylamino)prop-1-enyl]-2-[1-(3-ethoxy-4-methoxyphenyl)-2-methylsulfonylethyl]isoindoline-1,3-dione; 2-[1-(3-Ethoxy-4-methoxyphenyl)-2-methylsulfonylethyl]-4-(5-methyl-1,3,4-oxadiazol-2-yl)isoindoline-1,3-dione; 2-[1-(3-Ethoxy-4-methoxyphenyl)-2-methylsulfonylethyl]-4-pyrrolylisoindoline-1,3-dione; 4-(Aminomethyl)-2-[1-(3-ethoxy-4-methoxyphenyl)-2-methylsulfonylethyl]-isoindoline-1,3-dione; 2-[1-(3-Ethoxy-4-methoxyphenyl)-2-methylsulfonylethyl]-4-(pyrrolylmethyl)isoindoline-1,3-dione; N-{2-[1-(3-ethoxy-4-methoxyphenyl)-3-hydroxybutyl]-1,3-dioxoisoindolin-4-yl} acetamide; N-{2-[1-(3-Ethoxy-4-methoxyphenyl)-3-oxobutyl]-1,3-dioxoisoindolin-4-yl}acetamide; N-{2-[1R-(3-ethoxy-4-methoxyphenyl)-3-hydroxybutyl]-1,3-dioxoisoindolin-4-yl}acetamide; N-{2-[1R-(3-ethoxy-4-methoxyphenyl)-3-oxobutyl]-1,3-dioxoisoindolin-4-yl} acetamide; N-{2-[1S-(3-Ethoxy-4-methoxyphenyl)-3-hydroxybutyl]-1,3-dioxoisoindolin-4-yl}acetamide; N-{2-[1S-(3-ethoxy-4-methoxyphenyl)-3-oxobutyl]-1,3-dioxoisoindolin-4-yl}acetamide; 4-Amino-2-[1-(3-ethoxy-4-methoxyphenyl)-3-hydroxybutylisoindoline-1,3-dione; 4-Amino-2-[1-(3-ethoxy-4-methoxyphenyl)-3-oxobutyl]isoindoline-1,3-dione; 2-[1-(3-Ethoxy-4-methoxyphenyl)-3-oxobutyl]-4-pyrrolylisoindoline-1,3-dione; 2-Chloro-N-{2-[1-(3-ethoxy-4-methoxyphenyl)-3-oxobutyl]-1,3-dioxoisoindol-4-yl}acetamide; 2-(Dimethylamino)-N-{2-[1-(3-ethoxy-4-methoxyphenyl)-3-oxobutyl]-1,3-dioxoisoindolin-4-yl}acetamide; 4-Amino-2-[1R-(3-ethoxy-4-methoxyphenyl)-3-hydroxybutyl]isoindoline-1,3-dione; 4-Amino-2-[1R-(3-ethoxy-4-methoxyphenyl)-3-oxobutyl]isoindoline-1,3-dione; 2-[1R-(3-ethoxy-4-methoxyphenyl)-3-oxobutyl]-4-pyrrolylisoindoline-1,3-dione; 2-(Dimethylamino)-N-{2-[1R-(3-ethoxy-4-methoxyphenyl)-3-oxobutyl]-1,3-dioxoisoindolin-4-yl}acetamide; Cyclopentyl-N-{2-[1-(3-ethoxy-4-methoxyphenyl)-2-(methylsulfonyl)ethyl]-1,3-dioxoisoindolin-4-yl}carboxamide; 3-(Dimethylamino)-N-{2-[1-(3-ethoxy-4-methoxyphenyl)-2-(methylsulfonyl)ethyl]-1,3-dioxoisoindolin-4-yl}propanamide; 2-(Dimethylamino)-N-{2-[1-(3-ethoxy-4-methoxyphenyl)-2-(methylsulfonyl)ethyl]-1,3-dioxoisoindolin-4-yl}propanamide; N-{2-[(1R)-1-(3-ethoxy-4-methoxyphenyl)-2-(methylsulfonyl)ethyl]-1,3-dioxoisoindolin-4-yl}-2-(dimethylamino)acetamide; N-{2-[(1S)-1-(3-ethoxy-4-methoxyphenyl)-2-(methylsulfonyl)ethyl]-1,3-dioxoisoindolin-4-yl}-2-(dimethylamino)acetamide; 4-{3-[(Dimethylamino)methyl]pyrrolyl}-2-[1-(3-ethoxy-4-methoxyphenyl)-2-(methylsulfonyl)ethyl]isoindoline-1,3-dione; Cyclopropyl-N-{2-[(1S)-1-(3-ethoxy-4-methoxyphenyl)-2-(methylsulfonyl)ethyl]-1,3-dioxoisoindolin-4-yl}carboxamide; 2-[1-(3,4-dimethoxyphenyl)-2-(methylsulfonyl)ethyl]-4-pyrrolylisoindoline-1,3-dione; N-{2-[1-(3,4-dimethoxyphenyl)-2-(methylsulfonyl)ethyl]-1,3-dioxoisoindolin-4-yl}-2-(dimethylamino)acetamide; Cyclopropyl-N-{2-[1-(3,4-dimethoxyphenyl)-2-(methylsulfonyl)ethyl]-1,3-dioxoisoindolin-4-yl}carboxamide; Cyclopropyl-N-{2-[1-(3-ethoxy-4-methoxyphenyl)-2-(methylsulfonyl)ethyl]-3-oxoisoindolin-4-yl}carboxamide; 2-(Dimethylamino)-N-{2-[1-(3-ethoxy-4-methoxyphenyl)-2-(methylsulfonyl)ethyl]-3-oxoisoindolin-4-yl}acetamide; Cyclopropyl-N-{2-[(1S)-1-(3-ethoxy-4-methoxyphenyl)-2-(methylsulfonyl)ethyl]-3-oxoisoindolin-4-yl}carboxamide; Cyclopropyl-N-{2-[(1R)-1-(3-ethoxy-4-methoxyphenyl)-2-(methylsulfonyl)ethyl]-3-oxoisoindolin-4-yl}carboxamide; (3R)-3-[7-(Acetylamino)-1-oxoisoindolin-2-yl]-3-(3-ethoxy-4-methoxyphenyl)-N,N-dimethylpropanamide; (3R)-3-[7-(Cyclopropylcarbonylamino)-1-oxoisoindolin-2-yl]-3-(3-ethoxy-4-methoxyphenyl)-N,N-dimethylpropanamide; 3-{4-[2-(Dimethylamino)acetylamino]-1,3-dioxoisoindolin-2-yl}-3-(3-ethoxy-4-methoxyphenyl)-N,N-dimethylpropanamide; (3R)-3-[7-(2-Chloroacetylamino)-1-oxoisoindolin-2-yl]-3-(3-ethoxy-4-methoxy-phenyl)-N,N-dimethylpropanamide; (3R)-3-{4-[2-(dimethylamino)acetylamino]-1,3-dioxoisoindolin-2-yl}-3-(3-ethoxy-4-methoxyphenyl)-N,N-dimethylpropanamide; 3-(1,3-Dioxo-4-pyrrolylisoindolin-2-yl)-3-(3-ethoxy-4-methoxyphenyl)-N,N-dimethylpropanamide; 2-[1-(3-Ethoxy-4-methoxyphenyl)-2-(methylsulfonyl)ethyl]-4-(imidazolyl-methyl)isoindoline-1,3-dione; N-({2-[1-(3-Ethoxy-4-methoxyphenyl)-2-(methylsulfonyl)ethyl]-1,3-dioxoisoindolin-4-yl}methyl)acetamide; 2-Chloro-N-({2-[1-(3-ethoxy-4-methoxyphenyl)-2-(methylsulfonyl)ethyl]-1,3-dioxoisoindolin-4-yl}methyl)acetamide; 2-(Dimethylamino)-N-({2-[1-(3-ethoxy-4-methoxyphenyl)-2-(methylsulfonyl)ethyl]-1,3-dioxoisoindolin-4-yl}methyl)acetamide; 4-[Bis(methylsulfonyl)amino]-2-[1-(3-ethoxy-4-methoxyphenyl)-2-(methylsulfonyl)ethyl]isoindoline-1,3-dione; 2-[1-(3-Ethoxy-4-methoxyphenyl)-2-(methylsulfonyl)ethyl]-4-[(methylsulfonyl)amino]isoindoline-1,3-dione; N-{2-[1-(3-Ethoxy-4-methoxyphenyl)-3-hydroxypentyl]-1,3-dioxoisoindolin-4-yl}acetamide; N- {2-[1-(3-Ethoxy-4-methoxyphenyl)-3-oxopentyl]1,3-dioxoisoindolin-4-yl}acetamide; 2-[(1R)-1-(3-Ethoxy-4-methoxyphenyl)-3-hydroxybutyl]-4-(pyrrolylmethyl)isoindoline-1,3-dione; 2-[(1R)-1-(3-Ethoxy-4-methoxyphenyl)-3-oxobutyl]-4-(pyrrolylmethyl)isoindoline-1 ,3-dione; N-{2-[1-(3-Cyclopentyloxy-4-methoxyphenyl)-3-hydroxybutyl]-1,3-dioxoisomdolin-4-yl}acetamide; N-{2-[1-(3-Cyclopentyloxy-4-methoxyphenyl)-3-oxobutyl]-1,3-dioxoisoindolin-4-yl}acetamide; 2-[1-(3-Cyclopentyloxy-4-methoxyphenyl)-3-oxobutyl]-4-pyrrolylisoindoline-1,3-dione; 2-[1-(3,4-Dimethoxyphenyl)-3-oxobutyl]-4-[bis(methylsulfonyl)amino]isoindoline-1,3-dione; and pharmaceutically acceptable salts, solvates, and stereoisomers thereof.

Still other PDE 4 modulators include, but are not limited to, imido and amido substituted acylhydroxamic acids (for example, (3-(1,3-dioxoisoindoline-2-yl)-3-(3-ethoxy-4-methoxyphenyl) propanoylamino) propanoate disclosed in WO 01/45702 and U.S. patent no. 6,699,899. Representative compounds are of formula: 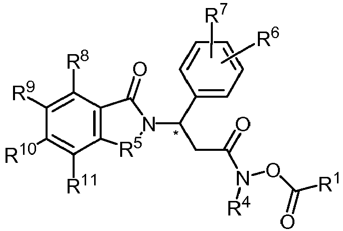 wherein:
the carbon atom designated * constitutes a center of chirality,
R⁴ is hydrogen or -(C=O)-R¹²,
each of R¹ and R¹², independently of each other, is alkyl of 1 to 6 carbon atoms, phenyl, benzyl, pyridyl methyl, pyridyl, imidazoyl, imidazolyl methyl, or
CHR*(CH₂)ₙNR*R⁰,
wherein R* and R⁰, independently of the other, are hydrogen, alkyl of 1 to 6 carbon atoms, phenyl, benzyl, pyridyl methyl, pyridyl, imidazoyl or imidazolylmethyl, and n = 0, 1, or 2;
R⁵ is C=O, CH₂, CH₂-CO-, or SO₂;
each of R⁶ and R⁷, independently of the other, is nitro, cyano, trifluoromethyl, carbethoxy, carbomethoxy, carbopropoxy, acetyl, carbamoyl, acetoxy, carboxy, hydroxy, amino, alkyl of 1 to 6 carbon atoms, alkoxy of 1 to 6 carbon atoms, cycloalkoxy of 3 to 8 carbon atoms, halo, bicycloalkyl of up to 18 carbon atoms, tricycloalkoxy of up to 18 carbon atoms, 1-indanyloxy, 2-indanyloxy, C₄-C₈-cycloalkylidenemethyl, or C₃-C₁₀-alkylidenemethyl;
each of R⁸, R⁹, R¹⁰, and R¹¹, independently of the others, is
   (i) hydrogen, nitro, cyano, trifluoromethyl, carbethoxy, carbomethoxy, carbopropoxy, acetyl, carbamoyl, acetoxy, carboxy, hydroxy, amino, alkylamino, dialkylamino, acylamino, alkyl of 1 to 10 carbon atoms, alkoxy of 1 to 10 carbon atoms, halo, or
   (ii) one of R⁸, R⁹, R¹⁰, and R¹¹ is acylamino comprising a lower alkyl, and the remaining of R⁸, R⁹, R¹⁰, and R¹¹ are hydrogen, or
   (iii) hydrogen if R⁸ and R⁹ taken together are benzo, quinoline, quinoxaline, benzimidazole, benzodioxole, 2-hydroxybenzimidazole, methylenedioxy, dialkoxy, or dialkyl, or
   (iv) hydrogen if R¹⁰ and R¹¹, taken together are benzo, quinoline, quinoxaline, benzimidazole, benzodioxole, 2-hydroxybenzimidazole, methylenedioxy, dialkoxy, or dialkyl, or
   (v) hydrogen if R⁹ and R¹⁰ taken together are benzo.

Other PDE 4 modulators include, but are not limited to, 7-amido-isoindolyl compounds disclosed in U.S. patent publication no. 2004/0254214. Representative compounds are of formula: 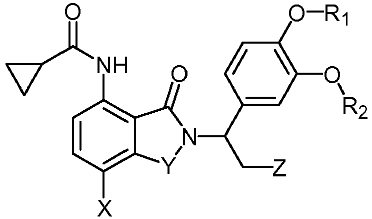 wherein:
Y is -C(O)-, -CH₂, -CH₂C(O)- or SO₂;
X is H;
Z is (C₀₋₄-alkyl)-C(O)R³, C₁₋₄-alkyl, (C₀₋₄-alkyl)-OH, (C₁₋₄-alkyl)-O(C₁₋₄-alkyl), (C₁₋₄-alkyl)-SO₂(C₁₋₄-alkyl), (C₀₋₄-alkyl)-SO(C₁₋₄-alkyl), (C₀₋₄-alkyl)-NH₂, (C₀₋₄-alkyl)-N(C₁₋₈akyl)₂, (C₀₋₄-alkyl)-N(H)(OH), or CH₂NSO₂(C₁₋₄-alkyl);
R₁ and R₂ are independently C₁₋₈-alkyl, cycloalkyl, or (C₁₋₄-alkyl)cycloalkyl;
R³ is, NR⁴ R⁵, OH, or O-(C₁₋₈-alkyl);
R⁴ is H;
R⁵ is -OH, or -OC(O)R⁶;
R⁶ is C₁₋₈-alkyl, amino-(C₁₋₈-alkyl), (C₁₋₈-alkyl)-(C₃₋₆-cycloalkyl), C₃₋₆-cycloalkyl, phenyl, benzyl, or aryl;
or a pharmaceutically acceptable salt, solvate, hydrate, stereoisomer, clathrate, or prodrug thereof; or formula: 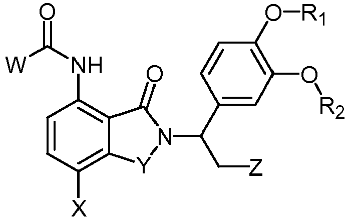 wherein:
   Y is -C(O)-, -CH₂, -CH₂C(O)-, or SO₂;
   X is halogen, -CN, -NR₇R₈, -NO₂, or -CF₃;
   Z is (C₀₋₄alkyl)-SO₂(C₁₋₄-alkyl), -(C₀₋₄-alkyl)-CN, -(C₀₋₄-alkyl)-C(O)R³, C₁₋₄-alkyl, (C₀₋₄₋alkyl)OH, (C₀₋₄-alkyl)O(C₁₋₄-alkyl), (C₀₋₄-alkyl)SO(C₁₋₄-alkyl), (C₀₋₄-alkyl)NH₂, (C₀₋₄-alkyl)N(C₁₋₈-alkyl)₂, (C₀₋₄-alkyl) N(H)(OH), (C₀₋₄-alkyl)-dichloropyridine or (C₀₋₄-alkyl)NSO₂(C₁₋₄-alkyl);
   W is -C₃₋₆-cycloalkyl, -(C₁₋₈-alkyl)-(C₃₋₆-cycloalkyl), -(C₀₋₈-alkyl)-(C₃₋₆-cycloalkyl)NR₇R₈, (C₀₋₈-alkyl)-NR₇R₈, (C₀₋₄alkyl)-CHR₉-(C₀₋₄alkyl)-NR₇R₈;
   R₁ and R₂ are independently C₁₋₈-alkyl, cycloalkyl, or (C₁₋₄-alkyl)cycloalkyl;
   R³ is C₁₋₈-alkyl, NR⁴R⁵, OH, or O-(C₁₋₈-alkyl);
   R⁴ and R⁵ are independently H, C₁₋₈-alkyl, (C₀₋₈-alkyl)-(C₃₋₆-cycloalkyl), OH, or -OC(O)R⁶;
   R⁶ is C₁₋₈-alkyl, (C₀₋₈-alkyl)-(C₃₋₆-cycloalkyl), amino-(C₁₋₈-alkyl), phenyl, benzyl, or aryl;
   R₇ and R₈ are each independently H, C₁₋₈-alkyl, (C₀₋₈-alkyl)-(C₃₋₆-cycloalkyl), phenyl, benzyl, aryl, or can be taken together with the atom connecting them to form a 3 to 7 membered heterocycloalkyl or heteroaryl ring;
   R₉ is C₁₋₄ alkyl, (C₀₋₄alkyl)aryl, (C₀₋₄alkyl)-(C₃₋₆-cycloalkyl), (C₀₋₄alkyl)-heterocylcle; or a pharmaceutically acceptable salt, solvate, hydrate, stereoisomer, clathrate, or prodrug thereof.

In one described aspect, W is

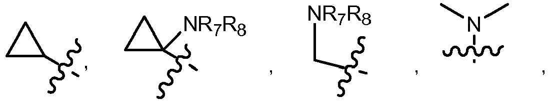

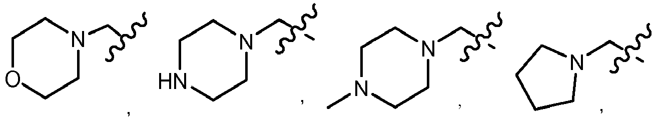

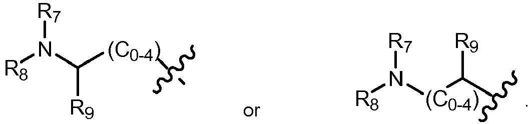

In another described aspect, representative compounds are of formula: 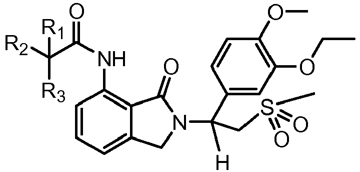 wherein:
R₁, R₂ and R₃ are independently H or C₁₋₈-alkyl, with the proviso that at least one of R₁, R₂ and R₃ is not H;
or a pharmaceutically acceptable salt, solvate, hydrate, stereoisomer, clathrate, or prodrug thereof.

Other PDE 4 modulators include, but are not limited to, isoindoline compounds disclosed in U.S. patent publication no. 2006/0025457. Representative compounds are listed in Table 1 below, for which pharmaceutically acceptable prodrugs, salts, solvates, and stereoisomers thereof are also encompassed:

**Table 1.**

| **No.** | **Structure** | **No.** | **Structure** |
|---|---|---|---|
| 1 | 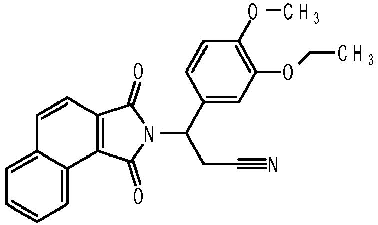 | 2 | 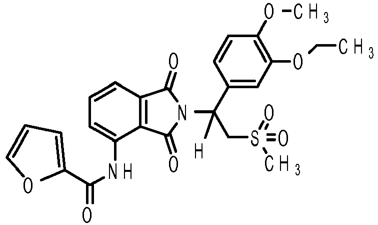 |
| 3 | 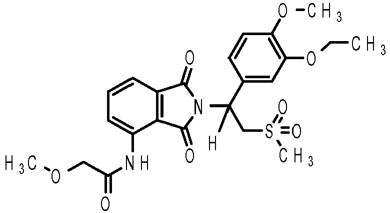 | 4 | 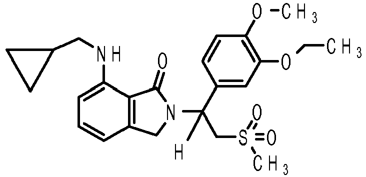 |
| 5 | 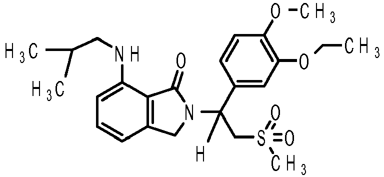 | 6 | 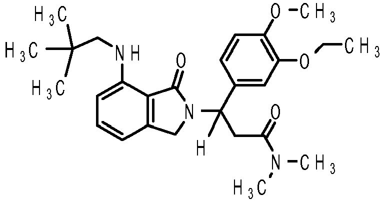 |
| 7 | 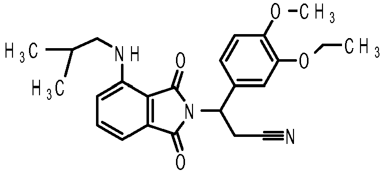 | 8 | 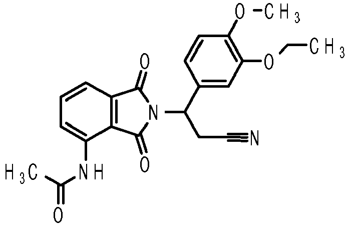 |
| 9 | 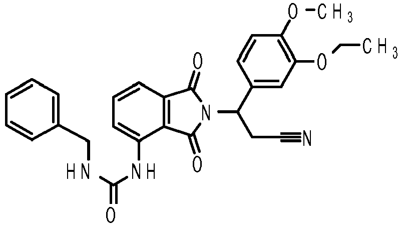 | 10 | 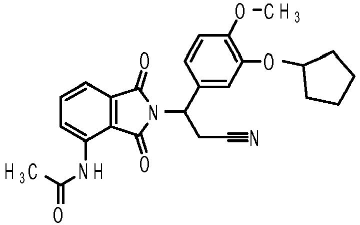 |
| 11 | 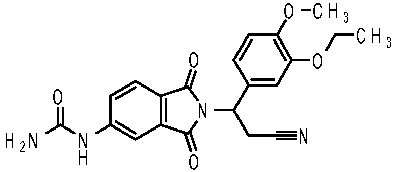 | 12 | 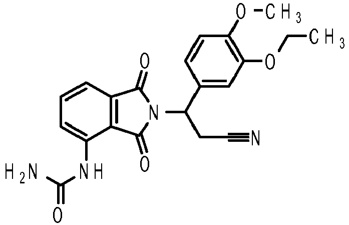 |
| 13 | 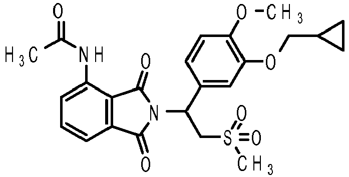 | 14 | 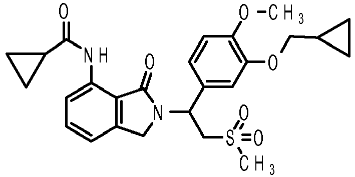 |
| 15 | 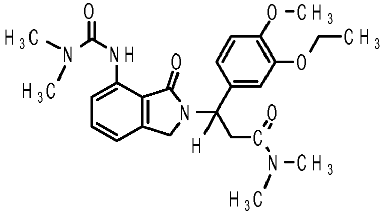 | 16 | 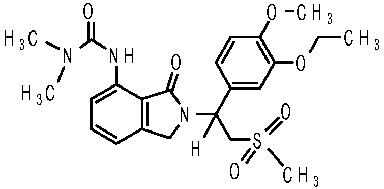 |

In another described aspect, also discussed are 2-[1-(3-ethoxy-4-methoxyphenyl)-2-methylsulfonylethyl]-4,5-dinitroisoindoline-1,3-dione and its acid addition salts. In another described aspect, a hydrochloride salt of 2-[1-(3-ethoxy-4-methoxyphenyl)-2-methylsulfonylethyl]-4,5-dinitroisoindoline-1,3-dione is also provided.

Other PDE 4 modulators include, but are not limited to, isoindoline compounds disclosed in U.S. patent publication no. 2006/0084815. Representative compounds are cyclopropanecarboxylic acid {2-[1-(3-ethoxy-4-methoxy-phenyl)-2-[1,3,4]oxadiazol-2-yl-ethyl]-3-oxo-2,3-dihydro-1H-isoindol-4-yl}-amide, which has the following chemical structure, and pharmaceutically acceptable salts, solvates, prodrugs, and stereoisomers thereof:

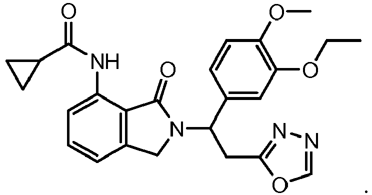

Other PDE 4 modulators include, but are not limited to, N-alkyl-hydroxamic acid-isoindolyl compounds disclosed in U.S. patent publication no. 2004/0259873. Representative compounds are of formula: 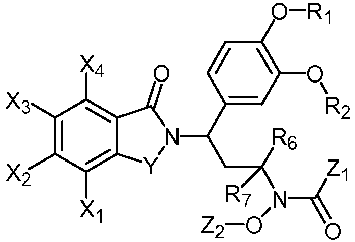 wherein:
Y is -C(O)-, -CH₂, -CH₂C(O)- or SO₂;
R₁ and R₂ are independently C₁₋₈-alkyl, CF₂H, CF₃, CH₂CHF₂, cycloalkyl, or (C₁₋₈-alkyl)cycloalkyl;
Z₁ is H, C₁₋₆-alkyl, -NH₂ -NR₃R₄ or OR₅;
Z₂ is H or C(O)R₅;
X₁, X₂, X₃ and X₄ are each independent H, halogen, NO₂, OR₃, CF₃, C₁₋₆-alkyl, (C₀₋₄-alkyl)-(C₃₋₆-cycloalkyl), (C₀₋₄-alkyl)-N-(R₈R₉), (C₀₋₄-alkyl)-NHC(O)-(R₈), (C₀₋₄-alkyl)-NHC(O)CH(R₈)(R₉), (C₀₋₄-alkyl)-NHC(O)N(R₈R₉), (C₀₋₄-alkyl)-NHC(O)O(R₈), (C₀₋₄-alkyl)-O-R₈, (C₀₋₄-alkyl)-imidazolyl, (C₀₋₄-alkyl)-pyrrolyl, (C₀₋₄-alkyl) oxadiazolyl, (C₀₋₄-alkyl)-triazolyl or (C₀₋₄-alkyl)-heterocycle;
R₃, R₄, and R₅ are each independently H, C₁₋₆-alkyl, O-C₁₋₆-alkyl, phenyl, benzyl, or aryl;
R₆ and R₇ are independently H or C₁₋₆-alkyl;
R₈ and R₉ are each independently H, C₁₋₉-alkyl, C₃₋₆-cycloalkyl, (C₁₋₆-alkyl)-(C₃₋₆cycloalkyl), (C₀₋₆-alkyl)-N(R₄R₅), (C₁₋₆-alkyl)-OR₅, phenyl, benzyl, aryl, piperidinyl, piperizinyl, pyrolidinyl, morpholino, or C₃₋₇-heterocycloalkyl;
or a pharmaceutically acceptable salt, solvate, hydrate, stereoisomer, clathrate, or prodrug thereof.

Other PDE 4 modulators include, but are not limited to, diphenylethylene compounds disclosed in U.S. patent publication no. 2005/0014727. Representative compounds are of formula: 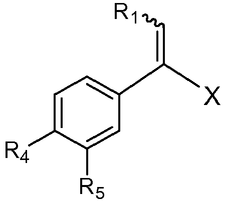 or a pharmaceutically acceptable salt, solvate, or hydrates thereof,
wherein:
R₁ is -CN, lower alkyl, -COOH, -C(O)-N(R₉)₂, -C(O)-lower alkyl, -C(O)-benzyl, -C(O)O-lower alkyl, -C(O)O-benzyl;
R₄ is -H, -NO₂, cyano, substituted or unsubstituted lower alkyl, substituted or unsubstituted alkoxy, halogen, -OH, -C(O)(R₁₀)₂, -COOH, -NH₂, -OC(O)-N(R₁₀)₂;
R₅ is substituted or unsubstituted lower alkyl, substituted or unsubstituted alkoxy, or substituted or unsubstituted alkenyl;
X is substituted or unsubstituted phenyl, substituted or unsubstituted pyridine, substituted or unsubstituted pyrrolidine, substituted or unsubstituted imidizole, substituted or unsubstituted naphthalene, substituted or unsubstituted thiophene, or substituted or unsubstituted cycloalkyl;
each occurrence of R₉ is independently -H or substituted or unsubstituted lower alkyl; and
each occurrence of R₁₀ is independently -H or substituted or unsubstituted lower alkyl.

In another described aspect, representative compounds are of formula: 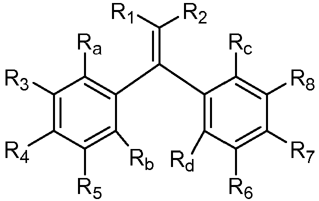 or a pharmaceutically acceptable salt, solvate, or hydrate thereof,
wherein:
R₁ and R₂ are independently -H, -CN, substituted or unsubstituted lower alkyl, substituted or unsubstituted alkenyl, substituted or unsubstituted alkynyl, -COOH,-C(O)-lower alkyl, -C(O)O-lower alkyl, -C(O)-N(R₉)₂, substituted or unsubstituted aryl, or substituted or unsubstituted heterocycle;
each occurrence of Rₐ, R_{b}, R_{c} and R_{d} is independently -H, substituted or unsubstituted lower alkyl, substituted or unsubstituted aryl, substituted or unsubstituted heterocycle, substituted or unsubstituted cycloalkyl, substituted or unsubstituted alkoxy, halogen, cyano, -NO₂, -OH, -OPO(OH)₂, -N(R₉)₂, -OC(O)-R₁₀, -OC(O)-R₁₀-N(R₁₀)₂, -C(O)N(R₁₀)₂, -NHC(O)-R₁₀, -NHS(O)₂-R₁₀, -S(O)₂-R₁₀, -NHC(O)NH-R₁₀, -NHC(O)N(R₁₀)₂, -NHC(O)NHSO₂-R₁₀, -NHC(O)-R₁₀-N(R₁₀)₂, -NHC(O)CH(R₁₀)(N(R₉)₂) or -NHC(O)-R₁₀-NH₂;
R₃ is -H, substituted or unsubstituted lower alkyl, substituted or unsubstituted aryl, substituted or unsubstituted heterocycle, substituted or unsubstituted cycloalkyl, substituted or unsubstituted alkoxy, halogen, cyano, -NO₂, -OH, -OPO(OH)₂, -N(R₉)₂, -OC(O)-R₁₀, -OC(O)-R₁₀-N(R₁₀)₂, -C(O)N(R₁₀)₂, -NHC(O)-R₁₀, -NHS(O)₂-R₁₀, -S(O)₂-R₁₀, -NHC(O)NH-R₁₀, -NHC(O)N(R₁₀)₂, -NHC(O)NHSO₂-R₁₀, -NHC(O)-R₁₀-N(R₁₀)₂, -NHC(O)CH(R₁₀)(N(R₉)₂) or -NHC(O)-R₁₀-NH₂, or R₃ with either Rₐ or with R₄, together form -O-C(R₁₆R₁₇)-O- or -O-(C(R₁₆R₁₇))₂-O-;
R₄ is -H, substituted or unsubstituted lower alkyl, substituted or unsubstituted aryl, substituted or unsubstituted heterocycle, substituted or unsubstituted cycloalkyl, substituted or unsubstituted alkoxy, halogen, cyano, -NO₂, -OH, -OPO(OH)₂, -N(R₉)₂, -OC(O)-R₁₀, -OC(O)-R₁₀-N(R₁₀)₂, -C(O)N(R₁₀)₂, -NHC(O)-R₁₀, -NHS(O)₂-R₁₀, -S(O)₂-R₁₀, -NHC(O)NH-R₁₀, -NHC(O)N(R₁₀)₂, -NHC(O)NHSO₂-R₁₀, -NHC(O)-R₁₀-N(R₁₀)₂, -NHC(O)CH(R₁₀)(N(R₉)₂) or -NHC(O)-R₁₀-NH₂;
R₅ is -H, substituted or unsubstituted lower alkyl, substituted or unsubstituted aryl, substituted or unsubstituted heterocycle, substituted or unsubstituted cycloalkyl, substituted or unsubstituted alkoxy, halogen, cyano, -NO₂, -OH, -OPO(OH)₂, -N(R₉)₂,-OC(O)-R₁₀, -OC(O)-R₁₀-N(R₁₀)₂, -C(O)N(R₁₀)₂, -NHC(O)-R₁₀, -NHS(O)₂-R₁₀, -S(O)₂-R₁₀, -NHC(O)NH-R₁₀, -NHC(O)N(R₁₀)₂, -NHC(O)NHSO₂-R₁₀, -NHC(O)-R₁₀-N(R₁₀)₂, -NHC(O)CH(R₁₀)(N(R₉)₂) or -NHC(O)-R₁₀-NH₂;
R₆ is -H, substituted or unsubstituted lower alkyl, substituted or unsubstituted aryl, substituted or unsubstituted heterocycle, substituted or unsubstituted cycloalkyl, substituted or unsubstituted alkoxy, halogen, cyano, -NO₂, -OH, -OPO(OH)₂, -N(R₉)₂, -OC(O)-R₁₀, -OC(O)-R₁₀-N(R₁₀)₂, -C(O)N(R₁₀)₂, -NHC(O)-R₁₀, -NHS(O)₂-R₁₀, -S(O)₂-R₁₀, -NHC(O)NH-R₁₀, -NHC(O)N(R₁₀)₂, -NHC(O)NHSO₂-R₁₀, -NHC(O)-R₁₀-N(R₁₀)₂, -NHC(O)CH(R₁₀)(N(R₉)₂) or -NHC(O)-R₁₀-NH₂;
R₇ is -H, substituted or unsubstituted lower alkyl, substituted or unsubstituted aryl, substituted or unsubstituted heterocycle, substituted or unsubstituted cycloalkyl, substituted or unsubstituted alkoxy, halogen, cyano, -NO₂, -OH, -OPO(OH)₂, -N(R₉)₂, -OC(O)-R₁₀, -OC(O)-R₁₀-N(R₁₀)₂, -C(O)N(R₁₀)₂, -NHC(O)-R₁₀, -NHS(O)₂-R₁₀, -S(O)₂-R₁₀, -NHC(O)NH-R₁₀, -NHC(O)N(R₁₀)₂, -NHC(O)NHSO₂-R₁₀, -NHC(O)-R₁₀-N(R₁₀)₂, -NHC(O)CH(R₁₀)(N(R₉)₂) or -NHC(O)-R₁₀-NH₂;
R₈ is -H, substituted or unsubstituted lower alkyl, substituted or unsubstituted aryl, substituted or unsubstituted heterocycle, substituted or unsubstituted cycloalkyl, substituted or unsubstituted alkoxy, halogen, cyano, -NO₂, -OH, -OPO(OH)₂, -N(R₉)₂, -OC(O)-R₁₀, -OC(O)-R₁₀-N(R₁₀)₂, -C(O)N(R₁₀)₂, -NHC(O)-R₁₀, -NHS(O)₂-R₁₀, -S(O)₂-R₁₀, -NHC(O)NH-R₁₀, -NHC(O)N(R₁₀)₂, -NHC(O)NHSO₂-R₁₀, -NHC(O)-R₁₀-N(R₁₀)₂, -NHC(O)CH(R₁₀)(N(R₉)₂) or -NHC(O)-R₁₀-NH₂, or R₈ with either R_{c} or with R₇, together form -O-C(R₁₆R₁₇)-O- or -O-(C(R₁₆R₁₇))₂-O-;
each occurrence of R₉ is independently -H, substituted or unsubstituted lower alkyl, or substituted or unsubstituted cycloalkyl;
each occurrence of R₁₀ is independently substituted or unsubstituted lower alkyl, substituted or unsubstituted cycloalkyl, substituted or unsubstituted aryl, substituted or unsubstituted lower hydroxyalkyl, or R₁₀ and a nitrogen to which it is attached form a substituted or unsubstituted heterocycle, or R₁₀ is -H where appropriate; and
each occurrence of R₁₆ and R₁₇ is independently -H or halogen.

In one described aspect, compounds provided herein are 2-[1-(3-ethoxy-4-methoxyphenyl)-2-methylsulfonylethyl]-4-acetylaminoisoindoline-1,3-dione and cyclopropyl-N-{2-[1-(3-ethoxy-4-methoxyphenyl)-2-(methylsulfonyl)ethyl]-3-oxoisoindolin-4-yl}carboxamide, which respectively have the following structures: 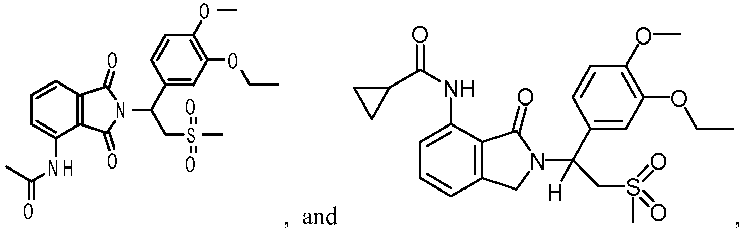 or a pharmaceutically acceptable salt, solvate, or prodrug thereof. In another described aspect, stereoisomers of these compounds are also encompassed.

All of the compounds described can either be commercially purchased or prepared according to the methods described in the patents or patent publications disclosed herein. Further, optically pure compounds can be asymmetrically synthesized or resolved using known resolving agents or chiral columns as well as other standard synthetic organic chemistry techniques.

Compounds used herein may be small organic molecules having a molecular weight less than about 1,000 g/mol, and are not proteins, peptides, oligonucleotides, oligosaccharides or other macromolecules.

In one specific described aspect, the PDE4 modulator is (S)-N-(2-(1-(3-ethoxy-4-methoxyphenyl)-2-(methylsulfonyl)ethyl)-1,3-dioxoisoindolin-4-yl)acetamide: 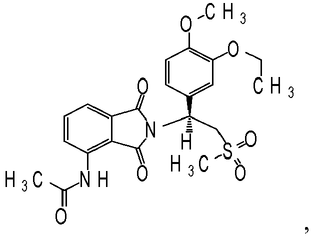 or a pharmaceutically acceptable salt, solvate or stereoisomer thereof.

In another embodiment, the PDE4 modulator is cyclopropanecarboxylic acid {2-[(1S)-1-(3-ethoxy-4-methoxy-phenyl)-2-methanesulfonyl-ethyl]-3-oxo-2,3-dihydro-1H-isoindol-4-yl}-amide: 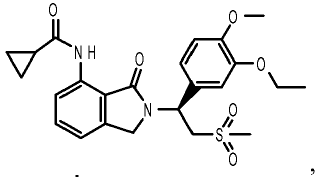 or a pharmaceutically acceptable salt or solvate thereof.

It should be noted that if there is a discrepancy between a depicted structure and a name given that structure, the depicted structure is to be accorded more weight. In addition, if the stereochemistry of a structure or a portion of a structure is not indicated with, for example, bold or dashed lines, the structure or portion of the structure is to be interpreted as encompassing all stereoisomers of it.

### 4.5 Methods of Detecting mRNA or Protein Levels in a Sample

Any suitable method of detecting differences the levels of mRNA or protein biomarkers can be used. In some embodiments, the biomarker to be detected is an mRNA molecule. In other embodiments, the method of measuring gene or protein expression can involve methods such as cDNA hybridization, flow cytometry, immunofluorescence, immunoblots, ELISAs or microspotted-antibody immunofluorescence assays, an antibody-based dipstick assay, cytometric bead arrays, or other common mRNA or protein detecting methods.

### 4.5.1 Methods of Detecting mRNA Levels in a Sample

Several methods of detecting or quantitating mRNA levels are known in the art. Exemplary methods include but are not limited to northern blots, ribonuclease protection assays, PCR-based methods, and the like. When the biomarker is an mRNA molecule, the mRNA sequence, or a fragment thereof, can be used to prepare a probe that is at least partially complementary. The probe can then be used to detect the mRNA sequence in a sample, using any suitable assay, such as PCR-based methods, Northern blotting, a dipstick assay, and the like.

In other embodiments, a nucleic acid assay for testing for immunomodulatory activity in a biological sample can be prepared. An assay typically contains a solid support and at least one nucleic acid contacting the support, where the nucleic acid corresponds to at least a portion of an mRNA that has altered expression during an immunomodulatory treatment in a patient. The assay can also have a means for detecting the altered expression of the mRNA in the sample.

The assay method can be varied depending on the type of mRNA information desired. Exemplary methods include but are not limited to Northern blots and PCR-based methods (*e.g.*, qRT-PCR). Methods such as qRT-PCR can also accurately quantitate the amount of the mRNA in a sample.

Any suitable assay platform can be used to determine the presence of the mRNA in a sample. For example, an assay may be in the form of a dipstick, a membrane, a chip, a disk, a test strip, a filter, a microsphere, a slide, a multiwell plate, or an optical fiber. An assay system may have a solid support on which a nucleic acid corresponding to the mRNA is attached. The solid support may comprise, for example, a plastic, silicon, a metal, a resin, glass, a membrane, a particle, a precipitate, a gel, a polymer, a sheet, a sphere, a polysaccharide, a capillary, a film a plate, or a slide. The assay components can be prepared and packaged together as a kit for detecting an mRNA.

The nucleic acid can be labeled, if desired, to make a population of labeled mRNAs. In general, a sample can be labeled using methods that are well known in the art (*e.g*., using DNA ligase, terminal transferase, or by labeling the RNA backbone, etc.; *see, e.g.,* Ausubel, et al., Short Protocols in Molecular Biology, 3rd ed., Wiley & Sons 1995 and Sambrook et al., Molecular Cloning: A Laboratory Manual, Third Edition, 2001 Cold Spring Harbor, N.Y.). In some embodiments, the sample is labeled with fluorescent label. Exemplary fluorescent dyes include but are not limited to xanthene dyes, fluorescein dyes, rhodamine dyes, fluorescein isothiocyanate (FITC), 6 carboxyfluorescein (FAM), 6 carboxy-2',4',7',4,7-hexachlorofluorescein (HEX), 6 carboxy 4', 5' dichloro 2', 7' dimethoxyfluorescein (JOE or J), N,N,N',N' tetramethyl 6 carboxyrhodamine (TAMRA or T), 6 carboxy X rhodamine (ROX or R), 5 carboxyrhodamine 6G (R6G5 or G5), 6 carboxyrhodamine 6G (R6G6 or G6), and rhodamine 110; cyanine dyes, *e.g.* Cy3, Cy5 and Cy7 dyes; Alexa dyes, *e.g.* Alexa-fluor-555; coumarin, Diethylaminocoumarin, umbelliferone; benzimide dyes, *e.g.* Hoechst 33258; phenanthridine dyes, e.g. Texas Red; ethidium dyes; acridine dyes; carbazole dyes; phenoxazine dyes; porphyrin dyes; polymethine dyes, BODIPY dyes, quinoline dyes, Pyrene, Fluorescein Chlorotriazinyl, R110, Eosin, JOE, R6G, Tetramethylrhodamine, Lissamine, ROX, Napthofluorescein, and the like.

The nucleic acids may be present in specific, addressable locations on a solid support; each corresponding to at least a portion of mRNA sequences that are differentially expressed upon treatment of an immunomodulatory compound in a cell or a patient.

A typical mRNA assay method can contain the steps of 1) obtaining surface-bound subject probes; 2) hybridization of a population of mRNAs to the surface-bound probes under conditions sufficient to provide for specific binding (3) post-hybridization washes to remove nucleic acids not bound in the hybridization; and (4) detection of the hybridized mRNAs. The reagents used in each of these steps and their conditions for use may vary depending on the particular application.

Hybridization can be carried out under suitable hybridization conditions, which may vary in stringency as desired. Typical conditions are sufficient to produce probe/target complexes on a solid surface between complementary binding members, i.e., between surface-bound subject probes and complementary mRNAs in a sample. In certain embodiments, stringent hybridization conditions may be employed.

Hybridization is typically performed under stringent hybridization conditions. Standard hybridization techniques (*e.g.* under conditions sufficient to provide for specific binding of target mRNAs in the sample to the probes) are described in Kallioniemi et al., Science 258:818-821 (1992) and WO 93/18186. Several guides to general techniques are available, *e.g.,* Tijssen, Hybridization with Nucleic Acid Probes, Parts I and II (Elsevier, Amsterdam 1993). For descriptions of techniques suitable for *in situ* hybridizations, see Gall et al. Meth. Enzymol., 21:470-480 (1981); and Angerer et al. in Genetic Engineering: Principles and Methods (Setlow and Hollaender, Eds.) Vol 7, pgs 43-65 (Plenum Press, New York 1985). Selection of appropriate conditions, including temperature, salt concentration, polynucleotide concentration, hybridization time, stringency of washing conditions, and the like will depend on experimental design, including source of sample, identity of capture agents, degree of complementarity expected, etc., and may be determined as a matter of routine experimentation for those of ordinary skill in the art.

Those of ordinary skill will readily recognize that alternative but comparable hybridization and wash conditions can be utilized to provide conditions of similar stringency.

After the mRNA hybridization procedure, the surface bound polynucleotides are typically washed to remove unbound nucleic acids. Washing may be performed using any convenient washing protocol, where the washing conditions are typically stringent, as described above. The hybridization of the target mRNAs to the probes is then detected using standard techniques.

### 4.5.2 PCR-based methods of detecting mRNA biomarkers

Other methods, such as PCR-based methods, can also be used to follow the expression of the mRNA biomarkers. Examples of PCR methods can be found in the literature. Examples of PCR assays can be found in U.S. Patent No. 6,927,024. Examples of RT-PCR methods can be found in U.S. Patent No. 7,122,799. A method of fluorescent in situ PCR is described in U.S. Patent No. 7,186,507.

In some embodiments, Real-Time Reverse Transcription-PCR (qRT-PCR) can be used for both the detection and quantification of RNA targets (Bustin, et al., 2005, Clin. Sci., 109:365-379). Quantitative results obtained by qRT-PCR are generally more informative than qualitative data. Thus, in some embodiments, qRT-PCR-based assays can be useful to measure mRNA levels during cell-based assays. The qRT-PCR method is also useful to monitor patient therapy. Examples of qRT-PCR-based methods can be found, for example, in U.S. Patent No. 7,101,663.

In contrast to regular reverse transcriptase-PCR and analysis by agarose gels, real-time PCR gives quantitative results. An additional advantage of real-time PCR is the relative ease and convenience of use. Instruments for real-time PCR, such as the Applied Biosystems 7500, are available commercially, as are the reagents, such as TaqMan Sequence Detection chemistry. For example, TaqMan® Gene Expression Assays can be used, following the manufacturer's instructions. These kits are pre-formulated gene expression assays for rapid, reliable detection and quantification of human, mouse and rat mRNA transcripts. An exemplary PCR program, for example, is 50°C for 2 minutes, 95°C for 10 minutes, 40 cycles of 95°C for 15 seconds, then 60°C for 1 minute.

To determine the cycle number at which the fluorescence signal associated with a particular amplicon accumulation crosses the threshold (referred to as the CT), the data can be analyzed, for example, using a 7500 Real-Time PCR System Sequence Detection software v1.3 using the comparative CT relative quantification calculation method. Using this method, the output is expressed as a fold-change of expression levels. In some embodiments, the threshold level can be selected to be automatically determined by the software. In some embodiments, the threshold level is set to be above the baseline but sufficiently low to be within the exponential growth region of an amplification curve.

### 4.5.3 Methods of detecting polypeptide or protein biomarkers

When the biomarker is a protein, several protein detection and quantitation methods can be used to measure the presence of the biomarker. Any suitable protein quantitation method can be used. In some embodiments, antibody-based methods are used. Exemplary methods that can be used include but are not limited to immunoblotting (western blot), enzyme-linked immunosorbent assay (ELISA), immunohistochemistry, flow cytometry, cytometric bead array, mass spectroscopy, and the like. Several types of ELISA are commonly used, including direct ELISA, indirect ELISA, and sandwich ELISA.

### 4.6 Kits for Detecting mRNA Biomarkers

In some described aspects, a kit for detecting the mRNA biomarkers can be prepared. The kits can include, for example, a probe or probe set comprising oligonucleotides that can bind to the mRNA biomarker(s) of interest for psoriasis. Washing solutions, reagents for performing a hybridization assay, mRNA isolation or purification means, detection means, as well as positive and negative controls can also be included. The kit can also include instructions for using the components of the kit. The kit can be tailored for in-home use, clinical use, or research use.

### 4.7 Kits for Detecting Polypeptide or Protein Biomarkers

In some described aspects, a kit for detecting protein levels can be prepared. The kits can include, for example, a dipstick coated with an antibody that recognizes the protein, washing solutions, reagents for performing the assay, protein isolation or purification means, detection means, as well as positive and negative controls. The kit can also include instructions for using the components of the kit. The kit can be tailored for in-home use, clinical use, or research use.

### 5. EXAMPLES

The examples below are carried out using standard techniques, which are well known and routine to those of skill in the art, except where otherwise described in detail. The examples are intended to be merely illustrative.

### 5.1 Biomarkers for Psoriasis in Histological Analysis

Skin biopsies were evaluated on 20 patients in a clinical trial, where 20 mg of (S)-N-(2-(1-(3-ethoxy-4-methoxyphenyl)-2-(methylsulfonyl)ethyl)-1,3-dioxoisoindolin-4-yl)acetamide was administered b.i.d. to patients with psoriasis. Skin biopsies were done 4 weeks and 12 weeks post-treatment.

The biopsies were subjected to a histological analysis in which response was assessed in H&E stained sections of skin biopsies and after staining frozen sections of skin biopsies with antibodies to keratin 16, CD3, CD11c, ICAM-1, Langerin, CD56, Foxp3, and HLA-DR. This analysis thus included an assessment of epidermal growth/differentiation, skin infiltration by T-cells and DC subsets, presence of regulatory T-cells, and presence of inflammation-regulating molecules in skin lesions.

Of the 20 cases analyzed, 19 showed good or active psoriasis lesions in baseline biopsies. One had minimally reactive epidermis in the baseline lesional biopsy and thus did not meet histologic criteria for active psoriasis vulgaris in the baseline biopsy. At day 29, 10 cases showed improvement in psoriasis, based on a reduction in epidermal hyperplasia and/or a reduction in keratin 16 staining. Using all measured values of thickness, there was a 23% reduction in epidermal thickness at week 4 (p = 0.07 or approaching statistical significance in a 2-sided test). Nine cases showed disease improvement in day 85 biopsies, with 5 cases showing absence of keratin 16 staining. At this timepoint, the median reduction in epidermal thickness was 34% (p-value 0.049). Hence, the quantitative reduction in epidermal thickness was significant for the group as a whole. It is very unusual that psoriasis shows spontaneous improvement, so the 5 cases where epidermis became K16- (keratin 16 negative) are highly significant in qualitative terms.

As to the infiltrating leukocytes in these biopsies, T-cell reductions were measured in day 29 and day 85 biopsies, but the heterogeneity of the response did not lead to statistical significance of the overall reductions. CD11c marks myeloid dendritic cells in human skin. Normally, there is a resident population of CD11c+ cells in the dermis, but psoriasis shows both an increase in dermal CD11c+ DCs and inappropriate migration of CD11c+ cells into the epidermis of skin lesions. Dermal CD11c+ DCs were reduced in week 4 and week 12 biopsies, and the 45% median reduction in week 4 biopsies was highly significant (p = 0.001). Epidermal CD11c+ infiltrates were even more strongly reduced (73-84%) at weeks 4 and 12, respectively. Both sets of reductions were highly significant (0.001 or better). This may indicate that the treatment compound has stronger suppressive effects on myeloid leukocytes, compared to effects on T-cells. CD56+ cells (NK cells or NK-T-cells) were minimally altered during treatment, *i.e.,* between week 4 and week 12. A small increase in epidermal Langerhan's cells was noted in day 85 biopsies, consistent with normalization of this cell population with effective therapy.

The expression of inflammation-associated molecules ICAM-1 and HLA-DR was reduced in parallel with disease improvement reflected by epidermal thickness or K16 staining. These were qualitative markers of inflammation, so the change has not been quantified and subjected to statistical analysis. However, one case showed a good example of progressive reductions in ICAM-1 and HLA-DR staining in parallel with reductions in epidermal hyperplasia.

The presence of Foxp3+ cells in the dermis of skin lesions was reduced over time in parallel with reductions in T-cells. One case showed a good example of this outcome. This suggests that the mechanism of action is more related to suppression of active inflammation, rather than suppression of inflammation through an increased Treg T-cell population.

The results observed in certain histological analysis are summarized below:

**Table 2**

| Histologic Measurement | Week 4 | | Week 12 | |
|---|---|---|---|---|
| | Median % change from baseline | P | Median % change from baseline | P |
| CD11c myeloid DC in Dermis | -45.8 | 0.001 | -54.6 | 0.363 |
| CD11c myeloid DC in Epidermis | -73.1 | 0.001 | -88.6 | >0.001 |
| CD3 T cells in Dermis | -24.5 | 0.82 | -62.0 | 0.255 |
| CD3 T cells in Epidermis | -35.0 | 0.286 | -47.4 | 0.136 |
| CD56 NK cells in Dermis | -27.6 | 0.544 | -12.5 | 0.656 |
| CD56 NK cells in Epidermis | -75.6 | 0.004 | -73.3 | 0.499 |
| Langerin in Dermis | -50.0 | 0.456 | -57.9 | 0.474 |
| Langerin in Epidermis | 9.5 | 0.082 | 17.1 | 0.026 |
| Epidermal Thickness | -22.9 | 0.070 | -34.3 | 0.049 |

### 5.2 Biomarkers in Genomic Analysis

mRNA abundance for a variety of inflammatory molecules was measured by real-time RT-PCR and expression was normalized to the house-keeping gene HARP (human acidic ribosomal protein). Inflammatory markers assessed by mRNA levels included the chemokine CXCL9, beta-defensin (DEFB4), interferon-gamma, IL-10, IL-17a, IL-22, IL-8, keratin 16, MX-1, IL-12/23 p40, IL-23 p19, iNOS and TNF. These are inflammatory molecules produced by activated DC populations, Th1, Th17, Th22 T-cells, and response genes to interferon (MX-1, CXCL9) or IL-17 (defensin). Keratin 16 was also measured by mRNA levels to assess the epidermal response by an alternate means.

At week 12, there was a median reduction in K16 mRNA by 78% (p = 0.045), which confirms the overall improvement of psoriasis as assessed by epidermal thickness measures at this same time-point. The reduction in K16 mRNA was of higher magnitude than the reduction in epidermal thickness, as this keratin is produced only in reactive (hyperplastic) epidermis. Normal epidermis has a thickness value, so the maximal case for a thickness reduction is to normal values.

From the histologic analysis, CD11c+ myeloid leukocytes showed more consistent reductions than T-cells in lesions. From the genomic standpoint, iNOS, p40, and p19 genes are products of inflammatory (CD11c+) DCs. Normalized iNOS mRNA expression was reduced by 61% at week 4 (p=0.015) and by 100% at week 12 (p=0.005). This is consistent with the core drug mechanism of reducing bioactive TNF in inflammatory skin lesions.

Another TNF-induced gene in CD11c+ DCs is the IL-12/23 p40 gene. One p40 custom probe set showed significant reductions in week 4 and week 12 biopsies. Without being limited by a particular theory, the prediction of this reduction is that levels of IL-12 and/or IL-23 would be reduced, with subsequent reductions in Th1, Th17, and Th22 T-cell activation, followed by reductions in downstream genes of IL-17 or interferon signaling. Normalized IL-17A mRNA levels were reduced by 49% at week 12 (p=0.023), and normalized IL-22 mRNA levels were reduced by 100% at week 12 (p=0.001).

DEFB4 is a defensin induced in keratinocytes by IL-17. Normalized expression of DEFB4 was reduced by 47% at week 4 (p=0.036) and by 45% in week 12 biopsies (p=0.011). IL-8 is also induced in keratinocytes by IL-17. Normalized expression of this inflammatory chemokine was reduced by 76% in week 4 biopsies (p=0.001) and by 66% in week 12 biopsies (p=0.017). Without being limited by a particular theory, the larger magnitude reduction in IL-8 (as compared to DEFB4), probably reflects the co-regulation of IL-8 by TNF and the fact that TNF signaling is probably reduced by the study drug.

Reduction in normalized MX-1 mRNA in biopsies was also consistently observed: median reduction of 51% at week 4 (p=0.005) and a reduction of 52% at week 12 (p<0.001). This most likely reflects a reduction in other interferons (Type 1 interferons) that were not directly measured by genomic probes.

The results are summarized in the following table:

**Table 3**

| mRNA | Week 4 | | Week 12 | |
|---|---|---|---|---|
| | Median % change from baseline (normalized to hARP) | P | Median % change from baseline (normalized to hARP) | P |
| IL-12/IL-23 p40 | -100 | 0.003 | -86.7 | 0.023 |
| iNOS | -61.0 | 0.015 | -100 | 0.005 |
| IL-22 | -74.4 | 0.900 | -100 | 0.001 |
| IL-8 | -76.4 | 0.001 | -66.5 | 0.017 |
| DEFB4 | -55.4 | 0.032 | -82.3 | 0.002 |
| MX-1 | -51.1 | 0.005 | -52.6 | <0.001 |
| K-16 | -62.0 | 0.143 | -78.6 | 0.045 |
| IL-17A | -72.5 | 0.091 | -49.4 | 0.023 |
| IL-23p19 | -53.4 | 0.333 | -68.3 | 0.257 |
| IL-10 | -49.1 | 0.871 | -26.5 | 0.912 |
| IFN-g | -38.2 | 0.221 | -37.6 | 0.812 |
| CXCL9 (MIG) | -30.7 | 0.701 | -36.4 | 0.538 |

### 5.3 Treatment of Psoriasis

The pharmacodynamic effects of (S)-N-(2-(1-(3-ethoxy-4-methoxyphenyl)-2-(methylsulfonyl)ethyl)-1,3-dioxoisoindolin-4-yl)acetamide were studied in skin biopsies from subjects treated with the compound in a phase 2, multi-center, open-label study evaluating the safety, tolerability, and efficacy of the compound in recalcitrant psoriasis vulgaris. Skin biopsies were taken from uninvolved and involved skin at baseline, and from involved skin after treatment with (S)-N-(2-(1-(3-ethoxy-4-methoxyphenyl)-2-(methylsulfonyl)ethyl)-1,3-dioxoisoindolin-4-yl)acetamide 20 mg BID after 4 and 12 weeks.

Skin biopsies were evaluated on 20 patients in this trial. The biopsies were analyzed by immunohistochemistry for a set of cellular markers (keratin 16, CD3, CD11c, ICAM-1, Langerin, CD56, Foxp3, and HLA-DR) to assess epidermal growth/differentiation, skin infiltration by T-cells and dendritic cells (DC), presence of regulatory T-cells, and the presence of inflammation-regulating molecules in skin lesions.

Antibody staining for ICAM-1, Foxp3, and HLA-DR was qualitative and no major changes were noted for these markers. Overall, there was a major reduction in inflammatory myeloid CD11c+ DCs, also called TIP-DCs (TNF- and iNOS-producing DCs) in both the epidermis (**FIG**. **1**) and dermis (**FIG**. **3**) at both week 4 and week 12. In particular, as shown in **FIG. 1****,** pathologic infiltration of psoriatic epidermis by the myeloid DC was inhibited most strongly. There were also significant reductions in CD3+ T cells in the epidermis and dermis, and in CD56+ NK cells in the epidermis.

Additionally, the reductions observed from biopsies were evaluated separately in responders and non-responders. In general, reductions in cellular infiltrates in epidermis (**FIG**. **2**) and dermis (**FIG**. **4**) were greater among responders (those with a change in PASI of 75% or more at week 12) than among non-responders. Langerin, a marker of Langerhans cells (LC), was slightly elevated in the epidermis (**FIG**. **1**) but not the dermis (**FIG**. **3**), though this effect was not statistically significant, it was attributable mostly to the increase in LC in the epidermis among non-responders (**FIG**. **2**).

The expression of proinflammatory gene products known to be involved in the pathogenesis of psoriasis was measured by real-time polymerase chain reaction (RT-PCR). With regard to gene expression, there were reductions in iNOS and IL-12/IL-23 p40 mRNAs, along with reductions in IL-17A and IL-22 mRNAs, indicating inhibition of the Th1, Th17, and Th22 pathways (**FIG**. **5**). The IL-12/23 p40 mRNA levels showed significant reductions in week 4 and week 12 biopsies. Normalized IL-17A mRNA levels were reduced by 49% at week 12 and normalized IL-22 mRNA levels were reduced by 100% at week 12. DEFB4, a defensin induced in keratinocytes by IL-17, was reduced by 55% in week 4 biopsies and by 82% in week 12 biopsies. IL-8, which is induced in keratinocytes by IL-17, was reduced by 76% in week 4 biopsies and by 66% in week 12 biopsies.

Additionally, the reduction of gene expression was evaluated separately in responders and non-responders. In general, inhibition of gene expression was greater among responders than non-responders (**FIG**. **6**). The results of this analysis strongly support the biological activity of (S)-N-(2-(1-(3-ethoxy-4-methoxyphenyl)-2-(methylsulfonyl)ethyl)-1,3-dioxoisoindolin-4-yl)acetamide in psoriasis vulgaris by a mechanism involving inhibition of myeloid dendritic cells, and suppression of genes contributing to the Th1, Th17, and Th17 pro-inflammatory pathways.

### 5.4 Correlation of Changes in Cellular Infiltration and Gene Expression with PASI Score

To identify changes in cellular infiltration or gene expression that correlate with changes in PASI score, a correlation analysis was performed using the Spearman rank-order correlation method. As shown in Table 4, a significant correlation was observed between the decrease in CD56+ NK cells in the epidermis and the decrease in the PASI score at week 4 (p=0.009). A strong trend was observed between the decrease in CD11c+ myeloid DC in the epidermis the decrease in PASI score at week 4 (p=0.052).

**Table 4. Correlation of Percent Changes from Baseline (week 0) in PASI Score and the Associated Skin Biopsy Parameters**

| **Test** | **Week** | **No. *** | **PSAI Median Change (%)** | **Biopsy Parameters Median Change (%)** | **Spearman Rank-order Correlation Coefficient** | **p-value**** |
|---|---|---|---|---|---|---|
| CD3 in Dermis | 4 | 19 | -29.0 | -24.5 | -0.143 | 0.559 |
| | 12 | 15 | -48.0 | -63.1 | 0.211 | 0.451 |
| CD3 in Epidermis | 4 | 19 | -29.0 | -35.0 | 0.044 | 0.858 |
| | 12 | 15 | -48.0 | -56.1 | 0.304 | 0.271 |
| CD11c in Dermis | 4 | 20 | -32.0 | -45.8 | 0.302 | 0.196 |
| | 12 | 14 | -52.5 | -56.0 | 0.073 | 0.805 |
| CD11c in Epidermis | 4 | 18 | -26.5 | -73.1 | 0.465 | 0.052 |
| | 12 | 13 | -45.0 | -97.5 | 0.448 | 0.125 |
| CD56 in Dermis | 4 | 20 | -32.0 | -27.6 | 0.232 | 0.326 |
| | 12 | 15 | -48.0 | -18.2 | 0.432 | 0.108 |
| CD56 in Epidermis | 4 | 16 | -15.0 | -75.6 | 0.631 | 0.009 |
| | 12 | 12 | -42.5 | -77.6 | 0.189 | 0.557 |
| Langerin in Dermis | 4 | 17 | -18.0 | -50.0 | 0.465 | 0.06 |
| | 12 | 12 | -54.0 | -67.0 | 0.416 | 0.178 |
| Langerin in Epidermis | 4 | 20 | -32.0 | 9.49 | 0.336 | 0.148 |
| | 12 | 15 | -48.0 | 41.67 | 0.289 | 0.296 |
| Thickness | 4 | 19 | -29.0 | -22.9 | 0.051 | 0.836 |
| | 12 | 14 | -46.5 | -34.8 | 0.341 | 0.233 |

| | | | | | | |
|---|---|---|---|---|---|---|
| * Subjects with values for both PASI % change and biopsy parameters % change were included ** p-value is for testing no correlation in rank-order % changes | | | | | | |

As shown below in Table 5, among the changes in gene expression and PASI change at week 4, there was a significant correlation for DEFB4 (p=0.005), MX-1 (p=0.008) and IL-12/IL-23p40 (p=0.033). At week 12, there was a significant correlation between the drop in PASI score and the drop in expression DEFB4 (p=0.009), IL-17A (p=0.03), K16 (p=0.001), and iNOS (p=0.033). At week 12, there was also a strong trend was also observed for the decrease in PASI score and the expression of IL-8 week (p=0.051) (Table 14.2.20.4).

**Table 5. Correlation of Percent Changes from Baseline (week 0) in PASI Score and Gene Expression of the Associated Inflammatory Biomarkers**

| **Test** | **Week** | **No.*** | **PSAI Median Change (%)** | **Gene Expression Median Change (%)** | **Spearman Rank-order Correlation Coefficient** | **p-value**** |
|---|---|---|---|---|---|---|
| CD83/hARP | 4 | 15 | -18.0 | -12.4 | -0.097 | 0.732 |
| | 12 | 11 | -48.0 | 27.39 | 0 | 1 |
| CXCL9/hARP | 4 | 17 | -29.0 | -30.7 | 0.286 | 0.266 |
| | 12 | 11 | -48.0 | -15.8 | 0.182 | 0.593 |
| DEFB4/hARP | 4 | 16 | -23.5 | -55.4 | 0.669 | 0.005 |
| | 12 | 10 | -46.5 | -82.0 | 0.769 | 0.009 |
| IPNγ/hARP | 4 | 15 | -35.0 | -38.2 | 0.448 | 0.094 |
| | 12 | 12 | -54.0 | -15.3 | -0.376 | 0.185 |
| IL-10/hARP | 4 | 18 | -32.0 | -49.1 | -0.018 | 0.945 |
| | 12 | 14 | -54.0 | -15.3 | -0.376 | 0.185 |
| IL-17A/hARP | 4 | 16 | -23.5 | -72.5 | 0.424 | 0.102 |
| | 12 | 10 | -46.5 | -46.7 | 0.681 | 0.03 |
| IL-2/hARP | 4 | 12 | -32.0 | 3.33 | -0.358 | 0.253 |
| | 12 | 10 | -54.0 | -25.0 | 0.091 | 0.802 |
| IL-22/hARP | 4 | 10 | -18.5 | -74.4 | 0.323 | 0.363 |
| | 12 | 6 | -46.5 | -100 | 0.135 | 0.798 |
| IL-8/hARP | 4 | 18 | -32.0 | -76.4 | 0.076 | 0.763 |
| | 12 | 14 | -54.0 | -79.2 | 0.53 | 0.051 |
| K16/hARP | 4 | 18 | -32.0 | -62.0 | 0.419 | 0.084 |
| | 12 | 14 | -54.0 | -82.5 | 0.798 | 0.001 |
| MX1/hARP | 4 | 17 | -29.0 | -51.1 | 0.62 | 0.008 |
| | 12 | 11 | -48.0 | -51.5 | 0.018 | 0.958 |
| p40/hARP | 4 | 13 | -29.0 | -100 | 0.593 | 0.033 |
| | 12 | 7 | -48.0 | -51.5 | 0.018 | 0.958 |
| TNFα/hARP | 4 | 17 | -29.0 | -13.0 | -0.307 | 0.231 |
| | 12 | 13 | -60.0 | -41.3 | -0.049 | 0.873 |
| iNOS/hARP | 4 | 15 | -35.0 | -61.0 | 0.465 | 0.067 |
| | 12 | 11 | -48.0 | -100 | 0.642 | 0.033 |
| p19/hARP | 4 | 18 | -32.0 | -53.4 | 0.182 | 0.47 |
| | 12 | 14 | -54.0 | -67.9 | 0.411 | 0.144 |

| | | | | | | |
|---|---|---|---|---|---|---|
| * Subjects with values for both PASI % change and biopsy parameters % change were included ** p-value is for testing no correlation in rank-order % changes | | | | | | |

Additionally, in order to identify specific changes in cellular infiltration or gene expression at week 4 that might predict eventual clinical response as defined by a PASI-75 or greater at week 12, a Spearman rank-order correlation analysis was performed. For example, if the IFN-γ /hARP ratio decreases by -70% to -100% at Week 4, it was predicted that the subject will have PASI-75 response; otherwise non PASI-75 response. From this analysis, a significant correlation was found between the change in IFN-γ expression at week 4 and a later change in PASI score at week 12 (p=0.04) (**FIG. 7**). As shown in **FIG. 7****,** among four subjects who had PASI-75 response at week 12, three showed IFNγ change between -70% to -100% at week 4, indicating a 75% accuracy in this prediction model. Indeed, the three subjects who attained a PASI-75 or better at week 12 showed a 100% reduction in IFN-γ gene expression in the skin at week 4. In addition, all of the eight subjects who had non PASI-75 response at week 12 showed IFNγ change was less than 70% decrease, indicating a 100% accuracy.

Alternatively, the analysis may be set to designate PASI-75 response to be change in IFNγ expression between -60% and -100%. When this analysis was conducted, the prediction accuracies were about 75% for PASI-75 response and 80% for non PASI-75 response, respectively. Therefore, monitoring a subject for a decrease in IFN-γ expression in the skin or peripheral blood may provide a method of early prediction for eventual attainment of a beneficial clinical response.

Another useful predictive marker may be the reduction in the amount of Langerin (a marker of Langerhans cells, or LC) in the dermis at week 4. Similar to the IFNγ analysis, if the Langerin staining in the dermis is decreased by -70% to -100% at Week 4, it was predicted that the subject will have PASI-75 response; otherwise non PASI-75 response. As shown in **FIG. 8****,** among four subjects who had PASI-75 response at week 12, three showed Langerin change between -70% to -100% at week 4, indicating a 75% accuracy in this prediction model. In addition, nine out of the ten subjects who had non PASI-75 response at week 12 showed Langerin change less than 70% decrease, indicating a 90% accuracy.

## Claims

1. A method of predicting whether a patient will be responsive to a treatment for psoriasis comprising:
culturing cells from a skin sample of the patient in the presence or absence of the treatment compound;
measuring the level of a cell marker or mRNA; and
comparing the level of the cell marker or mRNA in the cells cultured in the presence of the treatment compound to that in cells cultured in the absence of the treatment compound,
wherein the treatment compound is cyclopropanecarboxylic acid {2-[(1*S*)-1-(3-ethoxy-4-methoxy-phenyl)-2-methanesulfonyl-ethyl]-3-oxo-2,3-dihydro-1*H*-isoindol-4-yl}-amide, and
wherein a decreased level of the cell marker or mRNA in the presence of the treatment compound indicates the likelihood of an effective patient response to the treatment compound,
wherein the cell marker is selected from CD3, CD56, Foxp3, Langerin and a combination thereof or
wherein the mRNA is selected from mRNA for Keratin 16, IL-12/IL-23 p40, IL-23 p19, IL-17A, IL-22, DEFB4, IL-8, MX-1, IL-10, IFN-γ, CXCL9 and a combination thereof.

2. The method of claim 1, wherein (i) the level of only one of the cell markers or mRNAs is monitored, or (ii) the levels of two or more of the cell markers or mRNAs are monitored simultaneously.

3. A method of monitoring patient response to a psoriasis treatment comprising:
measuring the level of a cell marker or mRNA in a first skin sample from the patient;
measuring the level of the same cell marker or mRNA in a second skin sample obtained after administering a treatment compound to the patient; and
comparing the levels of the cell marker or mRNA obtained from first and second skin samples,
wherein the treatment compound is cyclopropanecarboxylic acid {2-[(*1S*)-1-(3-ethoxy-4-methoxy-phenyl)-2-methanesulfonyl-ethyl]-3-oxo-2,3-dihydro-1*H*-isoindol-4-yl}-amide, and
wherein a decreased level of the cell marker in the second skin sample indicates an effective response,
wherein the cell marker is selected from CD3, CD56, Foxp3, Langerin and a combination thereof, or
wherein the mRNA is selected from mRNA for Keratin 16, IL-12/IL-23 p40, IL-23 p19, IL-17A, IL-22, DEFB4, IL-8, MX-1, IL-10, IFN-γ, CXCL9 and a combination thereof.

4. The method of claim 3, wherein the level of CD3 obtained from the dermis region is monitored, and the decrease is about 15%, 20%, 25%, or 30% or more as compared to the level of CD3 in the first skin sample.

5. The method of claim 3, wherein the level of CD3 obtained from the epidermis region is monitored, and the decrease is about 25%, 30%, 35%, or 40% or more as compared to the level of CD3 in the first skin sample.

6. The method of claim 3, wherein the level of CD56 obtained from the dermis region is monitored, and the decrease is about 15%, 20%, 25%, or 30% or more as compared to the level of CD56 in the first skin sample.

7. The method of claim 3, wherein the level of CD56 obtained from the epidermis region is monitored, and the decrease is about 60%, 65%, 70%, or 75% or more as compared to the level of CD56 in the first skin sample.

8. The method of claim 3, wherein the level of Langerin obtained from the dermis region is monitored, and the decrease is about 40%, 45%, 50%, or 55% or more as compared to the level of Langerin in the first skin sample.

9. A method for monitoring patient compliance with a drug treatment protocol for psoriasis comprising:
measuring the level of a cell marker or mRNA in a skin sample from the patient; and
determining if the expression level is decreased in the skin sample compared to the expression level in a control untreated sample,
wherein the drug treatment protocol comprises administering cyclopropanecarboxylic acid {2-[(*1S*)-1-(3-ethoxy-4-methoxy-phenyl)-2-methanesulfonyl-ethyl]-3-oxo-2,3-dihydro-1*H*-isoindol-4-yl}-amide to the patient, and
wherein a decreased expression indicates patient compliance with said drug treatment protocol
wherein the cell marker is selected from CD3, CD56, Foxp3, Langerin and a combination thereof, or
wherein the mRNA is selected from mRNA for Keratin 16, IL-12/IL-23 p40, IL-23 p19, IL-17A, IL-22, DEFB4, IL-8, MX-1, IL-10, IFN-γ, CXCL9 and a combination thereof.

10. The method of claim 3 or 9, wherein
(i) the level of IL-12/IL-13 p40 mRNA is monitored, and the decrease is about 80%, 85%, 90%, or 95% or more as compared to the level of IL-12/IL-13 p40 mRNA in the first skin sample;
(ii) the level of IL-22 mRNA is monitored, and the decrease is about 65%, 70%, 75%, or 80% or more as compared to the level of IL-22 mRNA in the first skin sample;
(iii) the level of IL-8 mRNA is monitored, and the decrease is about 65%, 70%, 75%, or 80% or more as compared to the level of IL-8 mRNA in the first skin sample;
(iv) the level of DEFB4 mRNA is monitored, and the decrease is about 45%, 50%, 55%, or 60% or more as compared to the level of DEFB4 mRNAin the first skin sample;
(v) the level of MX-1 mRNA is monitored, and the decrease is about 40%, 45%, 50%, or 55% or more as compared to the level of MX-1 mRNA in the first skin sample;
(vi) the level of Keratin 16 mRNA is monitored, and the decrease is about 50%, 55%, 60%, or 65% or more as compared to the level of Keratin 16 mRNA in the first skin sample;
(vii) the level of IL-17A mRNA is monitored, and the decrease is about 60%, 65%, 70%, or 75% or more as compared to the level of IL-17A mRNA in the first skin sample;
(viii) the level of IL-23 p19 mRNA is monitored, and the decrease is about 40%, 45%, 50%, or 55% or more as compared to the level of IL-23 p19 mRNA in the first skin sample;
(ix) the level of IL-10 mRNA is monitored, and the decrease is about 40%, 45%, 50%, or 55% or more as compared to the level of IL-10 mRNA in the first skin sample;
(x) the level of IFN-γ mRNA is monitored, and the decrease is about 30%, 35%, 40%, or 45% or more as compared to the level of IFN-γ mRNA in the first skin sample; or
(xi) the level of CXCL9 mRNA is monitored, and the decrease is about 20%, 25%, 30%, or 35% or more as compared to the level of CXCL9 mRNA in the first skin sample.

## Patentansprüche

1. Verfahren zum Prognostizieren, ob ein Patient auf eine Behandlung gegen Schuppenflechte ansprechen wird, wobei das Verfahren Folgendes umfasst:
Kultivieren von Zellen einer Hauptprobe des Patienten in Anwesenheit oder Abwesenheit der Behandlungsverbindung;
Messen des Niveaus eines Zellmarkers oder einer mRNA; und
Vergleichen des Niveaus des Zellmarkers oder der mRNA in den Zellen, die in Anwesenheit der Behandlungsverbindung kultiviert wurden, mit dem der Zellen, die in Abwesenheit der Behandlungsverbindung kultiviert wurden;
wobei die Behandlungsverbindung Cyclopropancarbonsäure {2-[(1*S*)-1-*3-ethoxy-4-methoxy-phenyl-2-methansulfonyl-ethyl-3-oxo-2,3-dihydro -1H-isoindol-4yl*-amid ist und
wobei ein verringertes Niveau des Zellmarkers oder der mRNA in Anwesenheit der Behandlungsverbindung eine Wahrscheinlichkeit eines wirksamen Ansprechens des Patienten auf die Behandlungsverbindung anzeigt,
wobei der Zellmarker ausgewählt ist aus CD3, CD56, Foxp3, Langerin und einer Kombination davon oder
wobei die mRNA ausgewählt ist aus mRNA von Keratin 16, IL-12/IL-23 p40, IL-23 p19, IL-17A, IL-22, DEFB4, IL-8, MX-1, IL-10, IFN-γ, CXCL9 und einer Kombination davon.

2. Verfahren nach Anspruch 1, wobei (i) das Niveau von nur einem der Zellmarker oder der mRNAs überwacht wird oder (ii) die Niveaus von zwei oder mehr Zellmarkern oder mRNAs gleichzeitig überwacht werden.

3. Verfahren zum Überwachen eines Ansprechens eines Patienten auf eine Schuppenflechtenbehandlung, wobei das Verfahren Folgendes umfasst:
Messen des Niveaus eines Zellmarkers oder einer mRNA in einer ersten Hautprobe des Patienten;
Messen des Niveaus desselben Zellmarkers oder derselben mRNA in einer zweiten Hautprobe, die nach einer Verabreichung einer Behandlungsverbindung an den Patienten erhalten wurde; und
Vergleichen der Niveaus des Zellmarkers oder der mRNA, die von der ersten und von der zweiten Hautprobe erhalten wurden;
wobei die Behandlungsverbindung Cyclopropancarbonsäure {2-[(1*S*)-1-*3-ethoxy-4-methoxy-phenyl-2-methansulfonyl-ethyl-3-oxo-2,3-dihydro* -*1H-isoindol-4yl*-amid ist und
wobei ein verringertes Niveau des Zellmarkers in der zweiten Hautprobe ein wirksames Ansprechen anzeigt,
wobei der Zellmarker ausgewählt ist aus CD3, CD56, Foxp3, Langerin und einer Kombination davon oder
wobei die mRNA ausgewählt ist aus mRNA von Keratin 16, IL-12/IL-23 p40, IL-23 p19, IL-17A, IL-22, DEFB4, IL-8, MX-1, IL-10, IFN-γ, CXCL9 und einer Kombination davon.

4. Verfahren nach Anspruch 3, wobei das Niveau von CD3, das von dem Dermisbereich erhalten wird, überwacht wird und wobei die Verringerung etwa 15 %, 20 %, 25 % oder 30 % oder mehr beträgt im Vergleich zu dem Niveau von CD3 in der ersten Hautprobe.

5. Verfahren nach Anspruch 3, wobei das Niveau von CD3, das von dem Epidermisbereich erhalten wird, überwacht wird und die Verringerung etwa 25 %, 30 %, 35 % oder 40 % oder mehr beträgt im Vergleich zu dem Niveau von CD3 in der ersten Hautprobe.

6. Verfahren nach Anspruch 3, wobei das Niveau von CD56, das von dem Dermisbereich erhalten wird, überwacht wird und wobei die Verringerung etwa 15 %, 20 %, 25 % oder 30 % oder mehr beträgt im Vergleich zu dem Niveau von CD56 in der ersten Hautprobe.

7. Verfahren nach Anspruch 3, wobei das Niveau von CD56, das von dem Epidermisbereich erhalten wird, überwacht wird und die Verringerung etwa 60 %, 65 %, 70 % oder 75 % oder mehr beträgt im Vergleich zu dem Niveau von CD56 in der ersten Hautprobe.

8. Verfahren nach Anspruch 3, wobei das Niveau von Langerin, das von dem Dermisbereich erhalten wird, überwacht wird und wobei die Verringerung etwa 40 %, 45 %, 50 % oder 55 % oder mehr beträgt im Vergleich zu dem Niveau von Langerin in der ersten Hautprobe.

9. Verfahren zum Überwachen einer Patientencompliance mit einem Wirkstoffbehandlungsprotokoll gegen Schuppenflechte, das Folgendes umfasst:
Messen des Niveaus eines Zellmarkers oder einer mRNA in einer Hautprobe des Patienten; und
Bestimmen, ob das Expressionsniveau in der Hautprobe verringert ist im Vergleich zu dem Expressionsniveau in einer unbehandelten Kontrollprobe,
wobei das Wirkstoffbehandlungsprotokoll ein Verabreichen von Cyclopropancarbonsäure {2-[(1*S*)-1-(3-*ethoxy*-4-*methoxy*-*phenyl*)-*2-methansulfonyl-ethyl-3-oxo-2,3-dihydro-1H-isoindol-4yl-*amid an den Patienten umfasst und
wobei eine verringerte Expression eine Patientencompliance mit dem Wirkstoffbehandlungsprotokoll anzeigt,
wobei der Zellmarker ausgewählt ist aus CD3, CD56, Foxp3, Langerin und einer Kombination davon oder
wobei die mRNA ausgewählt ist aus einer mRNA von Keratin 16, IL-12/IL-23 p40, IL-23 p19, IL-17A, IL-22, DEFB4, IL-8, MX-1, IL-10, IFN-γ, CXCL9 und einer Kombination davon.

10. Verfahren nach Anspruch 3 oder Anspruch 9, wobei
(i) das Niveau von IL-12/IL-13-p40-mRNA überwacht wird und die Verringerung etwa 80 %, 85 %, 90 % oder 95 % oder mehr beträgt im Vergleich zu dem Niveau von IL-12/IL-13-p40-mRNA in der ersten Hautprobe;
(ii) das Niveau von IL-22-mRNA überwacht wird und die Verringerung etwa 65 %, 70 %, 75 % oder 80 % oder mehr beträgt im Vergleich zu dem Niveau von IL-22-mRNA in der ersten Hautprobe;
(iii) das Niveau von IL-8-mRNA überwacht wird und die Verringerung etwa 65 %, 70 %, 75 % oder 80 % oder mehr beträgt im Vergleich zu dem Niveau von IL-8-mRNA in der ersten Hautprobe;
(iv) das Niveau von DEFB4-mRNA überwacht wird und die Verringerung etwa 45 %, 50 %, 55 % oder 60 % oder mehr beträgt im Vergleich zu dem Niveau von DEFB4-mRNA in der ersten Hautprobe;
(v) das Niveau von MX-1-mRNA überwacht wird und die Verringerung etwa 40 %, 45 %, 50 % oder 55 % oder mehr beträgt im Vergleich zu dem Niveau von MX-1-mRNA in der ersten Hautprobe;
(vi) das Niveau von Keratin-16-mRNA überwacht wird und die Verringerung etwa 50 %, 55 %, 60 % oder 65 % oder mehr beträgt im Vergleich zu dem Niveau von Keratin-16-mRNA in der ersten Hautprobe;
(vii) das Niveau von IL-17A-mRNA überwacht wird und die Verringerung etwa 60 %, 65 %, 70 % oder 75 % oder mehr beträgt im Vergleich zu dem Niveau von IL-17A-mRNA in der ersten Hautprobe;
(viii) das Niveau von IL-23-p19-mRNA überwacht wird und Verringerung etwa 40 %, 45 %, 50 % oder 55 % oder mehr beträgt im Vergleich zu dem Niveau von IL-23-p19-mRNA in der ersten Hautprobe;
(ix) das Niveau von IL-10-mRNA überwacht wird und die Verringerung etwa 40 %, 45 %, 50 % oder 55 % oder mehr beträgt im Vergleich zu dem Niveau von IL-10-mRNA in der ersten Hautprobe;
(x) das Niveau von IFN-γ-mRNA überwacht wird und die Verringerung etwa 30 %, 35 %, 40 % oder 45 % oder mehr beträgt im Vergleich zu dem Niveau von IFN-γ-mRNA in der ersten Hautprobe; oder
(xi) das Niveau von CXCL9-mRNA überwacht wird und die Verringerung etwa 20 %, 25 %, 30 % oder 35 % oder mehr beträgt im Vergleich zu dem Niveau von CXCL9-mRNA in der ersten Hautprobe.

## Revendications

1. Procédé pour prédire si un patient sera sensible à un traitement pour le psoriasis comprenant :
la culture de cellules provenant d'un échantillon de peau du patient en présence ou en l'absence du composé du traitement ;
la mesure du taux d'un marqueur cellulaire ou d'un ARNm ; et
la comparaison du taux du marqueur cellulaire ou de l'ARNm dans les cellules cultivées en présence du composé du traitement à celui dans les cellules cultivées en l'absence du composé du traitement,
dans lequel le composé du traitement est le {2-[(1S)-1-(3-éthoxy-4-méthoxy-phényl)-2-méthanesulfonyl-éthyl]-3-oxo-2,3-dihydro-1*H*-isoindol-4-yl}-amide d'acide cyclopropanecarboxylique, et
dans lequel un taux diminué du marqueur cellulaire ou de l'ARNm en présence du composé du traitement indique la probabilité d'une réponse efficace du patient au composé du traitement,
dans lequel le marqueur cellulaire est choisi parmi le CD3, le CD56, Foxp3, la langérine et l'une de leurs combinaisons ou
dans lequel l'ARNm est choisi parmi l'ARNm pour la kératine 16, la p40 de l'IL-12/IL-23, la p19 de l'IL-23, l'IL-17A, l'IL-22, la DEFB4, l'IL-8, la MX-1, l'IL-10, l'IFN-γ, le CXCL9 et l'une de leurs combinaisons.

2. Procédé selon la revendication 1, dans lequel (i) le taux de seulement l'un des marqueurs cellulaires ou des ARNm est surveillé, ou (ii) les taux de deux ou plus des marqueurs cellulaires ou des ARNm sont surveillés simultanément.

3. Procédé pour surveiller la réponse d'un patient à un traitement du psoriasis comprenant :
la mesure du taux d'un marqueur cellulaire ou d'un ARNm dans un premier échantillon de peau provenant du patient ;
la mesure du taux du même marqueur cellulaire ou ARNm dans un second échantillon de peau obtenu après l'administration d'un composé du traitement au patient ; et
la comparaison des taux du marqueur cellulaire ou de l'ARNm obtenus à partir des premier et second échantillons de peau,
dans lequel le composé du traitement est le {2-[(1*S*)-1-(3-éthoxy-4-méthoxy-phényl)-2-méthanesulfonyl-éthyl]-3-oxo-2,3-dihydro-1*H*-isoindol-4-yl}-amide d'acide cyclopropanecarboxylique, et
dans lequel un taux diminué du marqueur cellulaire dans le second échantillon de peau indique une réponse efficace,
dans lequel le marqueur cellulaire est choisi parmi le CD3, le CD56, Foxp3, la langérine et l'une de leurs combinaisons, ou
dans lequel l'ARNm est choisi parmi l'ARNm pour la kératine 16, la p40 de l'IL-12/IL-23, la p19 de l'IL-23, l'IL-17A, l'IL-22, la DEFB4, l'IL-8, la MX-1, l'IL-10, l'IFN-γ, le CXCL9 et l'une de leurs combinaisons.

4. Procédé selon la revendication 3, dans lequel le taux de CD3 obtenu à partir de la région du derme est surveillé, et la diminution est d'environ 15 %, 20 %, 25 %, ou 30 % ou plus comparativement au taux de CD3 dans le premier échantillon de peau.

5. Procédé selon la revendication 3, dans lequel le taux de CD3 obtenu à partir de la région de l'épiderme est surveillé, et la diminution est d'environ 25 %, 30 %, 35 %, ou 40 % ou plus comparativement au taux de CD3 dans le premier échantillon de peau.

6. Procédé selon la revendication 3, dans lequel le taux de CD56 obtenu à partir de la région du derme est surveillé, et la diminution est d'environ 15 %, 20 %, 25 %, ou 30 % ou plus comparativement au taux de CD56 dans le premier échantillon de peau.

7. Procédé selon la revendication 3, dans lequel le taux de CD56 obtenu à partir de la région de l'épiderme est surveillé, et la diminution est d'environ 60 %, 65 %, 70 %, ou 75 % ou plus comparativement au taux de CD56 dans le premier échantillon de peau.

8. Procédé selon la revendication 3, dans lequel le taux de langérine obtenu à partir de la région du derme est surveillé, et la diminution est d'environ 40 %, 45 %, 50 %, ou 55 % ou plus comparativement au taux de langérine dans le premier échantillon de peau.

9. Procédé pour surveiller l'observance par le patient d'un protocole de traitement médicamenteux pour le psoriasis comprenant :
la mesure du taux d'un marqueur cellulaire ou d'un ARNm dans un échantillon de peau provenant du patient ; et
la détermination si le taux d'expression est diminué dans l'échantillon de peau comparativement au taux d'expression dans un échantillon témoin non traité,
dans lequel le protocole de traitement médicamenteux comprend l'administration de {2-[(1*S*)-1-(3-éthoxy-4-méthoxy-phényl)-2-méthanesulfonyl-éthyl]-3-oxo-2,3-dihydro-1*H*-isoindol-4-yl}-amide d'acide cyclopropane-carboxylique au patient, et
dans lequel une expression diminuée indique l'observance par le patient dudit protocole de traitement médicamenteux
dans lequel le marqueur cellulaire est choisi parmi le CD3, le CD56, Foxp3, la langérine et l'une de leurs combinaisons, ou
dans lequel l'ARNm est choisi parmi l'ARNm pour la kératine 16, la p40 de l'IL-12/IL-23, la p19 de l'IL-23, l'IL-17A, l'IL-22, la DEFB4, l'IL-8, la MX-1, l'IL-10, l'IFN-γ, le CXCL9 et l'une de leurs combinaisons.

10. Procédé selon la revendication 3 ou 9, dans lequel
(i) le taux d'ARNm de la p40 de l'IL-12/IL-13 est surveillé, et la diminution est d'environ 80 %, 85 %, 90 %, ou 95 % ou plus comparativement au taux d'ARNm de la p40 de l'IL-12/IL-13 dans le premier échantillon de peau ;
(ii) le taux d'ARNm de l'IL-22 est surveillé, et la diminution est d'environ 65 %, 70 %, 75 %, ou 80 % ou plus comparativement au taux d'ARNm de l'IL-22 dans le premier échantillon de peau ;
(iii) le taux d'ARNm de l'IL-8 est surveillé, et la diminution est d'environ 65 %, 70 %, 75 %, ou 80 % ou plus comparativement au taux d'ARNm de l'IL-8 dans le premier échantillon de peau ;
(iv) le taux d'ARNm de la DEFB4 est surveillé, et la diminution est d'environ 45 %, 50 %, 55 %, ou 60 % ou plus comparativement au taux d'ARNm de la DEFB4 dans le premier échantillon de peau ;
(v) le taux d'ARNm de la MX-1 est surveillé, et la diminution est d'environ 40 %, 45 %, 50 %, ou 55 % ou plus comparativement au taux d'ARNm de la MX-1 dans le premier échantillon de peau ;
(vi) le taux d'ARNm de la kératine 16 est surveillé, et la diminution est d'environ 50 %, 55 %, 60 %, ou 65 % ou plus comparativement au taux d'ARNm de la kératine 16 dans le premier échantillon de peau ;
(vii) le taux d'ARNm de l'IL-17A est surveillé, et la diminution est d'environ 60 %, 65 %, 70 %, ou 75 % ou plus comparativement au taux d'ARNm de l'IL-17A dans le premier échantillon de peau ;
(viii) le taux d'ARNm de la p19 de l'IL-23 est surveillé, et la diminution est d'environ 40 %, 45 %, 50 %, ou 55 % ou plus comparativement au taux d'ARNm de la p19 de l'IL-23 dans le premier échantillon de peau ;
(ix) le taux d'ARNm de l'IL-10 est surveillé, et la diminution est d'environ 40 %, 45 %, 50 %, ou 55 % ou plus comparativement au taux d'ARNm de l'IL-10 dans le premier échantillon de peau ;
(x) le taux d'ARNm de l'IFN-γ est surveillé, et la diminution est d'environ 30 %, 35 %, 40 %, ou 45 % ou plus comparativement au taux d'ARNm de l'IFN-γ dans le premier échantillon de peau ; ou
(xi) le taux d'ARNm du CXCL9 est surveillé, et la diminution est d'environ 20 %, 25 %, 30 %, ou 35 % ou plus comparativement au taux d'ARNm du CXCL9 dans le premier échantillon de peau.
